# EUROPEAN PATENT APPLICATION

(11) **EP 4 821 954 A1**
(43) Date of publication of application: **14.10.2026**
(21) Application number: 24900721.2
(22) Date of filing: 06.12.2024
(51) Int. Cl.: C07K 19/00, C07K 16/00, C12N 1/14, C12N 1/16, C12N 1/20, C12N 5/07, C12N 5/071, C12N 5/0783, C12N 15/09

(54) **FC-CELL COMPLEX AND METHOD FOR PRODUCING THE SAME**

(30) Priority: 06.12.2023 JP 2023206525
(71) Applicant: AJINOMOTO CO., INC., Chuo-ku Tokyo 104-8315 (JP)
(72) Inventor: CHIBA, Akira, Kawasaki-shi, Kanagawa 210-8681 (JP); MATSUMOTO, Takuya, Kawasaki-shi, Kanagawa 210-8681 (JP); ITO, Kenichiro, Kawasaki-shi, Kanagawa 210-8681 (JP); YAMADA, Kei, Kawasaki-shi, Kanagawa 210-8681 (JP); MATSUDA, Yoshihiko, Kawasaki-shi, Kanagawa 210-8681 (JP); TAKAHASHI, Kazutoshi, Kawasaki-shi, Kanagawa 210-8681 (JP); YAMAGUCHI, Hiroki, Kawasaki-shi, Kanagawa 210-8681 (JP); FUJII, Tomohiro, Kawasaki-shi, Kanagawa 210-8681 (JP); SEGAWA, Shinsuke, Kawasaki-shi, Kanagawa 210-8681 (JP); MATSUDA, Yutaka, Kawasaki-shi, Kanagawa 210-8681 (JP)
(74) Representative: Hoffmann Eitle
(86) International application number: PCT/JP2024/043299
(87) International publication number: WO 2025/121429

(57) **Abstract**

An Fc-cell complex comprising a cell and an antibody Fc region bound to a surface of the cell, wherein the antibody Fc region is formed by disulfide bonding of two Fc-constituting polypeptides, and wherein the antibody Fc region is chemically bound to the cell surface via a linker introduced into a functional group in a side chain of a particular amino acid residue present at one or more positions of one of the two antibody Fc-constituting polypeptides.

## Description

### TECHNICAL FIELD

The present invention relates to an Fc-cell complex (also referred to as Fc-cell conjugate) and a method of producing it.

### BACKGROUND ART

Techniques for binding an antibody to the surface of a cell in order to impart a function to the cell are known.

For example, Patent Document 1 (JP 2016-523237 A) discloses a technique of preparing a protein-cell conjugate by binding a protein such as an antibody to a cell surface by reacting a sulfhydryl group on the cell surface with a side-chain amino group of the protein using a bifunctional cross-linking agent. However, in this technique, the cross-linking agent randomly reacts with side-chain amino groups of the protein such as an antibody. Thus, the introduction ratio of the protein to cells cannot be easily controlled, and moreover, the binding positions of linkers in the protein become non-uniform, which is problematic.

### PRIOR ART DOCUMENTS

### Patent Documents

Patent Document 1: JP2016-523237A
Patent Document 2: WO2018/199337
Patent Document 3: WO2019/240287
Patent Document 4: WO2019/240288
Patent Document 5: WO2020/090979

### SUMMARY OF THE INVENTION

### PROBLEMS TO BE SOLVED BY THE INVENTION

To introduce an antibody-like molecule such as an antibody variable region or a functional protein-Fc fusion protein to a cell surface via the Fc region of an antibody, an Fc-cell complex in which the Fc region of the antibody is introduced to the cell surface via a linker is prepared. An object of the present invention is to provide a technique that enables, in such preparation, efficient introduction of the Fc region to the cell by controlling the binding position of the linker in the Fc region and the introduction ratio of the Fc region to the cell. Another object of the present invention is to provide an Fc-cell complex in which the binding position of the linker in the Fc region and the introduction ratio of the Fc region to the cell are controlled.

### MEANS FOR SOLVING THE PROBLEMS

The present applicant has reported a technique capable of preparing an antibody having, in a regioselective manner, a functional substance such as a drug by a chemical synthetic method using a predetermined compound containing an affinity peptide (Patent Documents 2 to 5).

In this technique, linkers are introduced to both of the two heavy chain constant region-derived polypeptides constituting an Fc region, but the present inventors considered that introduction of a linker to only one of the two heavy chain constant region-derived polypeptides constituting an Fc region is more preferred for the above object.

Thus, for the purpose of developing a technique capable of easily chemically modifying only one of the two heavy chain constant region-derived polypeptides constituting an Fc region, the present inventors intensively studied to improve the above technique.

As a result, the present inventors discovered that, by use of an affinity substance having affinity for an Fc region, preferably an affinity substance containing first and second affinity moieties, and a compound containing a reactive group for an Fc region, only one of the heavy chain constant region-derived polypeptides of the constituent units of an Fc region can be easily chemically modified. The present inventors also discovered that an Fc-cell complex in which an Fc region is bound to a cell surface via one of the polypeptides thereof can be obtained by using this technique, and that, when this Fc region contains an antibody variable region or a functional protein, the antibody or the functional protein can be introduced to the cell surface via the Fc region.

According to the above technique, an affinity substance having affinity for an Fc region, preferably an affinity substance (A) containing first and second affinity moieties, and a compound containing a reactive group (R) for an Fc region, can associate with the Fc region via the affinity substance (A), and then can react specifically, via the reactive group (R), with the side chain of a particular amino acid residue in one of the heavy chain constant region-derived polypeptides of the constituent units of the Fc region, thereby generating an affinity substance-modified Fc region in which only one of the two heavy chain constant region-derived polypeptides constituting the Fc is modified (Fig. 1) (WO2023/234416).

While not wishing to be bound by theory, the mechanism of modification of only one of the heavy chain constant region-derived polypeptides of the constituent units of the Fc region is as follows.

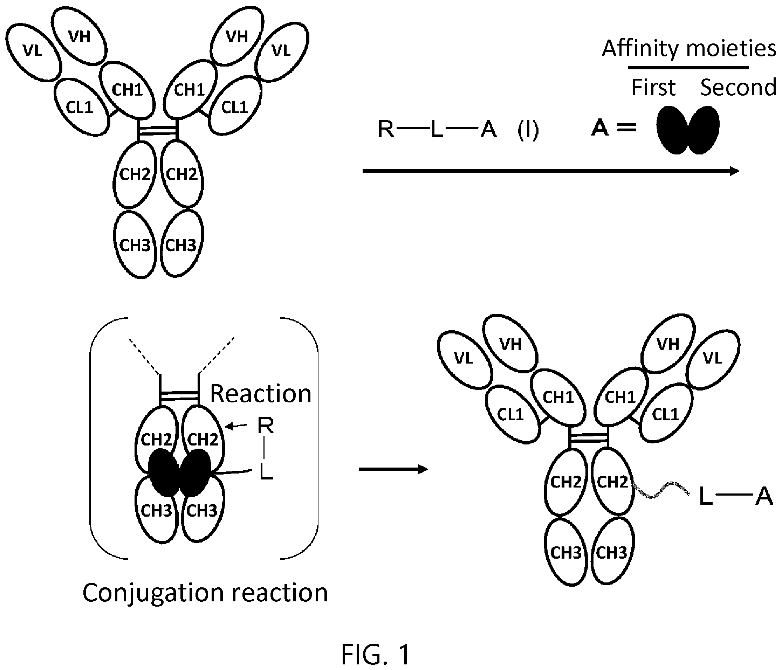

Since the affinity substance (A) contained in the compound can stably associate with the two heavy chain constant region-derived polypeptides constituting the Fc region, the reactive group (R) contained in the compound can modify only one of the heavy chain constant region-derived polypeptides (Fig. 1). At this time, since the other heavy chain constant region-derived polypeptide is sterically hindered due to association of an affinity portion, the reactive group (R) in the other molecule of the compound cannot react with the side chain of an amino acid residue of the other heavy chain constant region-derived polypeptide, so that only one heavy chain constant region-derived polypeptide can be modified (Fig. 1).

By binding the thus obtained Fc region, in which only one of the heavy chain constant region-derived polypeptides is modified, to a cell surface via a linker, it is possible to obtain an Fc-cell complex in which the Fc region is chemically bound to the cell surface via the linker introduced into a functional group in the side chain of a particular amino acid residue present at one or more positions of one of the two heavy chain constant region-derived polypeptides contained in the Fc region. When this Fc region contains an antibody variable region or a functional protein, it is possible to obtain an antibody-like molecule-cell complex in which an antibody-like molecule such as an antibody or a functional protein-Fc fusion protein is chemically bound to the cell surface via the linker introduced into a functional group in the side chain of a particular amino acid residue present at one or more positions of one of the two heavy chain constant region-derived polypeptides contained in the Fc region.

Specifically, the present invention is exemplified as follows.
[1] An Fc-cell complex comprising a cell and an antibody Fc region bound to a surface of the cell,
   wherein the antibody Fc region is formed by disulfide bonding of two Fc-constituting polypeptides, and
   wherein the antibody Fc region is chemically bound to the cell surface via a linker introduced into a functional group in a side chain of a particular amino acid residue present at one or more positions of one of the two antibody Fc-constituting polypeptides.
[2] The Fc-cell complex according to [1], wherein the particular amino acid residue is one or more of the following 14 amino acid residues: asparagine, glutamine, methionine, proline, serine, threonine, tryptophan, tyrosine, aspartic acid, glutamic acid, arginine, histidine, and lysine.
[3] The Fc-cell complex according to [1], wherein the particular amino acid residue is a lysine residue.
[4] The Fc-cell complex according to [3], wherein the lysine residue is one or more of the lysine residues at positions 246, 248, 274, 288, 290, 317, 320, 322, 360, 414, and 439.
[5] The Fc-cell complex according to any one of [1] to [4], wherein the antibody Fc region has a functional substance introduced therein via a linker introduced into a functional group in a side chain of a particular amino acid residue present at one or more positions of the other of the two Fc-constituting polypeptides.
[6] The Fc-cell complex according to [5], wherein the functional substance is a drug, a labeling substance, an affinity substance, a transporting substance, or a stabilizer.
[7] The Fc-cell complex according to any one of [1] to [6], wherein the antibody Fc region comprises an antibody variable region.
[8] The Fc-cell complex according to any one of [1] to [6], wherein the antibody Fc region comprises a fusion protein of an antibody Fc-constituting polypeptide and a functional polypeptide.
[9] The Fc-cell complex according to any one of [1] to [8], wherein the antibody Fc region is introduced into the cell surface by reaction between a bioorthogonal functional group introduced via a linker that is introduced into a functional group in a side chain of a particular amino acid residue present at one or more positions of one of the two Fc-constituting polypeptides, and a group that is present on or introduced into the cell surface and reacts with the bioorthogonal functional group.
[10] A method of producing a cell-Fc region complex, the method comprising
   (I) preparing an antibody Fc region formed by disulfide bonding of two antibody Fc-constituting polypeptides;
   (II) reacting the antibody Fc region with a compound represented by the following Formula (Ia) or Formula (Ib):

      [Chemical formula 1] R-L₁-CLE(B)-L₂-A (Ia)

      wherein
      R represents a reactive group for the Fc region,
      L₁ represents a first linker,
      L₂ represents a second linker,
      CLE(B) represents a cleavable portion capable of generating, by cleavage, a bioorthogonal functional group on the reactive group side, and
      A represents an affinity substance containing an affinity peptide, or
      [Chemical formula 2] 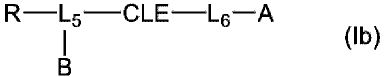 wherein R represents the reactive group,
      L₅ represents a fifth linker,
      L₆ represents a sixth linker,
      B represents a group containing a bioorthogonal functional group,
      CLE represents a cleavable portion, and
      A represents the affinity substance,
      to form an Fc-Compound (Ia) complex or an Fc-Compound (Ib) complex;
   (III) subjecting the Fc-Compound (Ia) complex or the Fc-Compound (Ib) complex to cleavage treatment at the cleavable portion, to form an Fc region that does not comprise the affinity substance and to which the bioorthogonal functional group has been introduced;
   (IV) preparing a cell having, on a surface thereof, a functional group that reacts with the bioorthogonal functional group; and
   (V) introducing the Fc region to the cell surface by reacting the Fc region, to which the bioorthogonal functional group is introduced, with the cell, through reaction of the bioorthogonal functional group of the Fc region with the functional group of the cell that reacts with the bioorthogonal functional group.
[11] The method of producing a cell-Fc region complex according to [10], wherein Compound (Ia) is represented by the following Formula (Ia-1): 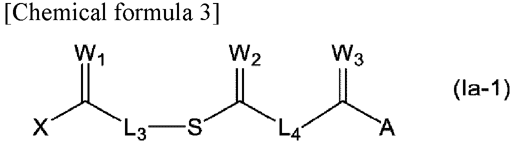 wherein
   X represents a leaving group,
   W₁, W₂, and W₃ each independently represent an oxygen atom or a sulfur atom,
   L₃ represents a third linker,
   L₄ represents a fourth linker,
   S represents a sulfur atom, and
   A represents the affinity substance.
[12] The method of producing a cell-Fc region complex according to [10], wherein Compound (Ib) is represented by the following Formula (Ib-1): 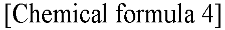 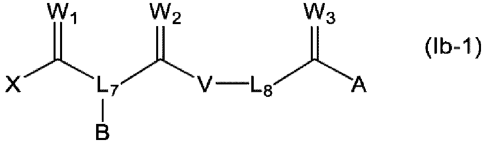 wherein
   X represents a leaving group,
   W₁, W₂, and W₃ each independently represent an oxygen atom or a sulfur atom,
   L₇ represents a seventh linker,
   L₈ represents an eighth linker,
   B represents a group containing a bioorthogonal functional group,
   V represents an oxygen atom or a sulfur atom, and
   A represents the affinity substance.
[13] The method according to any one of [10] to [12], wherein A is represented by AP1-L_{A}-AP2,
   wherein AP1 represents a first affinity peptide having affinity for a constant region of a heavy chain of an antibody, AP2 represents a second affinity peptide having affinity for a constant region of a heavy chain of an antibody, and L_{A} represents a linker.
[14] The method of producing a cell-Fc region complex according to any one of [10] to [13], wherein the bioorthogonal functional group is an azide group, an alkyne residue, a tetrazine residue, an alkene residue, a thiol group, a maleimide residue, a furan residue, or a halocarbonyl residue.
[15] The method of producing a cell-Fc region complex according to any one of [10] to [14], wherein the cell-Fc region complex is the Fc-cell complex according to any one of [1] to [9].
[16] The method of producing a cell-Fc region complex according to any one of [10] to [14], wherein the cell-Fc region complex is the Fc-cell complex according to [5], and
   wherein the method comprises introducing the functional substance to the functional group in the side chain of the particular amino acid residue present at one or more positions of the other of the two Fc-constituting polypeptides in the antibody Fc region.

### EFFECTS OF THE INVENTION

According to the present invention, only one of the constituent units (two heavy chain constant region-derived polypeptides) of an Fc region of an antibody can be easily modified. Further, according to the present invention, a regioselectively modified Fc region can be provided while easily modifying only one heavy chain constant region-derived polypeptide in the constituent units of the antibody Fc region. By using a modified Fc obtained by such a regioselective modification technique in which a bioorthogonal functional group is introduced into only one of the constituent units of the Fc region, an Fc-cell complex in which the Fc region is regioselectively introduced to a cell surface via only one of the constituent units of the Fc region can be obtained. By this, orientation of an antibody or a fusion protein containing an Fc region that is presented on a cell surface can be controlled, and the antibody or the fusion protein, even in a small amount, can be allowed to produce its effect without impairing the activity.

### BRIEF DESCRIPTION OF THE DRAWINGS

Fig. 1 shows a presumed principle of a method of modifying only one of the two heavy chain constant region-derived polypeptides constituting Fc with an affinity substance.
Fig. 2 shows a reaction using Compound (Ia) (Step (II) and Step (III)). r represents a modification rate for an immunoglobulin unit (the same applies hereinafter).
Fig. 3 shows a reaction using Compound (Ia-1) (Step (II) and Step (III)).
Fig. 4 shows a reaction using Compound (Ib) (Step (II) and Step (III)). T₁ represents a monovalent group generated by cleavage (the same applies to T₂ and the like below).
Fig. 5 shows a reaction using Compound (Ib-1) (Step (II) and Step (III)).
Fig. 6 shows a reaction using Compound (Ia) (Step (II) (Reactions 1 and 2) and Step (III) (Reaction 3)) (introduction of a bioorthogonal functional group to each of the two heavy chain constant region-derived polypeptides constituting Fc).
Fig. 7 shows a reaction using Compound (Ib) (Step (II) (Reactions 1 and 2) and Step (III) (Reaction 3)) (introduction of a bioorthogonal functional group to each of the two heavy chain constant region-derived polypeptides constituting Fc).
Fig. 8 shows the proportion of T-4 antibody-positive cells after adding T-4 antibody to cells at each concentration and allowing the cells to react for 1 hour or 24 hours in the presence or absence of DBCO. The results of flow cytometry are presented as a graph.
Fig. 9 shows the amount of fluorescence derived from T-5 bound to cells (left) or fluorescence derived from HER2-biotin/StAv-AF488 bound to cells (right) after adding T-5 antibody (left) and further adding HER2-biotin/StAv-AF488 (right) to the cells, as observed immediately after the addition or 48 hours after the addition.
Fig. 10 shows AF488-derived fluorescence in HEL cell/HER2-Fc/StAv-AF488 or HEL cell/DBCO-modified T-5/HER2-Fc/StAv-AF488.
Fig. 11 shows Cy5-derived fluorescence (top) and AF488-derived fluorescence (middle) at 0 hours, 24 hours, 48 hours, 72 hours, or 96 hours after adding T-5 antibody (without or with DBCO modification) and further adding HER2-biotin/StAv-AF488 to cells, and a graph illustrating the HER2-binding ability per antibody at 0 hours, 24 hours, and 48 hours after the addition (bottom). The graphs show results of flow cytometry.
Fig. 12A shows the amount of fluorescence derived from T-5, T-8, or T-9 bound to cells (left) or fluorescence derived from HER2-biotin/StAv-AF488 bound to cells (right) after adding 0.3 µM or 1 µM T-5 antibody, T-8 antibody, or T-9 antibody (left) and further adding HER2-biotin/StAv-AF488 (right) to the cells, as observed immediately after the addition or 48 hours after the addition.
Fig. 12B shows the results of calculation of the HER2-binding ability per antibody based on the amounts of fluorescence derived from T-5, T-8, or T-9 bound to cells and fluorescence derived from HER2-biotin/StAv-AF488 bound to cells after adding 0.3 µM or 1 µM T-5 antibody, T-8 antibody, or T-9 antibody and further adding HER2-biotin/StAv-AF488 to the cells, as observed immediately after the addition or 24 hours after the addition.
Fig. 13 shows the proportion of T-4 antibody-positive cells after adding T-4 antibody to cells at each concentration and allowing the reaction to proceed for 1 hour or 24 hours in the presence or absence of DBCO. The left panel shows Gated (%), and the right panel shows MFI (mean fluorescence intensity; after subtraction of the background). The graphs show results of flow cytometry.
Fig. 14 shows the expression of each polypeptide in each transformant (electrophoresis photographs).
Fig. 15 shows the luminescence intensity from a surface antibody of an antibody-NK cell conjugate (ACC-1). A control (NK cells) is also shown.
Fig. 16 shows the luminescence intensities from each surface antibody of antibody-NK cell conjugates (ACC-3, ACC-4, and ACC-5). A control (NK cells) is also shown.
Fig. 17 shows the cytotoxicity evaluation of ACC-1, ACC-2, ACC-3, ACC-4, and ACC-5 (the ratio of NK cells (control) or each antibody-NK cell conjugate to SK-BR-3 = 6:1).
Fig. 18 shows the cytotoxicity evaluation of ACC-3, ACC-4, ACC-5, and ACC-6 (effector: target ratio = 6:1). A control (NK cells) is also shown.
Fig. 19 shows the HER2-binding abilities of ACC-5 and ACC-6. A control (NK cells) is also shown.

### EMBODIMENTS FOR CARRYING OUT THE INVENTION

### <Fc-Cell Complex>

The Fc-cell complex of the present invention comprises a cell and an Fc region bound to a surface of the cell;
wherein the Fc region is formed by disulfide bonding of two Fc region-constituting units (that is, heavy chain constant region-derived polypeptides), and
wherein the Fc region is chemically bound to the cell surface via a linker introduced into a functional group in the side chain of a particular amino acid residue present at one or more positions, preferably one position, of one of the two Fc region-constituting units.

### <Cell>

The type and the origin of the cell are not particularly limited, and the cell may be a eukaryotic cell or a prokaryotic cell. Examples of the eukaryotic cell are not particularly limited, and may include animal cells, plant cells, yeast cells, and fungal cells. Examples of the prokaryotic cell are not particularly limited, and may include bacterial cells.

The cell is preferably a eukaryotic cell such as a mammalian cell. Typical examples of the cell include human cells, mouse cells, and rat cells. The cell is preferably a human cell.

The cell may be a somatic cell or a germ cell. Examples of the somatic cell are not particularly limited, and may include blood cells, immune cells, vascular cells, skeletal muscle cells, cardiac muscle cells, nerve cells, retinal cells, hepatocytes, digestive system cells, fibroblasts, ovarian cells, and testicular cells. Examples of the germ cell include sperm cells and egg cells.

The cell may be a stem cell. Examples of the stem cell include multipotent stem cells and pluripotent stem cells, such as induced pluripotent stem cells (iPSCs), embryonic stem cells (ESCs), and mesenchymal stem cells (MSCs).

The cell may be an immune system cell. Examples of the immune system cell include B cells, T cells, and natural killer (NK) cells.

Examples of the T cells include naive T (TN) cells; effector T (TEFF) cells; memory T cells and subtypes thereof, such as stem cell memory T (TSCM) cells, central memory T (TCM) cells, and effector memory T (TEM) cells or terminally differentiated effector memory T cells; tumor-infiltrating lymphocytes (TIL); immature T cells; mature T cells; helper T cells; cytotoxic T cells; mucosal-associated invariant T (MAIT) cells; natural and adaptive regulatory T (Treg) cells; helper T cells, such as TH1 cells, TH2 cells, TH3 cells, TH17 cells, TH9 cells, TH22 cells, and follicular helper T cells; alpha/beta T cells; and delta/gamma T cells.

The cell may be a monocyte, a granulocyte, a bone marrow cell, a macrophage, a neutrophil, a dendritic cell, a mast cell, an eosinophil, and/or a basophil.

The cell may be a cell containing one or more types of nucleic acid introduced by genetic manipulation, and may hence express a recombinant product or the like of the introduced nucleic acid.

The cell may be an established cell, or may be a primary cell.

The established cell can be obtained from a cell bank or the like.

In cases where the cell is a primary cell isolated from a subject, examples of the specimen from which the cell is isolated include whole blood, peripheral blood mononuclear cells (PBMC), leukocytes, bone marrow, thymus, tissue biopsy, tumor, leukemia, lymphoma, lymph node, gut-associated lymphoid tissue, mucosal-associated lymphoid tissue, spleen, other lymphoid tissues, liver, lung, stomach, intestine, colon, kidney, pancreas, breast, bone, prostate, cervix, testis, ovary, tonsil, or other organs, and/or cells derived therefrom.

### <Fc Region>

An antibody Fc region is formed by disulfide bonding of two Fc region-constituting units (that is, heavy chain constant region-derived polypeptides). Here, examples of the Fc region-constituting unit include a polypeptide containing the hinge region, the CH2 region, and the CH3 region of a heavy chain constant region. Between the two antibody Fc region-constituting units, a disulfide bond is formed by reaction between the thiol groups of cysteine residues in their hinge regions.

The antibody Fc region can be obtained, for example, by papain digestion of an immunoglobulin according to a known method.

The Fc region may further contain an antibody variable region, for example, an F(ab')₂ region.

In other words, the Fc-cell complex of the present invention may be an antibody-cell conjugate in which an antibody containing an F(ab')₂ region and an Fc region, that is, an immunoglobulin, is bound to a cell surface via a linker introduced to a side-chain functional group of a particular amino acid residue present in one of the heavy chain constant regions, preferably the CH2 region and the CH3 region.

The origin of the Fc or the antibody comprising Fc is not particularly limited, and for example, the antibody may be derived from an animal such as a mammal or a bird (for example, a chicken). The immunoglobulin unit is preferably derived from a mammal. Examples of such a mammal include primates (such as humans, monkeys, and chimpanzees), rodents (such as mice, rats, guinea pigs, hamsters, and rabbits), pets (such as dogs and cats), domestic animals (such as cows, pigs, and goats), and work animals (such as horses and sheep). Primates and rodents are preferred, and humans are more preferred.

The type of the antibody may be a polyclonal antibody or a monoclonal antibody. The antibody may be a divalent antibody (for example, IgG, IgD, or IgE) or a tetravalent or higher antibody (for example, IgA antibody or IgM antibody). The antibody is preferably a monoclonal antibody. Examples of the monoclonal antibody include chimeric antibodies, humanized antibodies, human antibodies, antibodies to which a predetermined glycan has been added (for example, an antibody modified so as to have a sugar chain-binding consensus sequence such as an N-type sugar chain-binding consensus sequence), bi-specific antibodies, Fc region proteins, Fc fusion proteins, and disulfide bond-reduced antibodies. Examples of the isotype of the monoclonal antibody include IgG (for example, IgG1, IgG2, IgG3, and IgG4), IgM, IgA, IgD, IgE, and IgY.

**In** cases where the Fc-cell conjugate is an antibody-cell conjugate, any antigen may be used as an antigen of the antibody. Examples of such an antigen include proteins [including oligopeptides and polypeptides; they may also be proteins modified with a biomolecule such as a sugar (for example, glycoproteins)], sugar chains, nucleic acids, and low molecular weight compounds. The antibody may be preferably an antibody that recognizes a protein as an antigen. Examples of the protein include cell membrane receptors, cell membrane proteins other than cell membrane receptors (for example, extracellular matrix proteins), ligands, and soluble receptors.

Specific examples of the monoclonal antibody include particular chimeric antibodies (for example, rituximab, basiliximab, infliximab, cetuximab, siltuximab, dinutuximab, and altertoxaximab), particular humanized antibodies (for example, daclizumab, palivizumab, trastuzumab, alemtuzumab, omalizumab, efalizumab, bevacizumab, natalizumab (IgG4), tocilizumab, eculizumab (IgG2), mogamulizumab, pertuzumab, obinutuzumab, vedolizumab, pembrolizumab (IgG4), mepolizumab, elotuzumab, daratumumab, ixekizumab (IgG4), reslizumab (IgG4), and atezolizumab), and particular human antibodies (for example, adalimumab (IgG1), panitumumab, golimumab, ustekinumab, canakinumab, ofatumumab, denosumab (IgG2), ipilimumab, belimumab, raxibacumab, ramucirumab, nivolumab, dupilumab (IgG4), secukinumab, evolocumab (IgG2), alirocumab, necitumumab, brodalumab (IgG2), and olaratumab) (cases not referring to the IgG subtype indicate that they are IgG1).

**In** another aspect, the Fc region may further contain a functional polypeptide. That is, a fusion protein of an Fc region and a functional polypeptide may be bound to a cell surface via a linker introduced into a side-chain functional group of a particular amino acid residue present in one of the heavy chain constant regions constituting the Fc region, preferably the CH2 region and the CH3 region, to form an Fc fusion protein-cell conjugate. **In** this case, the Fc fusion protein may be a dimer of functional polypeptide-Fc-constituting polypeptide (heavy chain constant region polypeptide) fusion proteins, or may be a dimer of a functional polypeptide-Fc-constituting polypeptide fusion protein and an Fc-constituting polypeptide. In the latter case, either of the two Fc-constituting polypeptides may be bound to the cell.

Examples of the functional polypeptide include, but are not particularly limited to, enzymes, fluorescent proteins, growth factors, hormones, cytokines, blood proteins, enzymes, antigens, antibodies, transcription factors, receptors, and partial peptides thereof.

Here, examples of the enzymes include lipase, protease, steroidogenic enzymes, kinase, phosphatase, xylanase, esterase, methylase, demethylase, oxidase, reductase, cellulase, aromatase, collagenase, transglutaminase, glycosidase, and chitinase.

Examples of the growth factors include epidermal growth factor (EGF), insulin-like growth factor (IGF), transforming growth factor (TGF), nerve growth factor (NGF), brain-derived neurotrophic factor (BDNF), vascular endothelial growth factor (VEGF), granulocyte colony-stimulating factor (G-CSF), granulocyte-macrophage colony-stimulating factor (GM-CSF), platelet-derived growth factor (PDGF), erythropoietin (EPO), thrombopoietin (TPO), fibroblast growth factor (FGF), and hepatocyte growth factor (HGF).

Examples of the cytokines include interleukins, interferons (IFNα, IFNβ, IFNγ), and tumor necrosis factor (TNF).

Examples of the blood proteins include thrombin, serum albumin, factor VII, factor VIII, factor IX, factor X, and tissue plasminogen activator.

Such a fusion protein can be obtained by ligating a polynucleotide encoding an Fc and a polynucleotide encoding a functional polypeptide in frame, expressing the resulting ligated polynucleotide in a host cell or the like, and then purifying the expression product.

The antibody Fc region is chemically bound to a cell surface via a linker introduced into a functional group in the side chain of a particular amino acid residue present at one or more positions, preferably at one position, of one of the two antibody Fc region-constituting polypeptides.

Here, the particular amino acid residue may be selected from one or more (for example, two, three, or four) of the following 14 amino acid residues present in the Fc region, preferably the CH2 domain: asparagine, glutamine, methionine, proline, serine, threonine, tryptophan, tyrosine, aspartic acid, glutamic acid, arginine, histidine, and lysine.

More preferred examples of such a particular amino acid residue include a lysine residue, a tyrosine residue, a serine residue, and a threonine residue.

For example, in a human IgG such as human IgG1, the following amino acid residues present in the heavy chain constant region can be exposed on the antibody surface, and hence these amino acid residues can be used for introduction of a particular cleavable site.
Exposed lysine residues
   CH2 domain (for example, positions 246, 248, 274, 288, 290, 317, 320, and 322)
   CH3 domain (for example, positions 360, 414, and 439)
Exposed tyrosine residues
   CH2 domain (for example, positions 278, 296, and 300)
   CH3 domain (for example, position 436)
Exposed serine residues
   CH2 domain (for example, positions 254, 267, and 298)
   CH3 domain (for example, positions 400, 415, and 440)
Exposed threonine residues
   CH2 domain (for example, positions 256 and 289)
   CH3 domain (for example, positions 335 and 359)

The positions of amino acid residues in the antibody and in the heavy chain constant region (for example, the CH2 domain), follow EU numbering (see http://www.imgt.org/IMGTScientificChart/Numbering/Hu_IGHGnber.html).

For example, in cases where human IgG is targeted, the lysine residue at position 246 corresponds to the 16th amino acid residue of the human IgG CH2 region; the lysine residue at position 248 corresponds to the 18th amino acid residue of the human IgG CH2 region; the lysine residue at position 288 corresponds to the 58th amino acid residue of the human IgG CH2 region; the lysine residue at position 290 corresponds to the 60th amino acid residue of the human IgG CH2 region; and the lysine residue at position 317 corresponds to the 87th amino acid residue of the human IgG CH2 region. The notation "position 246/248" indicates that a lysine residue at position 246 or 248 is the target. The notation "position 288/290" indicates that a lysine residue at position 288 or 290 is the target (see, for example, WO2016/186206, WO2018/199337, WO2019/240287, WO2019/240288, WO2020/009165, and WO2020/090979).

Preferably, the particular amino acid residue in the heavy chain constant region that is regioselectively modified can be a lysine residue (for example, a lysine residue at position 246/248 or position 288/290).

The linker introduced into the side chain of the particular amino acid residue in one of the polypeptides constituting the above Fc region is, on the other hand, chemically bound to a protein, a sugar chain, or the like present on the surface of a cell at the position of a functional group such as an amino group or a carboxyl group contained in the protein, sugar chain, or the like.

The length and the structure of the linker are not particularly limited as long as the function of the cell, the antibody, or the fusion protein is not inhibited. Those skilled in the art can arbitrarily select the length and structure of the linker. Depending on the type of linker compound used for preparing the Fc-cell complex, examples of the linker include the following divalent groups.

The divalent group is one group selected from, or a group having a backbone structure containing two or more groups selected from, the group consisting of a divalent linear hydrocarbon group, a divalent cyclic hydrocarbon group, a divalent heterocyclic group, -C(=O)-, -C(=S)-, -NR₁-, -C(=O)-NR₁-, -NR₁-C(=O)-, -C(=S)-NR₁-, -NR₁-C(=S)-, -O-, -S-, -(O-R₂)ₙ-, and -(S-R₂)ₘ-. R₁ represents a hydrogen atom or a substituent described later. R₂ represents a divalent linear hydrocarbon group, a divalent cyclic hydrocarbon group, or a divalent heterocyclic group. n and m are each an integer of 1 to 10, preferably an integer of 1 to 8, more preferably an integer of 1 to 6, still more preferably an integer of 1 to 5, especially preferably an integer of 1 to 3.

The divalent linear hydrocarbon group is a linear alkylene, a linear alkenylene, or a linear alkynylene.

The linear alkylene is a C₁₋₆ linear alkylene, preferably a C₁₋₄ linear alkylene. Examples of the linear alkylene include methylene, ethylene, n-propylene, n-butylene, n-pentylene, and n-hexylene.

The linear alkenylene is a C₂₋₆ linear alkenylene, preferably a C₂₋₄ linear alkenylene. Examples of the linear alkenylene include ethylenylene, n-propynylene, n-butenylene, n-pentenylene, and n-hexenylene.

The linear alkynylene is a C₂₋₆ linear alkynylene, preferably a C₂₋₄ linear alkynylene. Examples of the linear alkynylene include ethynylene, n-propynylene, n-butynylene, n-pentynylene, and n-hexynylene.

The divalent linear hydrocarbon group is preferably a linear alkylene.

The divalent cyclic hydrocarbon group is an arylene or a divalent nonaromatic cyclic hydrocarbon group.

The arylene is preferably a C₆₋₁₄ arylene, more preferably a C₆₋₁₀ arylene, especially preferably a C₆ arylene. Examples of the arylene include phenylene, naphthylene, and anthracenylene.

The divalent nonaromatic cyclic hydrocarbon group is preferably a C₃₋₁₂ monocyclic or polycyclic divalent nonaromatic cyclic hydrocarbon group, more preferably a C₄₋₁₀ monocyclic or polycyclic divalent nonaromatic cyclic hydrocarbon group, especially preferably a C₅₋₈ monocyclic divalent nonaromatic cyclic hydrocarbon group. Examples of the divalent nonaromatic cyclic hydrocarbon group include cyclopropylene, cyclobutylene, cyclopentylene, cyclohexylene, cycloheptylene, and cyclooctylene.

The divalent cyclic hydrocarbon group is preferably arylene.

The divalent heterocyclic group is a divalent aromatic heterocyclic group or a divalent nonaromatic heterocyclic group. The heterocycle preferably comprises, as a heteroatom(s) constituting it, one or more selected from the group consisting of an oxygen atom, a sulfur atom, a nitrogen atom, a phosphorus atom, a boron atom, and a silicon atom, more preferably comprises one or more selected from the group consisting of an oxygen atom, a sulfur atom, and a nitrogen atom. The divalent aromatic heterocyclic group is preferably a C₃₋₁₅ divalent aromatic heterocyclic group, more preferably a C₃₋₉ divalent aromatic heterocyclic group, especially preferably a C₃₋₆ divalent aromatic heterocyclic group. Examples of the divalent aromatic heterocyclic group include pyrrolediyl, furandiyl, thiophenediyl, pyridinediyl, pyridazinediyl, pyrimidinediyl, pyrazinediyl, triazinediyl, pyrazolediyl, imidazolediyl, thiazolediyl, isothiazolediyl, oxazolediyl, isoxazolediyl, triazolediyl, tetrazolediyl, indolediyl, purinediyl, anthraquinonediyl, carbazolediyl, fluorenediyl, quinolinediyl, isoquinolinediyl, quinazolinediyl, and phthalazinediyl.

The divalent nonaromatic heterocyclic group is preferably a C₃₋₁₅ nonaromatic heterocyclic group, more preferably a C₃₋₉ nonaromatic heterocyclic group, especially preferably a C₃₋₆ nonaromatic heterocyclic group. Examples of the divalent nonaromatic heterocyclic group include pyrroldionediyl, pyrrolinedionediyl, oxiranediyl, aziridinediyl, azetidinediyl, oxetanediyl, thietanediyl, pyrrolidinediyl, dihydrofurandiyl, tetrahydrofurandiyl, dioxolanediyl, tetrahydrothiophenediyl, pyrrolinediyl, imidazolidinediyl, oxazolidinediyl, piperidinediyl, dihydropyrandiyl, tetrahydropyrandiyl, tetrahydrothiopyrandiyl, morpholinediyl, thiomorpholinediyl, piperazinediyl, dihydrooxazinediyl, tetrahydrooxazinediyl, dihydropyrimidinediyl, and tetrahydropyrimidinediyl.

The divalent heterocyclic group is preferably a divalent aromatic heterocyclic group.

Examples of the substituent include the following:
(i) a halogen atom;
(ii) a monovalent hydrocarbon group;
(iii) a monovalent heterocyclic group;
(iv) aralkyl;
(v) Rₐ-O-, Rₐ-C(=O)-, Rₐ-O-C(=O)-, or Rₐ-C(=O)-O- (Rₐ represents a hydrogen atom or a monovalent hydrocarbon group); or
(vi) NR_{b}Rc-, NR_{b}R_{c}-C(=O)-, NR_{b}R_{c}-C(=O)-O-, or R_{b}-C(=O)-NR_{c}- (R_{b} and R_{c} are the same or different from each other, and each represents a hydrogen atom or a monovalent hydrocarbon group);
(vii) a nitro group, a sulfate group, a sulfonate group, a cyano group, and a carboxyl group.

The definitions, examples, and preferred examples of the halogen atom, the monovalent hydrocarbon group, and the monovalent heterocyclic group in the substituent are the same as those described above.

The aralkyl refers to arylalkyl. The definitions, examples, and preferred examples of the aryl and the alkyl in the arylalkyl are as described above. The aralkyl is preferably C₃₋₁₅ aralkyl. Examples of such an aralkyl include benzoyl, phenethyl, naphthylmethyl, and naphthylethyl.

As the linker, for example, one of, or a combination of two or more of, divalent groups derived from a substance selected from amino acids, peptides, nucleic acids, sugars, and other polymeric substances (for example, polyethylene glycol) may be used, or these may also be used in combination with the above-described divalent groups.

The total number of atoms constituting the backbone in the linker is not particularly limited, and may be, for example, not less than 1, not less than 2, not less than 5, not less than 10, or not less than 20. The total number of such atoms may be not more than 1000, not more than 500, not more than 300, not more than 200, or not more than 100.

The linker may be a chemical structure of the portion connecting the Fc to the cell surface, the portion being generated by reaction between a bioorthogonal functional group introduced into a particular amino acid residue of one of the polypeptides of the Fc region, and a group that is present on or introduced to the cell surface and reacts with the bioorthogonal functional group, more specifically, a chemical structure of the portion connecting a functional group in the side chain of a particular amino acid residue present in an Fc region-constituting polypeptide to a functional group on the cell surface.

### <Modification of the Other Polypeptide>

The antibody Fc region may contain a functional substance introduced via a linker that is introduced into a functional group in the side chain of a particular amino acid residue present at one or more positions of the other of the two Fc region-constituting polypeptides, that is, the polypeptide not involved in cell binding.

### (Functional Substance)

The functional substance is not particularly limited as long as it is a substance that imparts any function to the antibody, and examples of the functional substance include drugs, labeling substances, affinity substances, transporting substances, and stabilizers. Preferably, the functional substance may be a drug, a labeling substance, an affinity substance, or a transporting substance. The functional substance may be a single functional substance, or a substance in which two or more functional substances are linked.

The drug may be a drug for any disease. Examples of such a disease include cancers (such as lung cancer, stomach cancer, colon cancer, pancreatic cancer, renal cancer, liver cancer, thyroid cancer, prostatic cancer, bladder cancer, ovarian cancer, uterine cancer, bone cancer, skin cancer, brain tumor, and melanoma), autoimmune diseases and inflammatory diseases (such as allergic diseases, rheumatoid arthritis, and systemic lupus erythematosus), brain or nerve diseases (such as cerebral infarction, Alzheimer's disease, Parkinson's disease, and amyotrophic lateral sclerosis), infections (such as bacterial infections and viral infections), hereditary or rare diseases (such as hereditary spherocytosis and nondystrophic myotonia), eye diseases (such as age-related macular degeneration, diabetic retinopathy, and retinitis pigmentosa), diseases in the bone or orthopedic field (such as osteoarthritis), blood diseases (such as leukemia and purpura), and other diseases (such as diabetes, metabolic abnormalities including hyperlipidemia, liver diseases, kidney diseases, lung diseases, circulatory system diseases, and digestive system diseases). The drug may be a prophylactic or therapeutic agent for a disease, or an agent for alleviating side effects.

More specifically, the drug may be an anti-cancer agent. Examples of the anti-cancer agent include chemotherapeutic agents, toxins, and radioisotopes, and substances comprising them. Examples of the chemotherapeutic agents include DNA-damaging agents, antimetabolites, enzyme inhibitors, DNA intercalating agents, DNA cleaving agents, topoisomerase inhibitors, DNA-binding inhibitors, tubulin-binding inhibitors, cytotoxic nucleosides, and platinum compounds. Examples of the toxins include bacteriotoxins (such as diphtheria toxin) and phytotoxins (such as ricin). Examples of the radioisotopes include radioisotopes of a hydrogen atom (such as ³H), radioisotopes of a carbon atom (such as ¹⁴C), radioisotopes of a phosphorus atom (such as ³²P), radioisotopes of a sulfur atom (such as ³⁵S), radioisotopes of yttrium (such as ⁹⁰Y), radioisotopes of technetium (such as ^{99m}Tc), radioisotopes of indium (such as ¹¹¹In), radioisotopes of an iodine atom (such as ¹²³I, ¹²⁵I, ¹²⁹I, and ¹³¹I), radioisotopes of samarium (such as ¹⁵³Sm), radioisotopes of rhenium (such as ¹⁸⁶Re), radioisotopes of astatine (such as ²¹¹At), and radioisotopes of bismuth (such as ²¹²Bi). More specific examples of the drug include auristatin (MMAE, MMAF), maytansine (DM1, DM4), PBD (pyrrolobenzodiazepine), IGN, camptothecin analogs, calicheamicin, duocarmycin, eribulin, anthracycline, dmDNA31, and tubulysin.

The labeling substance is a substance that enables detection of a target (such as a tissue, a cell, or a substance). Examples of the labeling substance include enzymes (such as peroxidase, alkaline phosphatase, luciferase, and β-galactosidase), affinity substances (such as streptavidin, biotin, digoxigenin, and aptamers), fluorescent substances (such as fluorescein, fluorescein isothiocyanate, rhodamine, green fluorescent protein, and red fluorescent protein), luminescent substances (such as luciferin, aequorin, acridinium esters, tris(2,2'-bipyridyl)ruthenium, and luminol), and radioisotopes (such as those described above), or substances comprising them.

The affinity substance is a substance having affinity for a target. Examples of the affinity substances include affinity proteins or peptides such as antibodies; aptamers; lectins; and complementary strands of target nucleic acids. The affinity substances are preferably affinity proteins or affinity peptides, and may be antibodies. The type of the animal from which the antibody used as the functional substance is derived is as described above. Examples of the antibody used as the functional substance include a full-length antibody and a fragment thereof (fragment antibody). The fragment antibody is not limited as long as it maintains a binding ability to a desired antigen, and examples of the fragment antibody include Fab, Fab', F(ab')₂, scFv, and VHH antibody.

The transporting substance is a substance having an ability to transport a compound. The transporting substance is preferably a substance (for example, ferritin, a virus particle, or a virus-like particle) capable of encapsulating the compound in a protein shell (such as a multimer).

The stabilizer is a substance that enable stabilization of an antibody. Examples of the stabilizers include diols, glycerin, nonionic surfactants, anionic surfactants, natural surfactants, saccharides, and polyols.

The functional substance may also be a peptide, a protein, a nucleic acid, an organic compound, an inorganic compound, a sugar chain, a lipid, a high molecular weight polymer, a metal (such as gold), or a chelator. Examples of the peptide include cell membrane permeable peptides, blood-brain barrier permeable peptides, and peptide pharmaceuticals. Examples of the protein include enzymes, cytokines, fragment antibodies, lectins, interferons, serum albumin, antibodies, and ferritin. Examples of the nucleic acid include DNA, RNA, and artificial nucleic acids. Examples of the nucleic acid also include RNA interference-inducing nucleic acids (such as siRNAs), aptamers, and antisense nucleic acids. Examples of the organic compound include low molecular weight organic compounds such as proteolysis-inducing chimeras, dyes, and photodegradable compounds. Examples of the inorganic compound include silica, talc, and alumina.

In the Fc-constituting polypeptide not involved in cell binding, the particular amino acid residue to which the functional substance binds may be an amino acid residue described above such as a lysine residue.

The functional substance is introduced into the particular amino acid residue via a linker.

The linker that connects the functional substance to the Fc may be a divalent group, and the divalent groups described above may be used as the linker. In cases where the divalent group is substituted, examples of the substituent include those described above. Here, the total number of atoms constituting the backbone in the linker is not particularly limited, and may be, for example, not less than 1, not less than 2, not less than 5, not less than 10, or not less than 20. The total number of such atoms may be not more than 1000, not more than 500, not more than 300, not more than 200, not more than 100, or not more than 50.

The linker may be a chemical structure of the portion connecting the Fc to the functional substance, the portion being generated by reaction between a bioorthogonal functional group introduced into a particular amino acid residue of the polypeptide not involved in cell binding of the Fc region, and a group that is linked to the functional substance and reacts with the bioorthogonal functional group, more specifically, a chemical structure of the portion connecting a functional group in the side chain of a particular amino acid residue present in the Fc region-constituting polypeptide to the functional substance.

### <Uses of Fc-Cell Complex>

The Fc-cell complex can be used in various applications.

In cases where the Fc-cell complex is an antibody-cell complex, for example, in cases where the antibody is an antibody against a surface antigen of a target tissue, the Fc-cell complex can be used as a cellular medicine capable of delivering a cell to the target tissue.

Further, in a case where the Fc-cell complex is an antibody-cell complex, where the antibody is an antibody against a cancer cell-specific surface antigen, and where the cell is an NK cell, a T cell, or the like, the Fc-cell complex can be used as a cellular medicine having a cytotoxic effect against cancer cells.

In a case where the Fc-cell complex is a cell-Fc enzyme fusion protein complex, the Fc-cell complex can also be used for performing an enzyme reaction on the cell surface to produce a substance.

### <Method of Producing Fc-Cell Complex>

The method of producing an Fc-cell complex of the present invention comprises the following Steps (I) to (V):
(I) preparing an antibody Fc region formed by disulfide bonding of two antibody Fc region-constituting polypeptides;
(II) reacting the antibody Fc region with a compound represented by the following Formula (Ia) or Formula (Ib) to form an Fc-Compound (Ia) complex or an Fc-Compound (Ib) complex;
(III) subjecting the Fc-Compound (Ia) complex or the Fc-Compound (Ib) complex to cleavage treatment at the cleavable portion, to form an Fc region that does not comprise the affinity substance and to which the bioorthogonal functional group is introduced;
(IV) preparing a cell having, on a surface thereof, a functional group that reacts with the bioorthogonal functional group; and
(V) introducing the Fc region to the cell surface by reacting the Fc region, to which the bioorthogonal functional group is introduced, with the cell, through reaction of the bioorthogonal functional group of the Fc region with the functional group of the cell that reacts with the bioorthogonal functional group.

### Each step is described below.

### <Step (I): Step of Providing Antibody Fc Region>

The antibody Fc region is as described above. The Fc region may be an antibody further containing F(ab')₂, that is, an immunoglobulin, or the Fc region may be an Fc fusion protein further containing a functional polypeptide.

Such an antibody or Fc fusion protein may be a commercially available product, or may be one prepared by a known method.

For example, the antibody or Fc fusion protein may be recombinantly produced.

<Step (II): Step of Reacting the Antibody Fc Region with a Compound Represented by Formula (Ia) or Formula (Ib) (Also Referred to as Compound (Ia) or Compound (Ib)) to Form an Fc-Compound (Ia) Complex or an Fc-Compound (Ib) Complex>

First, components of Formula (Ia) or Formula (Ib) are described.

### <Affinity Substance A>

Affinity substance A is an affinity substance containing a peptide having affinity for a constant region of a heavy chain of an antibody. Here, the peptide having affinity for a constant region of a heavy chain of an antibody may be selected from the affinity peptide containing a lysine residue and the affinity peptide containing no lysine residue described later.

Affinity substance A may be an affinity substance represented by AP1-L_{A}-AP2, wherein AP1 represents a first affinity peptide having affinity for a constant region of a heavy chain of an antibody, AP2 represents a second affinity peptide having affinity for a constant region of a heavy chain of an antibody, and L_{A} represents a linker.

In Formula (A) and other formulae presented in relation to the present invention, - (hyphen) indicates that two units present on both sides thereof are covalently bound to each other. Thus, in Formula (A), AP1 is covalently bound to L; L is covalently bound to both AP1 and AP2; and AP2 is covalently bound to L.

The first and second affinity peptides are not particularly limited as long as they are peptides having affinity for a constant region of a heavy chain of an antibody, and may be selected from an affinity peptide containing a lysine residue and an affinity peptide containing no lysine residue. Preferably, one of the first and second affinity peptides may be an affinity peptide containing one lysine residue, and the other may be an affinity peptide containing no lysine residue.

A large number of peptides have been reported as affinity peptides having affinity for a constant region of a heavy chain of an antibody and containing one lysine residue (see, for example, WO2016/186206, WO2018/199337, WO2019/240287, WO2019/240288, and WO2020/090979). Thus, in the present invention, such peptides can be used as one of the first and second affinity peptides.

More specifically, examples of affinity peptides having affinity for a constant region of a heavy chain of an antibody and containing one lysine residue include the following:
affinity peptides containing the amino acid sequences of SEQ ID NOs: 39 to 72 of WO2018/199337;
affinity peptides containing the amino acid sequences of SEQ ID NOs: 5, 6, and 37 to 100 of WO2019/240288;
affinity peptides containing the amino acid sequences of SEQ ID NOs: 5, 8 to 57, and 68 to 92 of WO2019/240287;
affinity peptides having QET at the N-terminus (SEQ ID NOs: 7 to 10, 22 to 25, 52, and 53 of WO2020/090979); and
affinity peptides containing one lysine residue, among the following affinity peptides (1) to (12):
   (1) various IgG-binding peptides having affinity for a particular region (CH2 domain) of human IgG in general (that is, human IgG1, IgG2, IgG3, and IgG4; the same applies hereinafter) (see, for example, WO2008/054030, WO2013/027796, and WO2016/186206);
   (2) Protein A Mimetic (PAM) peptide having affinity for a particular region (CH2 domain) of human IgG in general (see, for example, Fassina G et al., JOURNAL OF MOLECULAR RECOGNITION, 1996, VOL. 6, 564-569);
   (3) EPIHRSTLTALL (SEQ ID NO: 1) having affinity for a particular region (CH2 domain) of human IgG in general (see, for example, Ehrlich G. K et al., J. Biochem. Biophys. Methods, 2001, VOL. 49, 443-454);
   (4) (NH₂-Cys1-X1-X2-X3-X4)2-Lys-Gly-OH having affinity for a particular region (Fc region) of human IgG in general (see, for example, Ruvo M et al., ChemBioChem, 2005, VOL. 6, 1242-1253);
   (5) FARLVSSIRY (SEQ ID NO: 2), FGRLVSSIRY (SEQ ID NO: 3), and TWKTSRISIF (SEQ ID NO: 4) having affinity for a particular region (Fc region) of human IgG in general (see, for example, Krook M et al., Journal of Immunological Methods, 1998, VOL. 221, 151-157);
   (6) QSYP (SEQ ID NO: 5) having affinity for a particular region of human IgG in general (see, for example, Jacobs J. M. et al., BioTechniques, 2003, VOL. 34, 132-141);
   (7) HWRGWV (SEQ ID NO: 6), HYFKFD (SEQ ID NO: 7), and HFRRHL (SEQ ID NO: 8) having affinity for a particular region (Fc region) of human IgG in general (see, for example, Carbonell R. G. et al., Journal of Chromatography A, 2009, VOL. 1216, 910-918);
   (8) DAAG (SEQ ID NO: 9) having affinity for a particular region (Fc region) of human IgG in general (see, for example, Lund L. N. et al., Journal of Chromatography A, 2012, VOL. 1225, 158-167);
   (9) Fc-I, Fc-II, and Fc-III having affinity for a particular region (Fc region) of human IgG in general (see, for example, Warren L. Delano et al., Science, 2000, VOL. 287, 1279-1283; and WO2001/045746); and
   (10) NARKFYKG (SEQ ID NO: 10) and NKFRGKYK (SEQ ID NO: 11) having affinity for a particular region (Fc region) of human IgG in general (see, for example, Biochemical Engineering Journal, 2013, VOL. 79, 33-40);
   (11) Protein A, Protein G, Protein L or Protein Z, or fragments thereof, having affinity for a particular region (Fc region) of human IgG in general (for example, Moks T et al., Eur J Biochem. 1986 May 2; 156(3):637-43; Sjobring UJ et al., Biol Chem. 1991 Jan5; 266(1): 399-405; Graille M et al., Structure. 2001 Aug; 9(8): 679-87; Nilsson B et al., Protein Eng. 1987 Feb-Mar; 1(2): 107-13));
   (12) various IgG-binding peptides having affinity for a particular region (Fc region or CH2 domain) of human IgG in general (see, for example, WO2018/199337, WO2019/240287, WO2019/240288, and WO2020/090979).

In a particular embodiment, examples of the affinity peptide having affinity for a constant region of a heavy chain of an antibody and containing one lysine residue include the following (1) to (4):
(1) an affinity peptide containing the amino acid sequence (Fc3K) of RGNCAYHKGQIIWCTYH (SEQ ID NO: 12);
(2) an affinity peptide containing an amino acid sequence that is the same as the amino acid sequence of RGNCAYHKGQIIWCTYH (SEQ ID NO: 12) except that one or two amino acid residues other than a lysine residue and a cysteine residue are substituted with other amino acid residues other than a lysine residue and a cysteine residue, and having affinity for a constant region of a heavy chain of an antibody;
(3) an affinity peptide containing the amino acid sequence (Z34CK) of FNKQCQRRFYEALHDPNLNEEQRNARIRSIREEC (SEQ ID NO: 13); and
(4) an affinity peptide containing an amino acid sequence that is the same as the amino acid sequence of FNKQCQRRFYEALHDPNLNEEQRNARIRSIREEC (SEQ ID NO: 13) except that one or two amino acid residues other than a lysine residue and a cysteine residue are substituted with other amino acid residues other than a lysine residue and a cysteine residue, and having affinity for a constant region of a heavy chain of an antibody.

Here, the two cysteine residues contained in the amino acid sequences described above may be cross-linked by disulfide bonding.

Many peptides have been reported as affinity peptides having affinity for a constant region of a heavy chain of an antibody and containing no lysine residue. Moreover, in many cases, in the above affinity peptides having affinity for a constant region of a heavy chain of an antibody and containing one lysine residue, the lysine residue is introduced not for maintaining the affinity for a constant region of a heavy chain of an antibody, but for derivatizing the affinity substance by covalent bonding to another portion (such as a partial compound containing a reactive group) (see, for example, WO2016/186206, WO2018/199337, WO2019/240287, WO2019/240288, and WO2020/090979). Therefore, even if such a lysine residue is substituted with another amino acid residue, the affinity for a constant region of a heavy chain of an antibody can be maintained. Thus, the affinity peptide having affinity for a constant region of a heavy chain of an antibody and containing no lysine residue may be the same as the affinity peptide having affinity for a constant region of a heavy chain of an antibody and containing one lysine residue except that the lysine residue is substituted with another amino acid residue, preferably a normal natural amino acid residue constituting a protein other than a lysine residue and a cysteine residue, as long as the affinity for a constant region of a heavy chain of an antibody is retained.

More specifically, as the affinity peptide having affinity for a constant region of a heavy chain of an antibody and containing no lysine residue, the following may be used:
SEQ ID NOs: 20 to 38, 73 to 75 (when Xaa1 is not a lysine residue), and SEQ ID NO: 92 of WO2018/199337;
SEQ ID NOs: 7, 11 to 14, and 108 of WO2019/240288;
affinity peptides that are the same as those in (1) to (4) listed above as examples of affinity peptides having affinity for a constant region of a heavy chain of an antibody and containing one lysine residue, except that the lysine residue is substituted with another amino acid residue, preferably an amino acid residue other than a cysteine residue; and
affinity peptides containing no lysine residue among the above-exemplified affinity peptides of (1) to (12).

In a particular embodiment, examples of the affinity peptide containing no lysine residue include the following (5) to (10):
(5) an affinity peptide containing the amino acid sequence (Z34CM) of FNMQCQRRFYEALHDPNLNEEQRNARIRSIREEC (SEQ ID NO: 14);
(6) an affinity peptide containing an amino acid sequence that is the same as the amino acid sequence of FNMQCQRRFYEALHDPNLNEEQRNARIRSIREEC (SEQ ID NO: 14) except that one or two amino acid residues other than a cysteine residue are substituted with other amino acid residues other than a lysine residue and a cysteine residue, and having affinity for a constant region of a heavy chain of an antibody;
(7) an affinity peptide containing the amino acid sequence (ProAR) of FNREQQNAFYEILHLPNLNEEQRNGFIQSLRDDPSQSANLLAEA (SEQ ID NO: 15);
(8) an affinity peptide containing an amino acid sequence that is the same as the amino acid sequence of FNREQQNAFYEILHLPNLNEEQRNGFIQSLRDDPSQSANLLAEA (SEQ ID NO: 15) except that one or two amino acid residues other than a cysteine residue are substituted with other amino acid residues other than a lysine residue and a cysteine residue, and having affinity for a constant region of a heavy chain of an antibody;
(9) an affinity peptide containing the amino acid sequence of RGNCAYHRGQIIWCTYH (SEQ ID NO: 16); and
(10) an affinity peptide containing an amino acid sequence that is the same as the amino acid sequence of RGNCAYHRGQIIWCTYH (SEQ ID NO: 16) except that one or two amino acid residues other than a cysteine residue are substituted with other amino acid residues other than a lysine residue and a cysteine residue, and having affinity for a constant region of a heavy chain of an antibody.

Here, the two cysteine residues contained in the amino acid sequences described above may be cross-linked by disulfide bonding.

The substitution of each amino acid residue may be a conservative substitution. The term "conservative substitution" refers to substitution of a given amino acid residue with an amino acid residue containing a similar side chain. Families of amino acid residues containing similar side chains are well known in the art. Examples of such families include amino acids with basic side chains (such as lysine, arginine, and histidine), amino acids with acidic side chains (such as aspartic acid and glutamic acid), amino acids with uncharged polar side chains (such as asparagine, glutamine, serine, threonine, tyrosine, and cysteine), amino acids with nonpolar side chains (such as glycine, alanine, valine, leucine, isoleucine, proline, phenylalanine, methionine, and tryptophan), amino acids with beta-branched side chains (such as threonine, valine, and isoleucine), amino acids with aromatic side chains (such as tyrosine, phenylalanine, tryptophan, and histidine), amino acids with side chains containing a hydroxyl group (for example, alcoholic or phenolic hydroxyl group) (such as serine, threonine, and tyrosine), and amino acids with sulfur-containing side chains (such as cysteine and methionine). Amino acids with uncharged polar side chains and amino acids with nonpolar side chains may be collectively referred to as neutral amino acids. Preferably, the conservative substitution of an amino acid may be substitution between aspartic acid and glutamic acid; substitution among arginine, lysine, and histidine; substitution between tryptophan and phenylalanine; substitution between phenylalanine and valine; substitution among leucine, isoleucine, and alanine; or substitution between glycine and alanine.

The linker represented by L_{A} is a divalent group. The divalent group may be substituted or unsubstituted. Examples of the divalent group include those described above.

In cases where the divalent group is substituted, examples of the substituent include those described above. Here, the total number of atoms constituting the backbone in the linker may be 2 to 10. The total number of such atoms may be not less than 3, or not less than 4. The total number of such atoms may be not more than 9, not more than 8, or not more than 7. More specifically, the total number of such atoms may be 3 to 9, 4 to 8, or 4 to 7.

Examples of the linker include a substance such as a peptide, a nucleic acid, a sugar, another polymeric substance (such as polyethylene glycol), or a divalent hydrocarbon group (such as an alkyl chain). A peptide linker is more preferred.

The number of amino acid residues constituting the peptide linker may be appropriately set according to conditions such as the type of amino acid residue (such as α-amino acid, β-amino acid, and γ-amino acid, preferably α-amino acid). For example, the peptide linker may be composed of not less than 20 amino acid residues. The peptide linker may be composed of not less than 22, not less than 24, not less than 26, not less than 28, not less than 30, not less than 32, not less than 34, not less than 36, not less than 38, or not less than 40 amino acid residues. Further, the peptide linker may be composed of less than 50, less than 49, less than 48, less than 47, less than 46, or less than 45 amino acid residues.

As the amino acid residues constituting the peptide linker, residues of the above-described natural amino acids or non-natural amino acids may be used. Preferably, the amino acid residues constituting the peptide linker may contain residues of only natural amino acids described above. Examples of amino acid residues suitable for the peptide linker are not particularly limited, and may include alanine, proline, serine, and glycine. As the peptide linker, those disclosed in WO2021/112249 and WO2011/144756 may also be used.

### <Reactive Group R for Antibody>

As the reactive group for the antibody, a reactive group for an amino acid residue containing an easily reactive side chain among the amino acid residues constituting the antibody (protein) may be used. Of the above-described 20 natural amino acids constituting proteins, glycine, which contains no side chain, and alanine, isoleucine, leucine, phenylalanine, and valine, whose side chains are hydrocarbon groups, are inert to ordinary reactions. Therefore, the reactive group for the antibody is a group that can react with the side chain of one or more (for example, two, three, or four) of the following 14 amino acids: asparagine, glutamine, methionine, proline, serine, threonine, tryptophan, tyrosine, aspartic acid, glutamic acid, arginine, histidine, and lysine.

The reactive group for the antibody is more preferably a reactive group specific for the side chain of any one of the amino acids lysine, tyrosine, tryptophan, and cysteine residues, still more preferably a reactive group specific for the side chain of any one of the amino acids lysine, tyrosine, and tryptophan residues, especially preferably a reactive group specific for the side chain of lysine or tyrosine residues, in particular, for the side chain of lysine residue. For details of such reactive groups, see, for example, WO2016/186206, WO2018/199337, WO2019/240287, WO2019/240288, and WO2020/090979.

The reactive group specific for the side chain of a lysine residue is a group that can react specifically with the amino group (NH₂) present in the side chain of a lysine residue, and examples of such a reactive group include an activated ester residue (such as an N-hydroxysuccinimide residue), a vinylsulfone residue, a sulfonyl chloride residue, an isocyanate residue, an isothiocyanate residue, an aldehyde residue, a 1,4,7,10-tetraazacyclododecane-1,4,7,10-tetraacetic acid residue, a 2-imino-2-methoxyethyl residue, a diazonium terephthalic acid residue, an α-halogen-substituted acetamide, and an α-halogen-substituted methylketone. By the reaction between the above-described reactive group specific for the side chain of a lysine residue and the amino group (NH₂) present in the side chain of the lysine residue, for example, an amide residue, a urea residue, a pyridine residue, a carbamate residue, or a sulfonamide residue can be generated as a linking portion.

### Group Containing Bioorthogonal Functional Group, B

The term "bioorthogonal functional group" refers to a group that does not react with, or reacts only slowly with, biological components (such as amino acids, proteins, nucleic acids, lipids, sugars, and phosphates), but that reacts selectively with a non-biological component. Bioorthogonal functional groups are well known in the art (see, for example, Sharpless K. B. et al., Angew. Chem. Int. Ed. 40, 2004 (2015); Bertozzi C. R. et al., Science 291, 2357 (2001); Bertozzi C. R. et al., Nature Chemical Biology 1, 13 (2005)).

A bioorthogonal functional group with respect to proteins is a group that does not react with, or reacts only slowly with, the side chains of the 20 naturally-occurring amino acid residues constituting proteins, but that reacts with a target functional group. The 20 naturally-occurring amino acids constituting proteins are alanine (A), asparagine (N), cysteine (C), glutamine (Q), glycine (G), isoleucine (I), leucine (L), methionine (M), phenylalanine (F), proline (P), serine (S), threonine (T), tryptophan (W), tyrosine (Y), valine (V), aspartic acid (D), glutamic acid (E), arginine (R), histidine (H), and lysine (L). Of these 20 naturally-occurring amino acids, glycine, which contains no side chain (in other words, contains a hydrogen atom as the side chain); and alanine, isoleucine, leucine, phenylalanine, and valine, which contain a hydrocarbon group as the side chain (in other words, whose side chain does not contain any heteroatom selected from the group consisting of a sulfur atom, a nitrogen atom, and an oxygen atom); are inert to typical reactions. Thus, the bioorthogonal functional group with respect to proteins is a group that does not react with, or reacts only slowly with, the side chains of these amino acids, whose side chains are inert to typical reactions, as well as the side chains of asparagine, glutamine, methionine, proline, serine, threonine, tryptophan, tyrosine, aspartic acid, glutamic acid, arginine, histidine, and lysine, while the bioorthogonal functional group reacts with a target functional group.

Examples of such a bioorthogonal functional group include an azide group, an aldehyde residue, a thiol group, an alkene residue (in other words, the residue may contain a vinylene (ethenylene) portion, which is a minimum unit having a carbon-carbon double bond; the same applies hereinafter), an alkyne residue (in other words, the residue may contain an ethynylene portion, which is a minimum unit having a carbon-carbon triple bond; the same applies hereinafter), a halogen residue, a tetrazine residue, a nitrone residue, a hydroxylamine residue, a nitrile residue, a hydrazine residue, a ketone residue, a boronic acid residue, a cyanobenzothiazole residue, an allyl residue, a phosphine residue, a maleimide residue, a disulfide residue, a thioester residue, an α-halocarbonyl residue (such as a carbonyl residue containing a fluorine atom, a chlorine atom, a bromine atom, or an iodine atom at the alpha position; the same applies hereinafter), an isonitrile residue, a sydnone residue, and a selenium residue. Examples of groups containing an alkyne residue (that is, an ethynylene portion) include dibenzocyclooctyne (DBCO) and diazacyclononyne (DACN).

The bioorthogonal functional group may or may not be protected. The bioorthogonal functional group refers to an unprotected bioorthogonal functional group or a protected bioorthogonal functional group. The unprotected bioorthogonal functional group corresponds to the bioorthogonal functional group described above. The protected bioorthogonal functional group is a group that generates a bioorthogonal functional group by cleavage of a protecting group. The protecting group can be cleaved by a particular treatment under conditions that cannot cause protein denaturation or degradation (for example, that cannot cause cleavage of an amide bond) (mild conditions). Examples of such a particular treatment include: (a) treatment with one or more substances selected from the group consisting of an acidic substance, a basic substance, a reducing agent, an oxidizing agent, and an enzyme; (b) treatment by physicochemical stimulation selected from the group consisting of light; or (c) being left to stand in a case where a cleavable linker containing a self-degradable cleavable portion is used. Such a protecting group and the conditions for cleaving it are common technical knowledge in the art (for example, G. Leriche, L. Chisholm, A. Wagner, Bioorganic & Medicinal Chemistry. 20, 571 (2012); Feng P. et al., Journal of the American Chemical Society. 132, 1500 (2010); Bessodes M. et al., Journal of Controlled Release, 99, 423 (2004); DeSimone, J. M., Journal of American Chemical Society. 132, 17928 (2010); Thompson, D. H., Journal of Controlled Release, 91, 187 (2003); Schoenmarks, R. G., Journal of Controlled Release, 95, 291 (2004)). Reaction conditions (such as the reaction temperature, the reaction time, and the reaction solution) for the mild conditions are as described later.

Examples of the protected bioorthogonal functional group include a disulfide residue, an ester residue, an acetal residue, a ketal residue, an imine residue, and a vicinal diol residue.

Preferably, the bioorthogonal functional group is an unprotected bioorthogonal functional group.

More preferably, the bioorthogonal functional group may be a particular bioorthogonal functional group having excellent reactivity with another bioorthogonal functional group (regarding the reaction level and/or the reaction specificity). Examples of such a bioorthogonal functional group include an azide group, an alkyne residue, preferably a cyclic group having a carbon-carbon triple bond, which may be substituted with a substituent as described above, a tetrazine residue, an alkene residue, a thiol group, a maleimide residue, a furan residue, and a halocarbonyl residue.

The group containing a bioorthogonal functional group, represented by B, may be a group consisting of a bioorthogonal functional group, or may be a group containing a bioorthogonal functional group and another portion. Examples of the other portion include a linking portion between the bioorthogonal functional group and a linker. The linking portion is, for example, a divalent group. The divalent group may be substituted or unsubstituted. The definitions, examples, and preferred examples of the bioorthogonal functional group, the divalent group, and the substituent (when the divalent group is substituted) are as described above.

### <Cleavable Portion CLE>

The cleavable portion is a site that can be cleaved by a particular treatment under conditions that cannot cause protein denaturation or degradation (for example, that cannot cause cleavage of an amide bond) (mild conditions). Thus, the cleavable portion can be said to be a site (a bond other than an amide bond) that can be cleaved by a particular cleavage treatment under mild conditions. Examples of such a particular treatment include: (a) treatment with one or more substances selected from the group consisting of an acidic substance, a basic substance, a reducing agent, an oxidizing agent, and an enzyme; (b) treatment by physicochemical stimulation such as light; or (c) being left to stand (incubation) in a case where a cleavable linker containing a self-degradable cleavable portion is used. Such a cleavable linker and the conditions for cleaving it are common technical knowledge in the art (for example, G. Leriche, L. Chisholm, A. Wagner, Bioorganic & Medicinal Chemistry. 20, 571 (2012); Feng P. et al., Journal of American Chemical Society. 132, 1500 (2010); Bessodes M. et al., Journal of Controlled Release, 99, 423 (2004); DeSimone, J. M., Journal of American Chemical Society. 132, 17928 (2010); Thompson, D. H., Journal of Controlled Release, 91, 187 (2003); Schoenmarks, R. G., Journal of Controlled Release, 95, 291 (2004)). Reaction conditions (such as the reaction temperature, the reaction time, and the reaction solution) for the mild conditions are as described later. Examples of the cleavable portion described above include a disulfide residue, an acetal residue, a ketal residue, an ester residue, a carbamoyl residue, an alkoxyalkyl residue, an imine residue, a tertiary alkyloxycarbamate residue (such as a tert-butyloxycarbamate residue), a silane residue, a hydrazone-containing residue (such as a hydrazone residue, an acylhydrazone residue, and a bisarylhydrazone residue), a phosphoramidate residue, an aconityl residue, a trityl residue, an azo residue, a vicinal diol residue, a selenium residue, an aromatic ring-containing residue containing an electron-withdrawing group, a coumarin-containing residue, a sulfone-containing residue, an unsaturated bond-containing chain residue, and a glycosyl residue.

The aromatic ring group containing an electron-withdrawing group is preferably one containing an aromatic ring group, selected from the group consisting of aryl, aralkyl, an aromatic heterocyclic group, and alkyl containing an aromatic heterocyclic group. Aralkyl, or alkyl containing an aromatic heterocyclic group is more preferred. The electron-withdrawing group is preferably bound to the 2-position of the ring. Still more preferably, the aromatic ring-containing residue containing an electron-withdrawing group is, for example, aralkyl (such as benzyl) containing an electron-withdrawing group at the 2-position. Examples of the electron-withdrawing group include a halogen atom, an alkyl substituted with a halogen atom (such as trifluoromethyl), a boronic acid residue, mesyl, tosyl, triflate, nitro, cyano, a phenyl group, and a keto group (such as acyl).

Examples of the ester residue include ordinary ester residues composed of carbon atoms and oxygen atoms [for example, alkyl esters (for example, tertiary alkyloxycarbonyls such as tert-butyloxycarbonyl), aryl esters (for example, phenacyl ester and 2-(diphenylphosphino)benzoate), glycosyl ester residues, and orthoester residues], ester residues containing a sulfur atom and an oxygen atom (for example, thioester residues such as an α-thiophenyl ester residue and an alkylthioester residue), ester residues containing a phosphorus atom and an oxygen atom (for example, a phosphodiester residue and a phosphotriester residue), and activated ester residues (for example, an N-hydroxysuccinimide residue).

Examples of the sulfone-containing residue include sulfone residues and quinolinyl benzenesulfonate residues.

The silane residue is preferably a silane residue containing a group selected from the group consisting of alkyl, aryl, aralkyl, and alkoxy. Examples of such a silane residue include dialkyldialkoxysilane residues (for example, dimethyldialkoxysilane and diethyldialkoxysilane) or diaryldialkoxysilane residues (for example, diphenyldialkoxysilane).

The alkoxyalkyl (that is, alkyloxyalkyl) residue is a group in which alkyloxy and alkyl as described above are combined, and examples of the alkoxyalkyl residue are not particularly limited, and may include a methoxymethyl residue, an ethoxymethyl residue, a methoxyethyl residue, and an ethoxyethyl residue.

The unsaturated bond-containing chain residue is a residue containing an unsaturated bond portion composed only of carbon atoms [for example, vinyl (ethenyl), which is a minimum unit having a carbon-carbon double bond, or acetylenyl (ethynyl), which is a minimum unit having a carbon-carbon triple bond], or a residue containing an unsaturated bond portion (for example, aldehyde or cyano) composed of a carbon atom and a heteroatom (for example, a nitrogen atom, a sulfur atom, or an oxygen atom). Examples of the unsaturated bond-containing chain residue include a vinyl ether residue, a cyanoethyl residue, an ethylene residue, and a malondialdehyde residue.

Examples of the acidic substance (also referred to as electrophilic reagent) include inorganic acidic substances such as hydrochloric acid, sulfuric acid, and nitric acid; and organic acidic substances such as formic acid, acetic acid, 4-(2-hydroxyethyl)-1-piperazinepropanesulfonic acid, 3-morpholinopropanesulfonic acid, sodium dihydrogen phosphate, citric acid, dodecylsulfuric acid, N-dodecanoylsarcosinic acid, and trifluoroacetic acid. Examples of the site cleavable by the acidic substance include an alkyloxyarylalkyl residue, a tertiary alkyloxycarbamate residue, an acetal residue, a silane residue, an imine residue, a vinyl ether residue, a β-thiopropionate residue, a trityl residue, a hydrazone residue, an aconityl residue, an orthoester residue, a carbamoyl residue, and a 2-(diphenylphosphino)benzoate residue.

Examples of the basic substance (also referred to as nucleophilic reagent) include inorganic basic substances such as sodium hydroxide, potassium hydroxide, sodium acetate, potassium acetate, and ammonium acetate; and organic basic substances such as hydroxylamine, triethylamine, and N,N'-diisopropylamine. Examples of the site cleavable by the basic substance include a silane residue, a cyanoethyl residue, a sulfone residue, an ethylene residue, a glycosyl disuccinate residue, an α-thiophenyl ester residue, an unsaturated vinyl sulfide residue, a malondialdehyde residue, an acylhydrazone residue, and an alkylthioester residue.

Examples of the reducing agent include cysteine, dithiothreitol, reduced glutathione, and β-mercaptoethanol. Examples of the site cleavable by the reducing agent include a disulfide residue, an alkoxyalkyl residue, and an azo residue.

Examples of the oxidizing agent include sodium periodate and oxidized glutathione. Examples of the site cleavable by the oxidizing agent include a vicinal diol residue and a selenium residue.

Examples of the enzyme include trypsin, papain, TEV, thrombin, cathepsin B, cathepsin D, cathepsin K, caspase, protease, matrix metalloproteinase, lipase, endoglycosidase, and PNGase F. Examples of the site cleavable by the enzyme include an ester residue, a phosphodiester residue, and a glycosyl residue.

Examples of the site cleavable by light include a 2-nitrobenzyl residue, a phenacyl ester residue, an 8-quinoline benzenesulfonate residue, a coumarin residue, a phosphotriester residue, a bis-arylhydrazone residue, and a bimane dithiopropionic acid residue.

Examples of the self-degradable cleavable portion include an activated ester residue (for example, an N-hydroxysuccinimide residue).

### <Cleavable Portion CLE(B) Capable of Generating Bioorthogonal Functional Group by Cleavage>

The cleavable portion may be one that is capable of generating a bioorthogonal functional group on the reactive-group side by cleavage. Examples of such a cleavable portion include a disulfide residue, an ester residue (including a normal ester residue, and other ester residues described above, such as a thioester residue), an acetal residue (including a normal ester residue, and other acetal residues such as a thioacetal residue), a ketal residue, an imine residue, and a vicinal diol residue.

### <Linkers>

The first linker, represented by L₁, and the second linker, represented by L₂, may be the same or different divalent groups. Each divalent group may be substituted or unsubstituted. Examples of the divalent group include those described above. In cases where the divalent group is substituted, examples of the substituent include those described above. Further, examples of the linker include a substance such as a peptide, a nucleic acid, a sugar, another polymeric substance (such as polyethylene glycol), or a divalent hydrocarbon group (such as an alkyl chain). Those skilled in the art can appropriately determine the presence or absence of each linker and the type of each linker, depending on the types of the first and second affinity moieties to be used.

In a particular embodiment, the total number of atoms constituting the backbones in the first linker and the second linker may be 2 to 10. The total number of such atoms may be not less than 3 or not less than 4. The total number of such atoms may be not more than 9, not more than 8, or not more than 7. More specifically, the total number of such atoms may be 3 to 9, 4 to 8, or 4 to 7.

The number of atoms constituting the backbone in each of the first linker and the second linker may be 1 to 9. The number of such atoms may be not less than 2 or not less than 3. The number of such atoms may be not more than 8, not more than 7, or not more than 6. More specifically, the number of such atoms may be 2 to 8, 3 to 7, or 3 to 6.

The backbone in each of the first linker and the second linker is constituted by a chain structure, a cyclic structure, or a structure containing a combination thereof. **In** cases where the backbone is a chain structure not containing a cyclic structure, the number of atoms of the backbone can be determined by counting the number of atoms in the chain structure. On the other hand, in cases where the backbone is a structure containing a cyclic structure, the number of atoms of the backbone can be determined by counting a predetermined number of atoms constituting the cyclic structure as part of the number of atoms of the backbone. Specifically, the number of atoms of the backbone in the cyclic structure can be determined by counting the number of atoms in the shortest path connecting the two bonding sites in the cyclic structure (see, for example, the paths shown in bold in (a) to (d) below). In cases where the backbone is a structure containing a combination of a chain structure and a cyclic structure, the number of atoms of the backbone can be determined as the sum of the number of atoms in the chain structure, which does not contain a cyclic structure, and the number of atoms in the shortest path connecting the two bonding sites in the cyclic structure. The method of counting the number of atoms of the backbone also applies to other linkers. 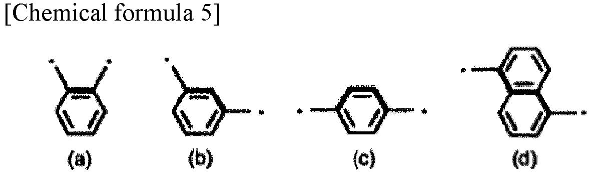 wherein "·" represents a bonding site.

In the case of (a), since the shortest path is the path shown in bold, the number of atoms in the divalent cyclic structure that are counted as part of the number of atoms of the backbone is 2.

In the case of (b), since the shortest path is the path shown in bold, the number of atoms in the divalent cyclic structure that are counted as part of the number of atoms of the backbone is 3.

In the case of (c), since both paths are the shortest paths (equidistant), the number of atoms in the divalent cyclic structure that are counted as part of the number of atoms of the backbone is 4.

In the case of (d), since the path through the condensed site is the shortest path, the number of atoms in the divalent cyclic structure that are counted as part of the number of atoms of the backbone is 4.

### <Compound (Ia)>

Compound (Ia) is a compound in which a reactive group for the Fc region is linked to an affinity substance via a linker, the linker containing a cleavable portion capable of generating, by cleavage, a bioorthogonal functional group on the reactive-group side.

[Chemical formula 6] R-L₁-CLE(B)-L₂-A (Ia)

wherein
R represents a reactive group for the Fc region,
L₁ represents a first linker,
L₂ represents a second linker,
CLE(B) represents a cleavable portion capable of generating, by cleavage, a bioorthogonal functional group on the reactive group side, and
A represents an affinity substance represented by AP1-L_{A}-AP2,
wherein AP1 represents a first affinity peptide having affinity for a constant region of a heavy chain of an antibody, AP2 represents a second affinity peptide having affinity for a constant region of a heavy chain of an antibody, and L_{A} represents a linker.

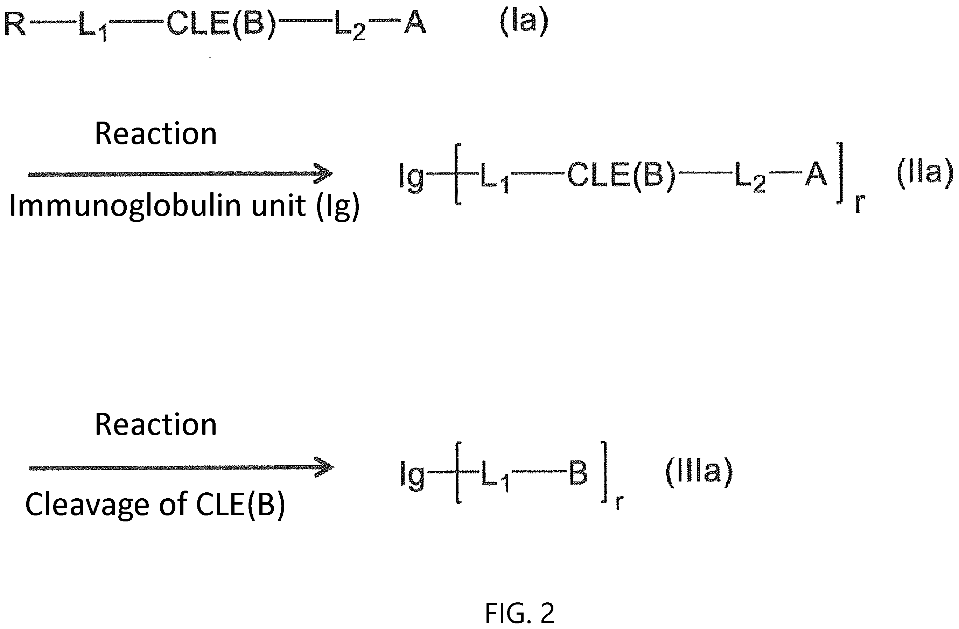

Fig. 2 shows reactions using Compound (Ia) (Step (II) and Step (III)).

One example of Compound (Ia) is a compound represented by the following Formula (Ia-1): 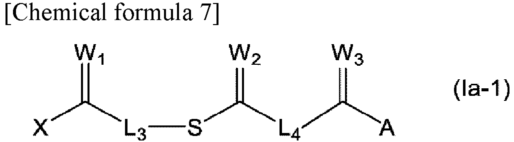 wherein
X represents a leaving group,
W₁, W₂, and W₃ each independently represent an oxygen atom or a sulfur atom,
L₃ represents a third linker,
L₄ represents a fourth linker,
S represents a sulfur atom, and
A represents an affinity substance containing first and second affinity moieties having affinity for a constant region of a heavy chain of an antibody, or a salt thereof. The definition, examples, and preferred examples of the affinity substance represented by A are as described above.

The leaving group, represented by X, is a group that can leave upon reaction between the carbon atom in C=W₁ adjacent to X, and an amino group. Those skilled in the art can appropriately set such a leaving group. Examples of such a leaving group include the following:
(a) R_{A}-S, wherein R_{A} represents a hydrogen atom, a monovalent hydrocarbon group optionally containing a substituent, or a monovalent heterocyclic group optionally containing a substituent; and S represents a sulfur atom;
(b) R_{A}-O, wherein R_{A} represents a hydrogen atom, a monovalent hydrocarbon group optionally containing a substituent, or a monovalent heterocyclic group optionally containing a substituent; and O represents an oxygen atom;
(c) R_{A}-(R_{B}-)N, wherein R_{A} and R_{B} each independently represent a hydrogen atom, a monovalent hydrocarbon group optionally containing a substituent, or a monovalent heterocyclic group optionally containing a substituent; and N represents a nitrogen atom; or
(d) a halogen atom.

W₁, W₂, and W₃ each independently represent an oxygen atom or a sulfur atom. Preferably, W₁, W₂, and W₃ may be oxygen atoms.

The third linker, represented by L₃, and the fourth linker, represented by L₄, may be the same or different divalent groups. Each divalent group may be substituted or unsubstituted. Examples of the divalent group include those described above. In cases where the divalent group is substituted, examples of the substituent include those described above.

In a particular embodiment, the total number of atoms constituting the backbones in the third linker and the fourth linker may be 2 to 10. The total number of such atoms may be not less than 3 or not less than 4. The total number of such atoms may be not more than 9, not more than 8, or not more than 7. More specifically, the total number of such atoms may be 3 to 9, 4 to 8, or 4 to 7.

The number of atoms constituting the backbone in each of the third linker and the fourth linker may be 1 to 9. The number of such atoms may be not less than 2 or not less than 3. The number of such atoms may be not more than 8, not more than 7, or not more than 6. More specifically, the number of such atoms may be 2 to 8, 3 to 7, or 3 to 6.

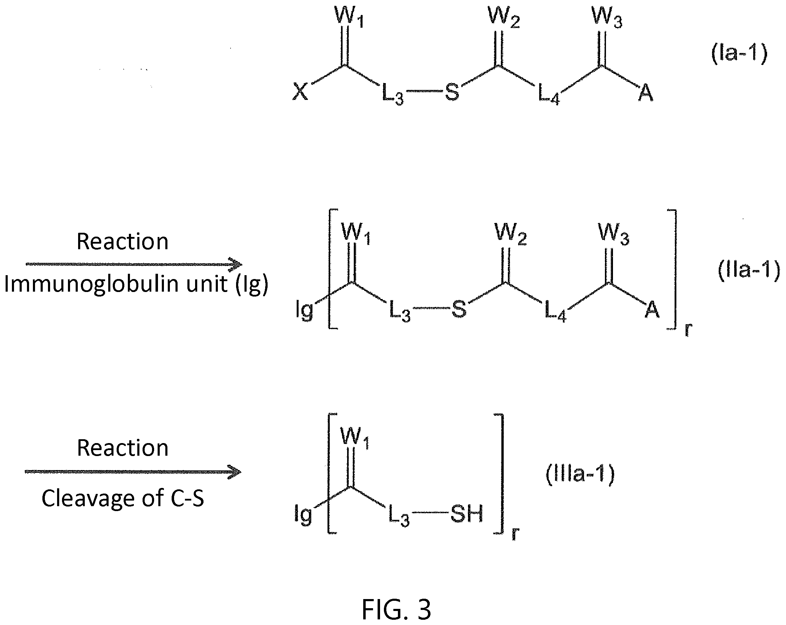

Fig. 3 shows reactions using Compound (Ia-1) (Step (II) and Step (III)).

### <Compound (Ib)>

Compound (Ib) is a compound in which a reactive group for the Fc region is linked to an affinity substance via a linker, the linker containing a cleavable portion therein, the compound further containing a bioorthogonal functional group between the reactive group for the antibody and the cleavable portion.

The bioorthogonal functional group is as described above. Preferred examples of the bioorthogonal functional group include an azide group, an alkyne residue, preferably a cyclic group having a carbon-carbon triple bond, which may be substituted with a substituent as described above, a tetrazine residue, an alkene residue, a thiol group, a maleimide residue, a furan residue, and a halocarbonyl residue. 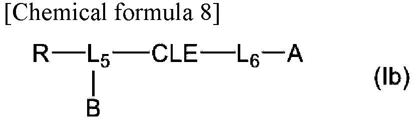 wherein
R represents the reactive group,
L₅ represents a fifth linker,
L₆ represents a sixth linker,
B represents a group containing a bioorthogonal functional group,
CLE represents a cleavable portion, and
A represents the affinity substance.

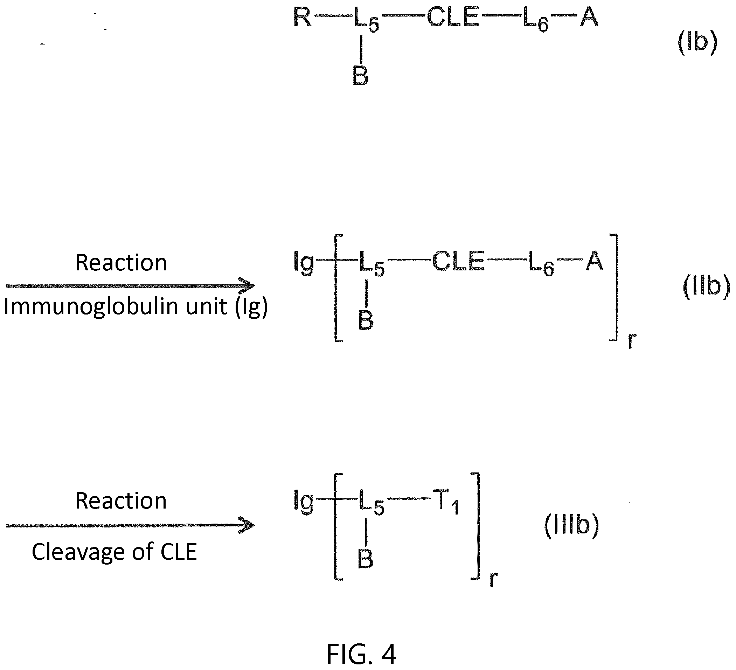

Fig. 4 shows reactions using Compound (Ib) (Step (II) and Step (III)).

Examples of Compound (Ib) include a compound represented by the following Formula (Ib-1). 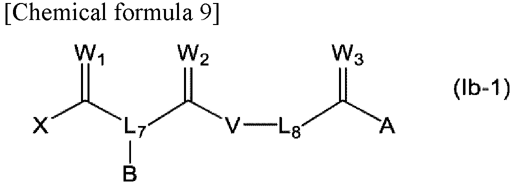 wherein
X represents a leaving group,
W₁, W₂, and W₃ each independently represent an oxygen atom or a sulfur atom;
L₇ represents a seventh linker,
L₈ represents an eighth linker,
B represents a group containing a bioorthogonal functional group,
V represents an oxygen atom or a sulfur atom, and
A represents an affinity substance containing first and second affinity moieties having affinity for a constant region of a heavy chain of an antibody, or a salt thereof. The definitions, examples, and preferred examples of the leaving group represented by X, the group containing a bioorthogonal functional group represented by B, and the affinity substance represented by A are as described above.

W₁, W₂, and W₃ each independently represent an oxygen atom or a sulfur atom. Preferably, W₁, W₂, and W₃ may be oxygen atoms.

The seventh linker, represented by L₇, and the eighth linker, represented by L₈, may be the same or different divalent groups. Each divalent group may be substituted or unsubstituted. Examples of the divalent group include those described above. In cases where the divalent group is substituted, examples of the substituent include those described above.

In a particular embodiment, the total number of atoms constituting the backbones in the seventh linker and the eighth linker may be 2 to 10. The total number of such atoms may be not less than 3, or not less than 4. The total number of such atoms may be not more than 9, not more than 8, or not more than 7. More specifically, the total number of such atoms may be 3 to 9, 4 to 8, or 4 to 7.

The number of atoms constituting the backbone in each of the seventh linker and the eighth linker may be 1 to 9. The number of such atoms may be not less than 2 or not less than 3. The number of such atoms may be not more than 8, not more than 7, or not more than 6. More specifically, the number of such atoms may be 2 to 8, 3 to 7, or 3 to 6.

V represents an oxygen atom or a sulfur atom. Preferably, V may be a sulfur atom.

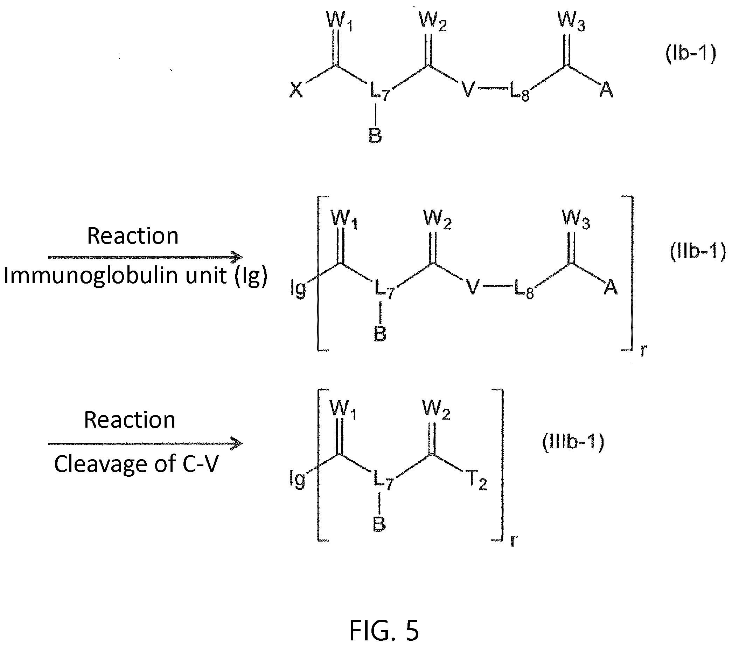

Fig. 5 shows reactions using Compound (Ib-1) (Step (II) and Step (III)).

The above-described series of compounds or salts thereof can be produced by reacting the affinity substance with a partial compound containing a reactive group for the antibody. For example, such a reaction may be carried out in an appropriate organic solvent system (for example, an organic solvent containing an alkyl halide such as CH₂Cl₂ (for example, a methyl halide), and an amine such as triethylamine) at an appropriate temperature (for example, about - 10 to 30°C). The reaction time is, for example, 1 minute to 20 hours, preferably 10 minutes to 15 hours, more preferably 20 minutes to 10 hours, still more preferably 30 minutes to 8 hours.

Generation of the above-described series of compounds or salts thereof may be confirmed by, for example, electrophoresis, chromatography (for example, gel filtration chromatography, ion-exchange chromatography, reversed-phase column chromatography, or HPLC), NMR, or mass spectrometry, depending on the molecular weights of the specific starting materials and the products. Such compounds or salts thereof may be appropriately purified by any method including chromatography (for example, the chromatography described above, and affinity chromatography).

The reaction between Fc and the compound represented by Formula (Ia) or Formula (Ib) is not particularly limited, and can be easily carried out by mixing Fc and the compound represented by Formula (Ia) or Formula (Ib) in a solution. After the reaction, an unreacted compound is preferably removed as appropriate.

As a result, an Fc-Compound (Ia) complex or an Fc-Compound (Ib) complex is formed.

### <Step (III): Subjecting an Fc-Compound (Ia) Complex or an Fc-Compound (Ib) Complex to Cleavage Treatment at the Cleavable Portion to Form an Fc Region That Does Not Contain the Affinity Substance and to Which a Bioorthogonal Functional Group Is Introduced>

Examples of the cleavage treatment include: (a) treatment with one or more substances selected from the group consisting of an acidic substance, a basic substance, a reducing agent, an oxidizing agent, and an enzyme as described above; (b) treatment by physicochemical stimulation such as light; or (c) being left to stand (incubation) in a case where a cleavable linker containing a self-degradable cleavable portion is used. For these cleavage treatments, reference may also be made to WO2019/240287, WO2019/240288, WO2020/009165, and WO2020/090979.

Such a cleavage reaction may be appropriately carried out under conditions that cannot cause protein denaturation or degradation (for example, that cannot cause cleavage of an amide bond) (mild conditions). For example, such mild conditions are as described above. Further, in cases where the cleavable site is an ester (for example, an ordinary ester, or another ester such as a thioester), the cleavage reaction may be carried out by performing incubation for an appropriate length of time (for example, 1 hour) in a hydroxylamine hydrochloride solution (for example, pH 4.0 to 8.0, 10 mM to 10 M) (for example, Vance, N. et al., Bioconjugate Chem. 2019, 30, 148-160).

Generation of the antibody or a salt thereof not containing the affinity substance, obtained by the cleavage reaction, may be confirmed by, for example, electrophoresis, chromatography (for example, gel filtration chromatography, ion-exchange chromatography, reversed-phase column chromatography, or HPLC), or mass spectrometry, depending on the molecular weights of the specific starting materials and the product. Regioselectivity may be confirmed by peptide mapping as described above. The number of affinity substances introduced may be confirmed by mass spectrometry (optionally using DAR calculator (software manufactured by Agilent) in combination). The affinity substance-modified antibody or a salt thereof may be appropriately purified by any method including chromatography (for example, the chromatography described above, and affinity chromatography).

By this, the bioorthogonal functional group can be bound to a side-chain functional group of a particular amino acid in one of the polypeptides constituting the Fc region. This bioorthogonal functional group may be reacted with a compound containing another bioorthogonal functional group, and the latter bioorthogonal functional group may be used as a bioorthogonal functional group to be reacted with a functional group on the cell surface.

### <Modification with Functional Substance>

In cases where the Fc-cell region complex is a complex in which a functional substance is introduced via a linker that is introduced into a functional group in the side chain of a particular amino acid residue present at one or more positions of the other of the two antibody Fc-constituting polypeptides (the polypeptide that does not bind to the cell), the method further includes a step of introducing the functional substance into a functional group in the side chain of a particular amino acid residue present at one or more positions of the other of the two antibody Fc-constituting polypeptides in the antibody Fc region.

In this case, in Step (II), the reaction between the Fc region and Compound (Ia) or Compound (Ib) may be carried out twice using Compound (Ia) or Compound (Ib) containing different kinds of bioorthogonal functional groups, wherein one of the reacted compounds may be for binding to the cell while the other may be for binding to the functional substance.

As a result, a Compound (Ia)-Fc-Compound (Ia) complex or a Compound (Ib)-Fc-Compound (Ib) complex can be obtained. The complex may also be a Compound (Ia)-Fc-Compound (Ib) complex.

After such a Compound-Fc-Compound complex is cleaved at the cleavable portions to obtain a modified Fc in which two types of bioorthogonal functional groups are respectively bound to the Fc-constituting polypeptides, but before reacting the modified Fc with the cell in step (V), a functional substance bound to a functional group that reacts with one of the bioorthogonal functional groups may be reacted with the bioorthogonal functional group, to introduce the functional substance to one of the polypeptides constituting the Fc region.

Subsequently, by reacting a cell therewith, a cell-Fc-functional substance complex can be obtained, the complex being, more specifically, an Fc-cell complex in which the cell is bound to an Fc modified with a functional substance, or to an antibody or Fc fusion protein containing the modified Fc.

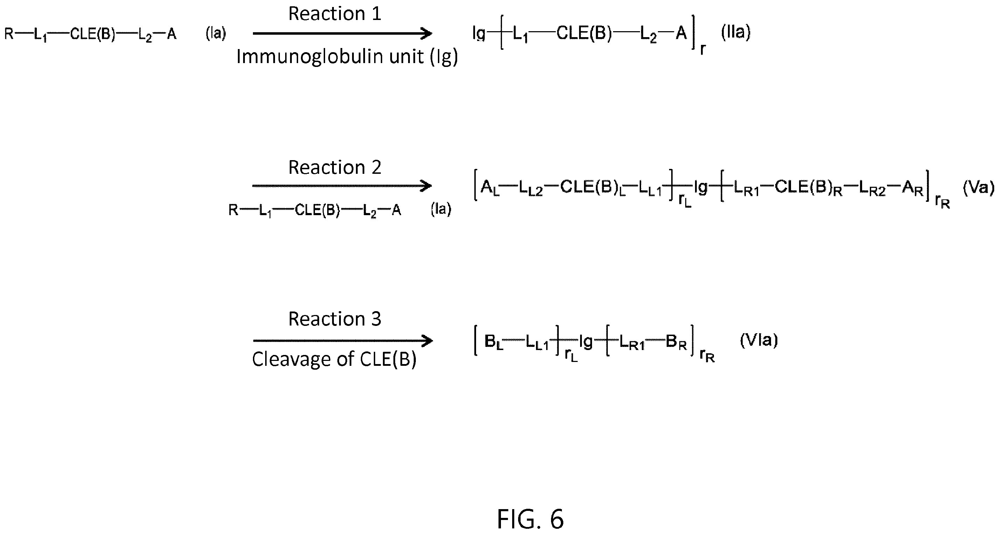

Fig. 6 shows reactions using Compound (Ia) (Step (II) (Reactions 1 and 2) and Step (III) (Reaction 3)).

By cleavage at each cleavable portion, the affinity substance is removed. Further, modified Fc (VIa), in which two kinds of bioorthogonal functional groups are respectively bound to the Fc-constituting polypeptides, is obtained. Therefore, a functional substance can be introduced into one of the two kinds of bioorthogonal functional groups B_{L} and B_{R}, and the other of the bioorthogonal functional groups can be used for introduction to the cell.

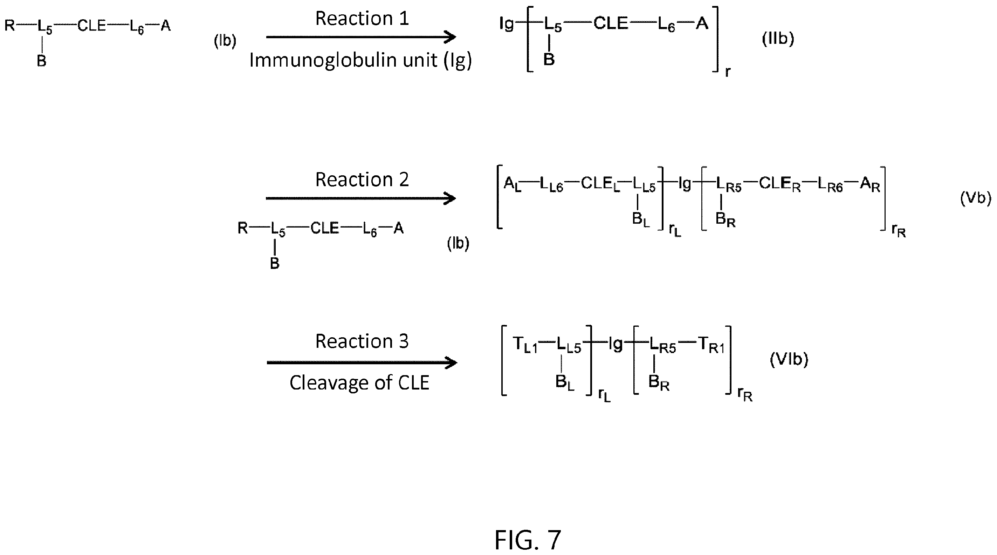

Fig. 7 shows reactions using Compound (Ib) (Step (II) (Reactions 1 and 2) and Step (III) (Reaction 3)).

By cleavage at each cleavable portion, the affinity substance is removed. As a result, modified Fc (VIb), in which two kinds of bioorthogonal functional groups are respectively bound to the Fc-constituting polypeptides, is obtained. Therefore, a functional substance can be introduced into one of the two kinds of bioorthogonal functional groups B_{L} and B_{R}, and the other of the bioorthogonal functional groups can be used for introduction to the cell.

### <Step (IV): Step of Providing a Cell Having, on a Surface Thereof, a Functional Group That Reacts with a Bioorthogonal Functional Group>

The cells as described above may be used.

The functional group that reacts with a bioorthogonal functional group may be appropriately selected depending on the type of the bioorthogonal functional group, and is not particularly limited. Examples of the functional group include an alkyne residue and ortho-phosphane benzoic acid for an azide group; a maleimide residue, an alkene residue, an alkyne residue, a haloamide residue, and a haloketone residue for a thiol group; and norbornene, isonitrile, and BCN (bicyclo[6.1.0]nonyne) for a tetrazine residue. Similarly, the combination of the bioorthogonal functional group and the functional group that reacts with the bioorthogonal functional group may be reversed, such as an azide group for an alkyne residue, and a thiol group for a maleimide residue.

In cases where the cell has, on a surface thereof, a functional group that reacts with a bioorthogonal functional group, the cell can be used as it is. However, usually, it is preferred to modify the cell surface and introduce, to the cell surface, a functional group that reacts with a bioorthogonal functional group. For example, a functional group that reacts with a bioorthogonal functional group can be introduced to the cell surface by means of, for example, cell-surface modification using an enzyme (for example, GlcNAc-tagging (glycocalyx tagging) using O-GlcNAcase (OGA) and N-acetylglucosaminyltransferase (OGT), HaloTag Technology using haloalkane dehalogenase, sortagging (sortase-mediated tagging) using sortase A, or Q-tagging using transglutaminase), introduction of the functional group by sugar-chain modification on the cell surface, or introduction of the functional group to a lipid membrane via a fatty chain. It is also preferred to achieve the introduction by reacting a cross-linking compound containing a functional group that reacts with a bioorthogonal functional group, or a reagent capable of generating a functional group that reacts with a bioorthogonal functional group, such as Traut's Reagent, with a functional group such as a thiol group, an amino group, or a carboxyl group present in a protein or the like on the cell surface. As the cross-linking compound, a known cross-linking compound may be used, and examples of the cross-linking compound that reacts with a thiol group include a maleimide compound, a haloacetic acid compound, a pyridyldisulfide compound, a thiosulfone compound, and a vinyl sulfone compound. Examples of the cross-linking compound that reacts with an amino group include an NHS ester compound, an imidoester compound, a pentafluorophenyl ester compound, and a hydroxymethylphosphine compound. Examples of the cross-linking compound that reacts with a carboxyl group include an oxazoline compound. Thus, a cell having, on the surface thereof, a functional group that reacts with a bioorthogonal functional group can be provided by reacting a compound containing: a cross-linking reactive group such as maleimide, haloacetic acid, pyridyldisulfide, thiosulfone, vinyl sulfone, NHS ester, imidoester, pentafluorophenyl ester, hydroxymethylphosphine, or oxazoline; and the functional group that reacts with the bioorthogonal functional group; with a functional group such as a thiol group, an amino group, or a carboxyl group on the cell surface.

In such a compound containing a cross-linking reactive group and a functional group that reacts with a bioorthogonal functional group, examples of a linker portion connecting the cross-linking reactive group to the functional group that reacts with the bioorthogonal functional group include one group selected from, or a group having a backbone structure containing two or more groups selected from, the group consisting of the above-described divalent linear hydrocarbon group, divalent cyclic hydrocarbon group, divalent heterocyclic group, -C(=O)-, - C(=S)-, -NR₁-, -C(=O)-NR₁-, -NR₁-C(=O)-, -C(=S)-NR₁-, -NR₁-C(=S)-, -O-, -S-, -(O-R₂)ₙ-, and -(S-R₂)ₘ-, as well as divalent groups derived from a substance selected from amino acids, peptides, nucleic acids, sugars, other polymeric substances (for example, polyethylene glycol), and the like. The total number of atoms constituting the backbone in the linker portion is not particularly limited, and may be, for example, not less than 1, not less than 2, not less than 5, or not less than 10. The total number of such atoms may be not more than 1000, not more than 500, not more than 300, not more than 200, not more than 100, not more than 50, or not more than 20.

### <Step (V): Step of Introducing the Fc Region to the Cell Surface by Reacting the Fc Region, to Which the Bioorthogonal Functional Group Is Introduced, with the Cell, through Reaction of the Bioorthogonal Functional Group of the Fc Region with the Functional Group of the Cell That Reacts with the Bioorthogonal Functional Group>

The reaction between the Fc region to which the bioorthogonal functional group is introduced and the cell having, on the surface thereof, the functional group that reacts with the bioorthogonal functional group is not particularly limited, and may be carried out by adding the Fc region, to which the bioorthogonal functional group is introduced, to a medium or a solution such as physiological saline containing the cell, and mixing the resulting mixture.

When appropriate, the reaction is preferably followed by removal of an Fc region to which the bioorthogonal functional group has been introduced but which has not reacted with the cell.

As a result, an Fc-cell complex is formed.

### EXAMPLES

The present invention is specifically described below by way of Examples. However, the present invention is not limited to the embodiments described in the following Examples.

Example 1 Synthesis of Antibody to Which One Fluorescent Molecule and One Azide Group Were Introduced per Antibody Molecule

### Example 1-1 Synthesis of Antibody to Which One AF488 Molecule and One Azide Group Were Introduced per Antibody Molecule (T-4)

### Example 1-1-1 Synthesis of Trastuzumab to Which One Peptide Reagent Molecule Was Introduced per Trastuzumab Molecule (T-1)

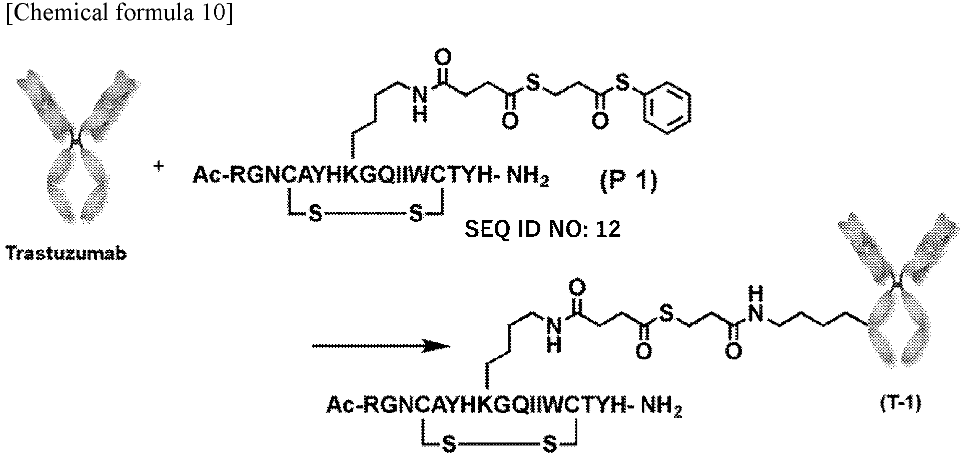

To a 10 mg/mL solution of anti-human HER2 monoclonal antibody Trastuzumab (Chugai Pharmaceutical Co., Ltd.) in acetate buffer (50 mM sodium acetate, pH 5.5), a solution of a peptide reagent (P1) described in a previous publication (WO2019/240287A1) in dimethylformamide (1 equivalent relative to the antibody, dimethylformamide (DMF) (8% v/v) solution) was added, and the resulting mixture was shaken at room temperature for 1 hour. After the reaction solution was purified using an NAP-25 desalting column (manufactured by Cytiva), an antibody to which one molecule of the peptide reagent was introduced per antibody molecule (T-1) was obtained using AKTA pure 25 (manufactured by Cytiva). HIC-HPLC analysis was performed according to a previous publication (Anal. Chem., 2019, 91, 20, 12724-12732) to confirm that one peptide reagent was introduced.

The purification using AKTA pure 25 (manufactured by Cytiva) was performed under the following conditions.
Column: RESOURCE S (manufactured by Cytiva)
Eluent A: 50 mM Sodium acetate, 0.1% Tween 20 (pH 5.0)
Eluent B: 50 mM Sodium acetate, 1 M NaCl, 0.1% Tween 20 (pH 5.0)
Flow rate: 5.0 mL/min
Detector: Detection was carried out at wavelengths of 215 nm and 280 nm.

### Example 1-1-2 Synthesis of Trastuzumab to Which Two Peptide Reagent Molecules Were Introduced per Trastuzumab Molecule (T-2)

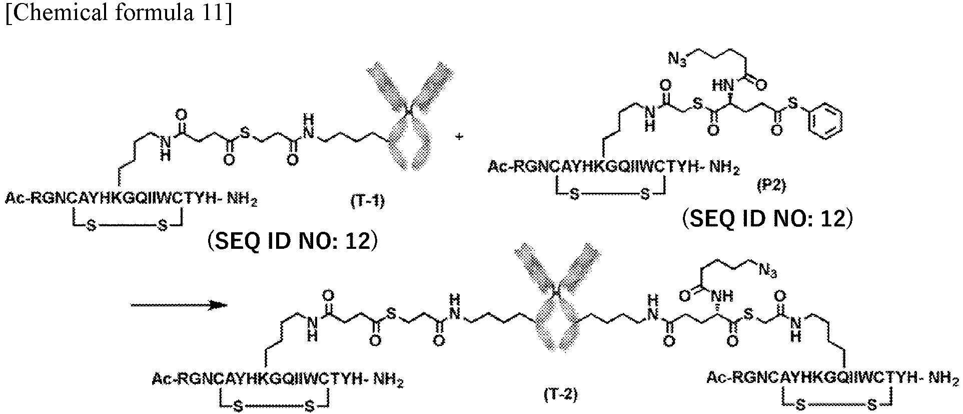

Subsequently, by using peptide reagent (P2), an antibody to which peptide reagents (P1) and (P2) were introduced (T-2) was obtained according to the method described in a previous publication (WO2022/191283A1). HIC-HPLC analysis was performed according to a previous publication (Anal. Chem., 2019, 91, 20, 12724-12732) to confirm that the two peptide reagents were introduced.

### Example 1-1-3 Synthesis of Trastuzumab to Which One Thiol Group and One Azide Group Were Introduced per Trastuzumab Molecule (T-3)

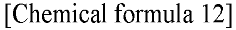

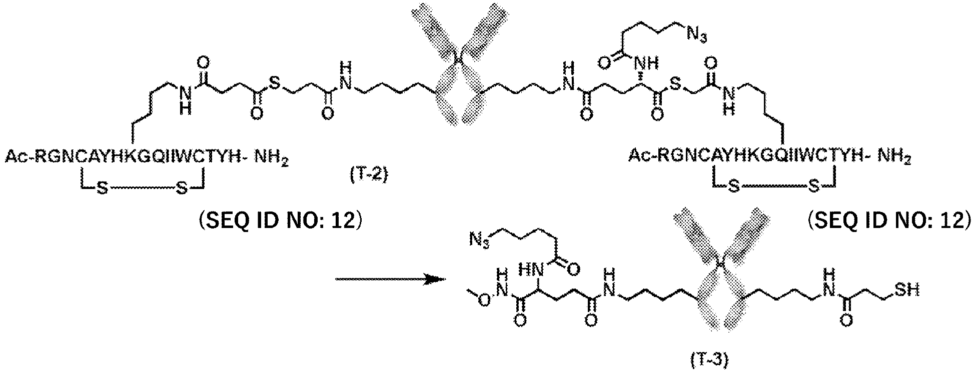

To the antibody obtained in Example 1-1-2 (T-2), to which peptide reagents (P1) and (P2) had been introduced, a methoxyamine solution was added according to a previous publication (WO2022/191283A1), and the resulting mixture was shaken at room temperature for 3 hours, to obtain an antibody to which one thiol group and one azide group were introduced per antibody molecule (T-3). HIC-HPLC analysis was performed according to a previous publication (Anal. Chem., 2019, 91, 20, 12724-12732), to confirm that one thiol group and one azide group were introduced therein.

### Example 1-1-4 Synthesis of Trastuzumab to Which One AF488 Molecule and One Azide Group Were Introduced per Trastuzumab Molecule (T-4)

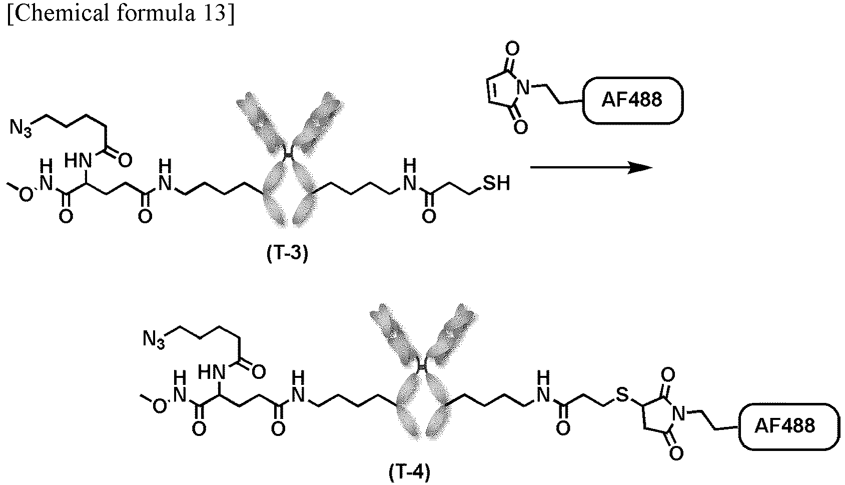

To a 3 mg/mL solution of Trastuzumab obtained in Example 1-1-3 (T-3), to which one thiol group and one azide group had been introduced per Trastuzumab molecule, in PBSE buffer (10 mM phosphate buffered saline (PBS), 10 mM EDTA, pH 7.4), a solution of Alexa Fluor 488 C5 maleimide (Invitrogen) in dimethylformamide (2.5 equivalents relative to the antibody, dimethylformamide (DMF) (8% v/v) solution) was added, and the resulting mixture was shaken at room temperature for 1 hour. The reaction solution was purified using an NAP-25 desalting column (manufactured by Cytiva) to obtain an antibody to which one fluorescent molecule was introduced per antibody molecule (T-4). HIC-HPLC analysis was performed according to a previous publication (Anal. Chem., 2019, 91, 20, 12724-12732) to confirm that AF488 was introduced.

### Example 1-2 Synthesis of Trastuzumab to Which One Cy5 Molecule and One Azide Group Were Introduced per Trastuzumab Molecule (T-5)

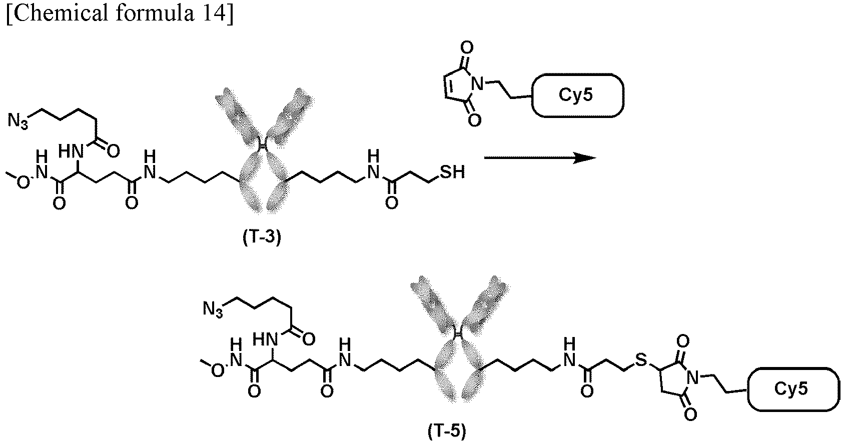

To a 3 mg/mL solution of Trastuzumab obtained in Example 1-1-3 (T-4), to which one thiol group and one azide group had been introduced per Trastuzumab Molecule, in PBSE buffer (10 mM phosphate buffered saline (PBS), 10 mM EDTA, pH 7.4), a solution of N-(m-PEG4)-N'-(PEG3-Mal)-Cy5 (BroadPharm) in dimethylformamide (2.5 equivalents relative to the antibody, dimethylformamide (DMF) (8% v/v) solution) was added, and the resulting mixture was shaken at room temperature for 1 hour. The reaction solution was purified using an NAP-25 desalting column (manufactured by Cytiva) to obtain an antibody to which one Cy5 molecule was introduced per antibody molecule (T-5). HIC-HPLC analysis was performed according to a previous publication (Anal. Chem., 2019, 91, 20, 12724-12732) to confirm that Cy5 was introduced.
Comparative Example 1 Synthesis of Antibody to Which One Cy5 Molecule and a Plurality of Azide Groups Were Introduced per Antibody Molecule
Comparative Example 1-1 Synthesis of Antibody to Which One Cy5 Molecule Was Introduced per Antibody Molecule, and to Which a Plurality of Azide Groups Were Introduced per Antibody Molecule via Lysine Residues
Comparative Example 1-1-1 Synthesis of Trastuzumab to Which One Thiol Group
Was Introduced per Trastuzumab Molecule (T-6)

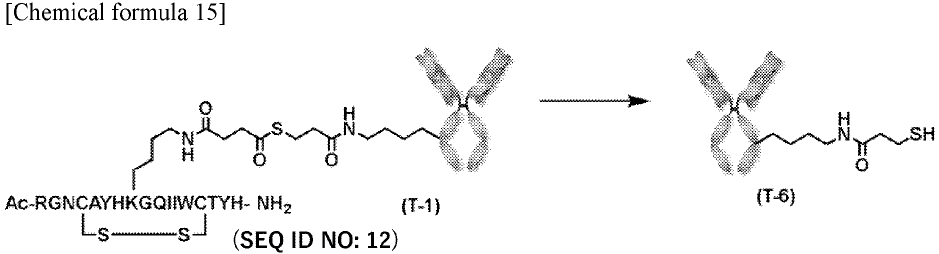

To the antibody obtained in Example 1-1-1 (T-1), to which peptide reagent (P1) had been introduced, a hydroxylamine solution was added according to a previous publication (WO2019/240287A1), and the resulting mixture was shaken at room temperature for 1 hour, to obtain an antibody to which one thiol group was introduced per antibody molecule (T-6). HIC-HPLC analysis was performed according to a previous publication (Anal. Chem., 2019, 91, 20, 12724-12732) to confirm that one thiol group was introduced.

### Comparative Example 1-1-2 Synthesis of Trastuzumab to Which One Cy5 Molecule Was Introduced per Trastuzumab Molecule (T-7)

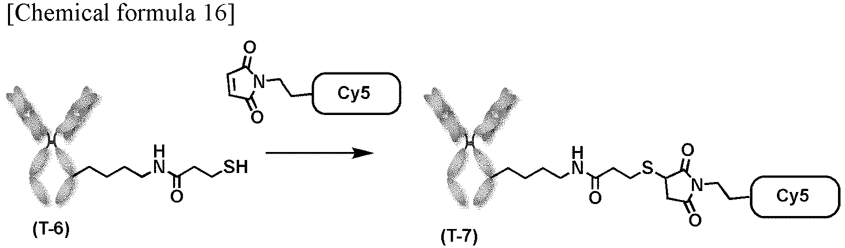

To a 3 mg/mL solution of Trastuzumab obtained in Comparative Example 1-1-1 (T-6), to which one thiol group had been introduced per Trastuzumab molecule, in PBSE buffer (10 mM phosphate buffered saline (PBS), 10 mM EDTA, pH 7.4), a solution of N-(m-PEG4)-N'-(PEG3-Mal)-Cy5 (BroadPharm) in dimethylformamide (2.5 equivalents relative to the antibody, dimethylformamide (DMF) (8% v/v) solution) was added, and the resulting mixture was shaken at room temperature for 1 hour. The reaction solution was purified using an NAP-25 desalting column (manufactured by Cytiva) to obtain an antibody to which one fluorescent molecule was introduced per antibody molecule (T-7). HIC-HPLC analysis was performed according to a previous publication (Anal. Chem., 2019, 91, 20, 12724-12732) to confirm that Cy5 was introduced.

### Comparative Example 1-1-3 Synthesis of Trastuzumab to Which One Cy5 Molecule Was Introduced per Trastuzumab Molecule, and to Which a Plurality of Azide Groups Were Introduced per Trastuzumab Molecule via Lysine Residues (T-8)

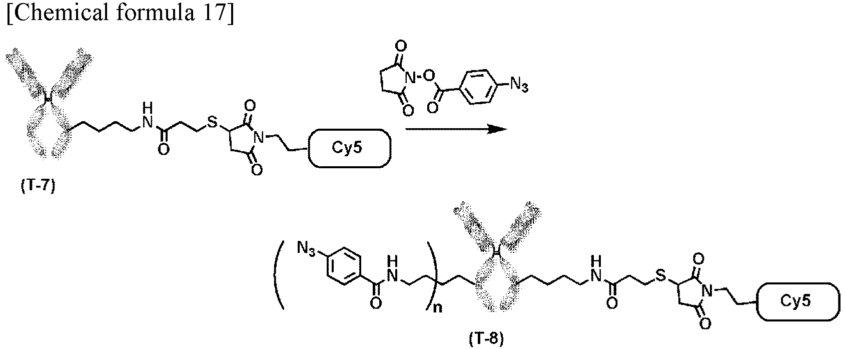

To a 3 mg/mL solution of Trastuzumab obtained in Comparative Example 1-1-2 (T-7), to which one Cy5 molecule had been introduced per Trastuzumab molecule, in PBS buffer (10 mM phosphate buffered saline (PBS), pH 7.4), a solution of 4-azidobenzoic acid N-hydroxysuccinimide ester in dimethylformamide (7 equivalents relative to the antibody, dimethylformamide (DMF) (8% v/v) solution) was added, and the resulting mixture was shaken at room temperature for 1 hour. The reaction solution was purified using an NAP-25 desalting column (manufactured by Cytiva) to obtain an antibody to which one Cy5 molecule and a plurality of azide groups were introduced per antibody molecule (T-8). QTOF-MS analysis was performed according to a previous publication (Anal. Chem., 2019, 91, 20, 12724-12732) to confirm that 4 to 5 azide groups were introduced.

### Comparative Example 1-2 Synthesis of Trastuzumab to Which One Cy5 Molecule Was Introduced per Trastuzumab Molecule, and to Which a Plurality of Azide Groups Were Introduced per Trastuzumab Molecule via Cysteine Residues (T-9)

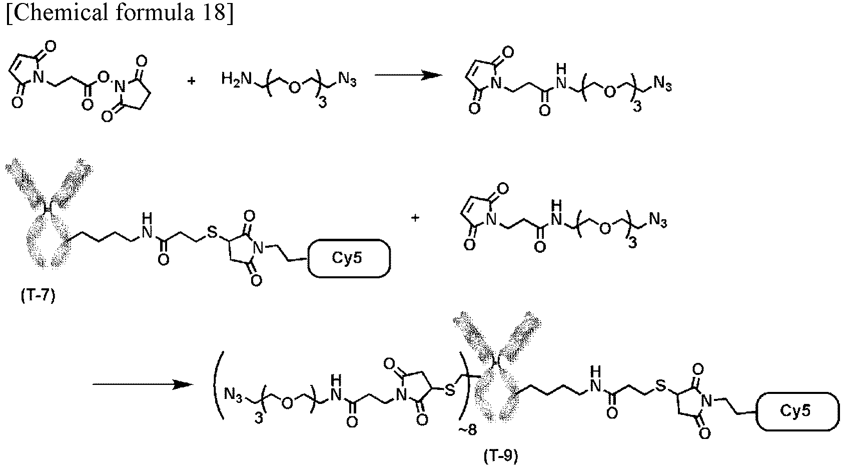

To a solution (20 mM) of commercially available N-succinimidyl 3-maleimidopropionate in dimethylformamide, the same amount of a solution (20 mM) of 3-azidopropylamine in dimethylformamide was added, and the resulting mixture was shaken for 30 minutes to prepare a solution (10 mM) of azido-PEG3-maleimide in dimethylformamide.

To a 3 mg/mL solution of Trastuzumab obtained in Comparative Example 1-1-2 (T-7), to which one Cy5 molecule had been introduced per Trastuzumab molecule, in PBSE buffer (10 mM phosphate buffered saline (PBS), 10 mM EDTA, pH 7.4), an aqueous solution of tris(2-carboxyethyl)phosphine (12 equivalents relative to the antibody) was added, and the resulting mixture was shaken at 37°C for 1.5 hours. The reaction solution was purified using an NAP-25 desalting column (manufactured by Cytiva). To the PBSE buffer of the obtained antibody, an azido-PEG3-maleimide solution (12 equivalents relative to the antibody, dimethylformamide (DMF) (8% v/v) solution) was added, and the resulting mixture was shaken at room temperature for 1 hour. The reaction solution was purified using an NAP-25 desalting column (manufactured by Cytiva) to obtain an antibody to which one Cy5 molecule and a plurality of azide groups were introduced per antibody molecule (T-9). QOF-MS analysis was performed according to a previous publication (Anal. Chem., 2019, 91, 20, 12724-12732) to confirm that 7 to 8 azide groups were introduced.

### Example 2 Synthesis of AF488 Antibody-Cell Conjugate

### Example 2-1 Cell Culture

HEL cells (JCRB) were cultured in D-MEM/F12 medium (Thermo Fisher Scientific) supplemented with 10% FBS (Thermo Fisher Scientific) and 1% penicillin-streptomycin (Nacalai Tesque, Inc.).

### Example 2-2 Synthesis of Antibody-Cell Conjugate

After HEL cells were detached by pipetting, the number of cells and the viability were measured with a Countess Automated Cell Counter (Thermo Fisher Scientific). HEL cells in an amount of 2.0 × 10⁶ cells were collected and centrifuged (at 6000 rpm for 15 seconds), and then the supernatant was removed. One milliliter of PBS was added to the HEL cells to wash the cells, and centrifugation was performed, followed by removing the supernatant. One milliliter of 0.1 mg/mL Traut's reagent (Sigma-Aldrich)/PBS was added to the HEL cells, and then the cells were incubated at room temperature for 20 minutes. Centrifugation was performed, and then the supernatant was removed. Thereafter, a step of adding 1 mL of PBS to the cells, washing the cells, performing centrifugation, and then removing the supernatant was repeated twice to obtain HEL cells having a cell surface modified with sulfhydryl groups. Subsequently, 1 mL of 20 µM dibenzocyclooctyne (DBCO)-PEG4-maleimide (BroadPharm)/PBS was added to the HEL cells, and the cells were incubated at room temperature for 30 minutes. Centrifugation was carried out, and then the supernatant was removed. Thereafter, a step of adding 1 mL of PBS to the cells, washing the cells, performing centrifugation, and then removing the supernatant was repeated twice to obtain HEL cells having a cell surface modified with DBCO.

HEL cells having a cell surface modified with DBCO, or HEL cells having an unmodified cell surface were collected in an amount of 0.5 × 10⁶ cells each, and suspended in 200 µL of D-MEM/F12 medium. To each type of cells, 0.33 µM or 1 µM T-4 was added, and the cells were incubated at 37 °C for 1 hour. Centrifugation was carried out, and then the supernatant was removed. Thereafter, a step of adding 1 mL of PBS to the cells, washing the cells, performing centrifugation, and then removing the supernatant was repeated twice to obtain an antibody-cell complex.

### Example 2-3 Evaluation of Antibody-Cell Conjugate

The amount of antibody bound to the cells was evaluated by subjecting 0.2 × 10⁶ cells to flow cytometry analysis using an Attune NxT Flow Cytometer (Thermo Fisher Scientific). The remaining cells were cultured in 200 µL of D-MEM/F12 + 10% FBS + 1% penicillin-streptomycin for 24 hours, and then subjected to analysis by flow cytometry in the same manner as described above.

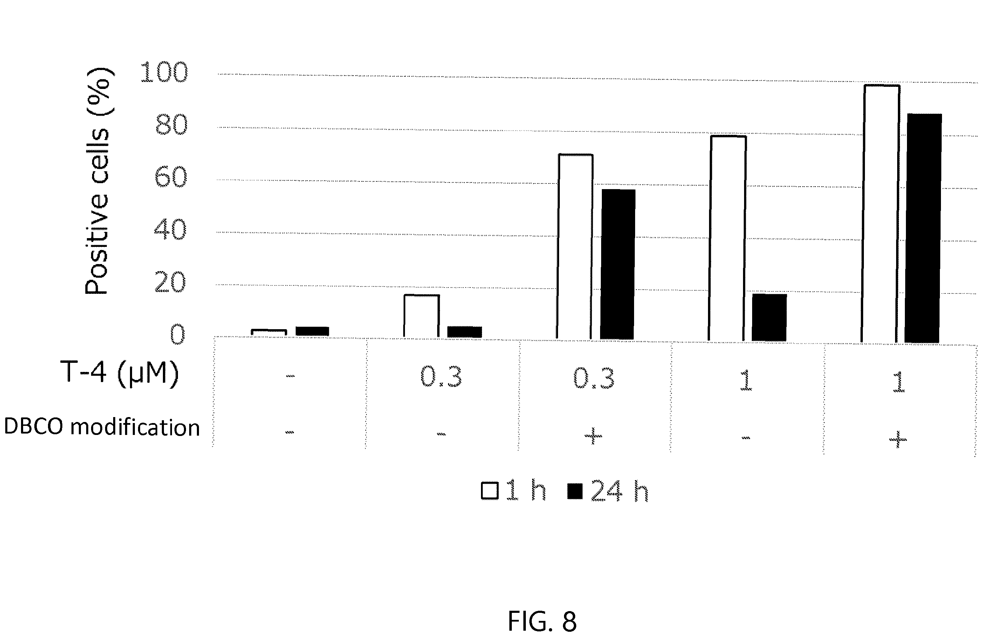

As shown in Fig. 8, the group in which T-4 antibody was reacted with the DBCO-modified cells had stronger fluorescence than the group in which T-4 antibody was reacted with the cells that had not been modified with DBCO, indicating that the antibody could be bound with higher efficiency in the former group. Further, 24 hours after the reaction, fluorescence was lost in most cells in the mixture of T-4 antibody and the cells that had not been modified with DBCO, while fluorescence was retained in most cells in the group in which T-4 antibody was reacted with the DBCO-modified cells, indicating that formation of a covalent bond between a DBCO group on the cell surface and an azide group of the antibody may contribute to high stability of the antibody modification.

### Example 3 Synthesis of Cy5 Antibody-Cell Conjugate and Evaluation of HER2-Binding Ability

### Example 3-1 Synthesis of Antibody-Cell Conjugate

HEL cells having a cell surface modified with DBCO were collected in an amount of 2 × 10⁶ cells each, and suspended in 200 µL of D-MEM/F12 medium. After adding 1 µM T-5 to the suspension, the resulting mixture was left to stand at 37 °C for 1 hour. Centrifugation was carried out, and then the supernatant was removed. Thereafter, a step of adding 1 mL of PBS to the cells, washing the cells, performing centrifugation, and then removing the supernatant was repeated twice to obtain an antibody-cell complex.

### Example 3-2 Evaluation of HER2 Binding of Antibody-Cell Conjugate

After subjecting 0.3 × 10⁶ cells to evaluation of HER2 binding, the remaining cells were cultured for 48 hours in 200 µL of D-MEM/F12 + 10% FBS + 1% penicillin-streptomycin, and then HER2 binding was evaluated in the same manner as described above.

To 0.3 × 10⁶ cells, 45 µL of FACS buffer (PBS + 1% FBS) supplemented with 2% Human TruStain FcX Fc Receptor Blocking Solution (BioLegend) was added, and the cells were allowed to react on ice for 10 minutes. Human recombinant HER2-Fc (R&D Systems) biotinylated using a Biotin Labeling Kit - NH₂ (Dojindo Laboratories) was added to the cells to a concentration of 10 µg/mL, and the cells were allowed to react on ice for 20 minutes. The cells were washed three times with FACS buffer, and 100 µL of FACS buffer supplemented with 20 µg/mL Streptavidin-Alexa Fluor 488 (Thermo Fisher Scientific) was added thereto, followed by allowing the cells to react on ice for 20 minutes. The cells were washed three times with FACS buffer, and suspended in 500 µL of FACS buffer.

By performing flow cytometry analysis using an Attune NxT Flow Cytometer (Thermo Fisher Scientific), the amount of antibody bound to the cells, and the binding amount of HER2-Fc were evaluated. Using BZ-X700 (Keyence), fluorescence microscopy was also performed for cells subjected to the same HER2 binding treatment.

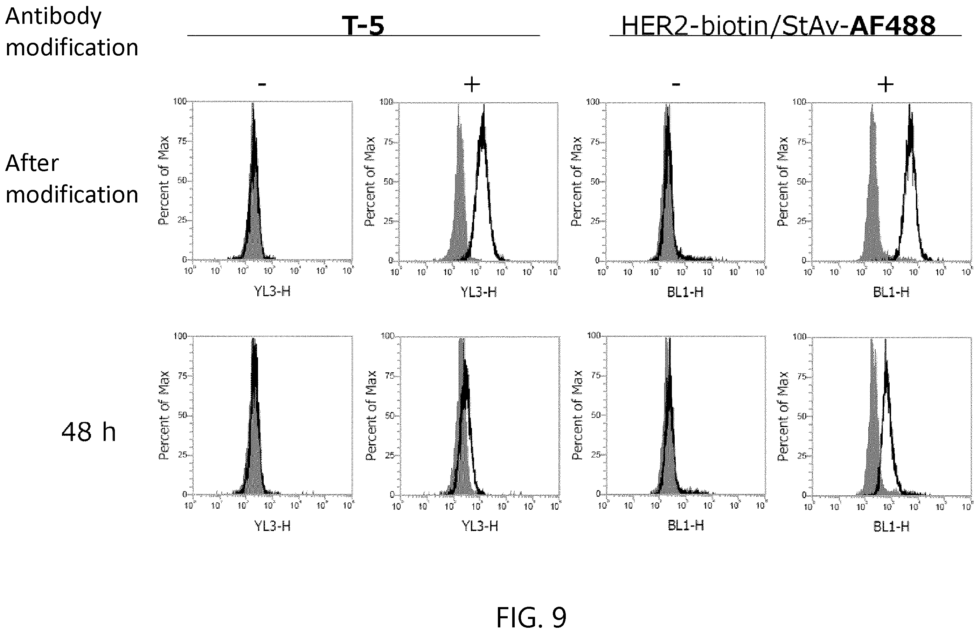

As shown in Fig. 9, from the cells conjugated with T-5 antibody, fluorescence derived from Cy5, with which the antibody was modified, and fluorescence derived from Streptavidin-Alexa 488 were detected, indicating that the cells were modified with the antibody, and that the human recombinant HER2-Fc was also bound thereto. On the other hand, the DBCO-modified cells and the unmodified cells showed neither fluorescence, indicating that HER2-Fc was not bound to the cells. From the above results, it was revealed that the T-5-cell conjugate acquired HER2-binding ability derived from the antibody. It was also confirmed that the HER2-binding ability was retained even after 48 hours.

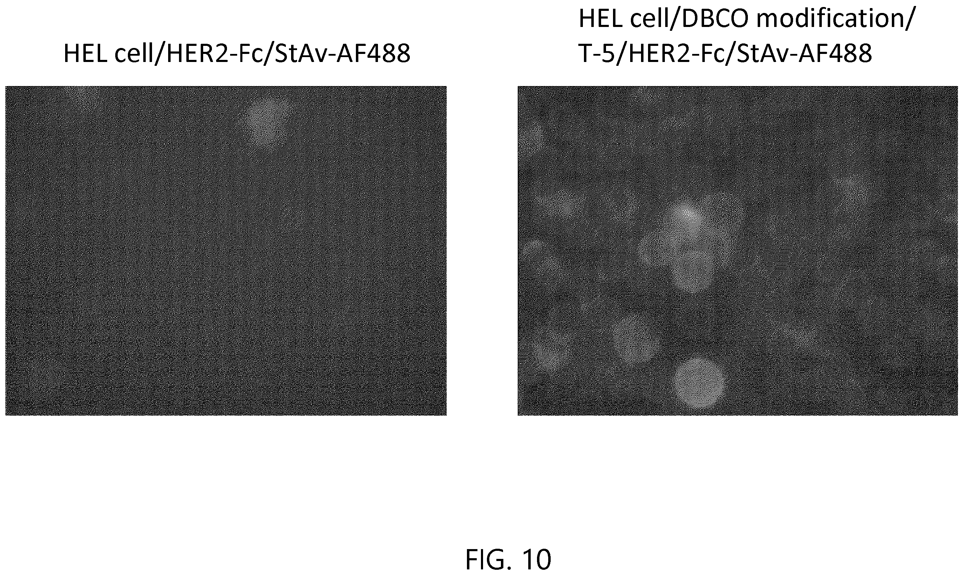

Further, as shown in Fig. 10, in the fluorescence microscopy, the cells conjugated with T-5 antibody showed fluorescence derived from Alexa Fluor 488 on the cell membrane, indicating that HER2 was bound to the antibody conjugated to the cells.

### Example 4 Comparison between Antibody-Cell Conjugated Body and NonConjugated Body

### Example 4-1 Synthesis of Antibody-Cell Conjugate and Preparation of NonConjugated Body

Synthesis of an antibody-cell conjugate was carried out as follows. HEL cells having a cell surface modified with DBCO were collected in an amount of 2 × 10⁶ cells each, and suspended in 200 µL of D-MEM/F12 medium. After adding 1 µM, the resulting mixture was left to stand at 37 °C for 1 hour. Centrifugation was carried out, and then the supernatant was removed. Thereafter, a step of adding 1 mL of PBS to the cells, washing the cells, performing centrifugation, and then removing the supernatant was repeated twice to obtain an antibody-cell complex. A non-conjugated body was prepared by performing the above-described operation for HEL cells whose cell surface had not been modified with DBCO.

### Example 4-2 Evaluation of HER2 Binding of Antibody-Cell Conjugate and Non-Conjugated Body

In the same manner as described above, the HER2-binding ability of each antibody-cell conjugate was evaluated by flow cytometry analysis.

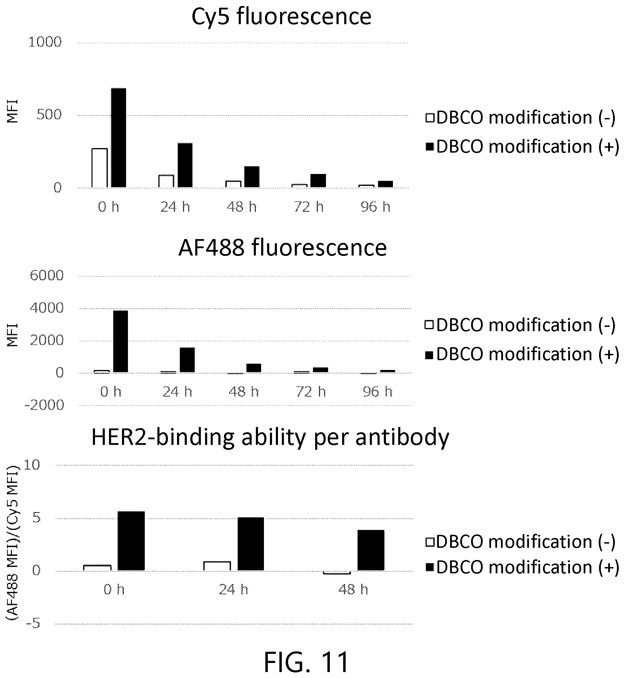

As shown in Fig. 11, the proportion of cells exhibiting fluorescence of Streptavidin-Alexa Fluor 488 was higher in the antibody-cell conjugate group than in the non-conjugate group. In addition, in each group, the amount of HER2 binding per antibody bound to the cells was determined by calculating (fluorescence intensity of Streptavidin-Alexa Fluor 488)/(fluorescence intensity of Cy5). As a result, the conjugate group showed a value that was not less than 6 times higher. Thus, it became clear that, by conjugating the antibody to the cells, the antigen-binding ability of the antibody was maintained higher than that of the non-conjugated body.

### Example 5 Comparison of Various Azide-Modified Antibody-Cell Conjugates

### Example 5-1 Synthesis of Antibody-Cell Conjugates

HEL cells having a cell surface modified with DBCO were collected in an amount of 2 × 10⁶ cells each, and suspended in 200 µL of D-MEM/F12 medium. After adding 0.3 µM or 1 µM T-5, T-8, or T-9 to the suspension, the resulting mixture was left to stand at 37 °C for 1 hour. Centrifugation was carried out, and then the supernatant was removed. Thereafter, a step of adding 1 mL of PBS to the cells, washing the cells, performing centrifugation, and then removing the supernatant was repeated twice to obtain an antibody-cell conjugate body.

### Example 5-2 Evaluation of HER2 Binding of Antibody-Cell Conjugates

In the same manner as described above, HER2 binding of each antibody-cell conjugate was evaluated using flow cytometry.

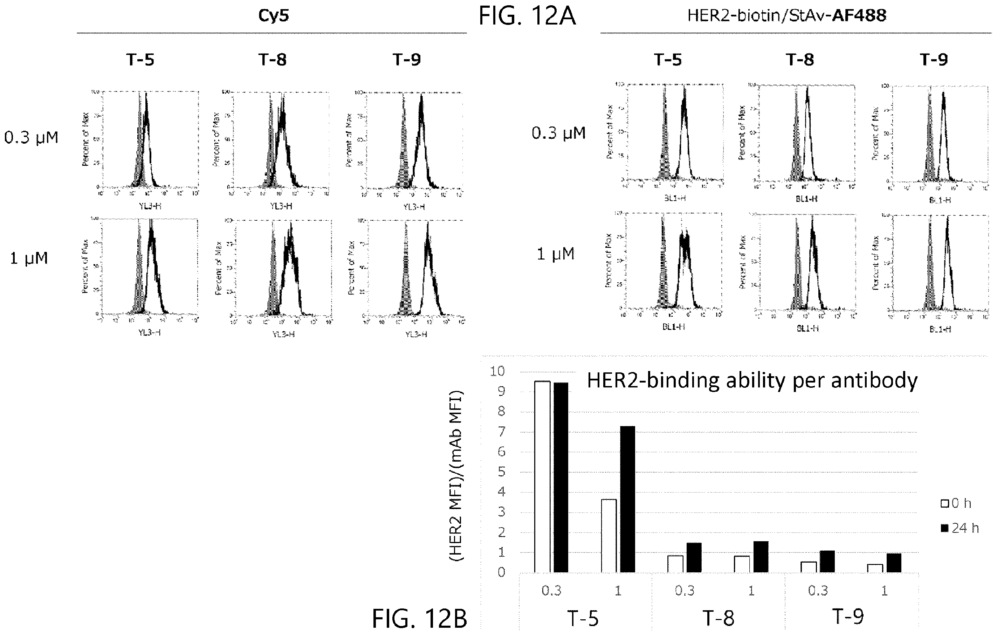

As shown in Fig. 12A, from all types of cells to which the modified antibodies were conjugated, fluorescence derived from Cy5, with which the antibodies were modified, and fluorescence derived from Streptavidin-Alexa 488 were detected. From this result, it was confirmed that the cells were modified with each antibody, and that human recombinant HER2-Fc was bound to the cells.

In each group, the amount of HER2 binding per antibody bound to the cells was determined by calculating (fluorescence intensity of Streptavidin-Alexa Fluor 488)/(fluorescence intensity of Cy5). As a result, as shown in Fig. 12B, the antibody-cell conjugate modified with T-5 showed a value that was not less than 6 times higher than those of the antibody-cell conjugates modified with T-8 or T-9. From this, it became clear that, by conjugating a sitespecifically modified antibody to cells, an antibody-cell conjugate having higher antigen-binding ability can be obtained compared to antibody-cell conjugates synthesized using an antibody in which lysine residues on the antibody were randomly modified or using an antibody in which sulfhydryl groups generated by reduction of disulfide bonds on the antibody were modified.

### Example 6 Synthesis of Cy5 Antibody-T Cell Conjugate and Evaluation of HER2-Binding Ability

### Example 6-1 Culture of T Cells

A 24-well plate was coated with DPBS supplemented with 5 µg/mL anti-CD3 antibody (Thermo Fisher Scientific) and 25 µg/mL RetroNectin (Takara Bio). The plate was washed three times with DPBS, T cells were added at 6×10⁴ cells/well to a medium supplemented with Optimizer (Thermo Fisher Scientific) and IL-2 (PeproTech), and cultured.

### Example 6-2 Synthesis of T Antibody-Cell Conjugate

After the T cells were collected, the cell count and viability were measured using a Countess Automated Cell Counter (Thermo Fisher Scientific). T cells in an amount of 2.5×10⁷ cells were collected and centrifuged (at 6000 rpm for 15 seconds), and then the supernatant was removed. One milliliter of PBS was added to the T cells to wash the cells, and centrifugation was performed, followed by removing the supernatant. One milliliter of 0.1 mg/mL Traut's reagent (Sigma-Aldrich)/PBS was added to the T cells, and then the cells were incubated at room temperature for 20 minutes. Centrifugation was carried out, and then the supernatant was removed. Thereafter, a step of adding 1 mL of PBS to the cells, washing the cells, performing centrifugation, and then removing the supernatant was repeated twice to obtain T cells having a cell surface modified with sulfhydryl groups. Subsequently, 1 mL of 20 µM dibenzocyclooctyne (DBCO)-PEG4-maleimide (BroadPharm)/PBS was added to the T cells, and the cells were incubated at room temperature for 30 minutes. Centrifugation was carried out, and then the supernatant was removed. Thereafter, a step of adding 1 mL of PBS to the cells, washing the cells, performing centrifugation, and then removing the supernatant was repeated twice to obtain T cells having a cell surface modified with DBCO.

T cells having a cell surface modified with DBCO, or T cells having an unmodified cell surface were collected in an amount of 0.5 × 10⁶ cells each, and suspended in 200 µL of a medium for T cells. To each type of cells, 0.33 µM or 1 µM T-4 was added, and the cells were incubated at 37 °C for 1 hour. Centrifugation was carried out, and then the supernatant was removed. Thereafter, a step of adding 1 mL of PBS to the cells, washing the cells, performing centrifugation, and then removing the supernatant was repeated twice to obtain an antibody-cell complex.

### Example 6-3 Evaluation of Antibody-Cell Conjugate

The amount of antibody bound to the cells was evaluated by subjecting 0.2 × 10⁶ cells to flow cytometry analysis using an Attune NxT Flow Cytometer (Thermo Fisher Scientific).

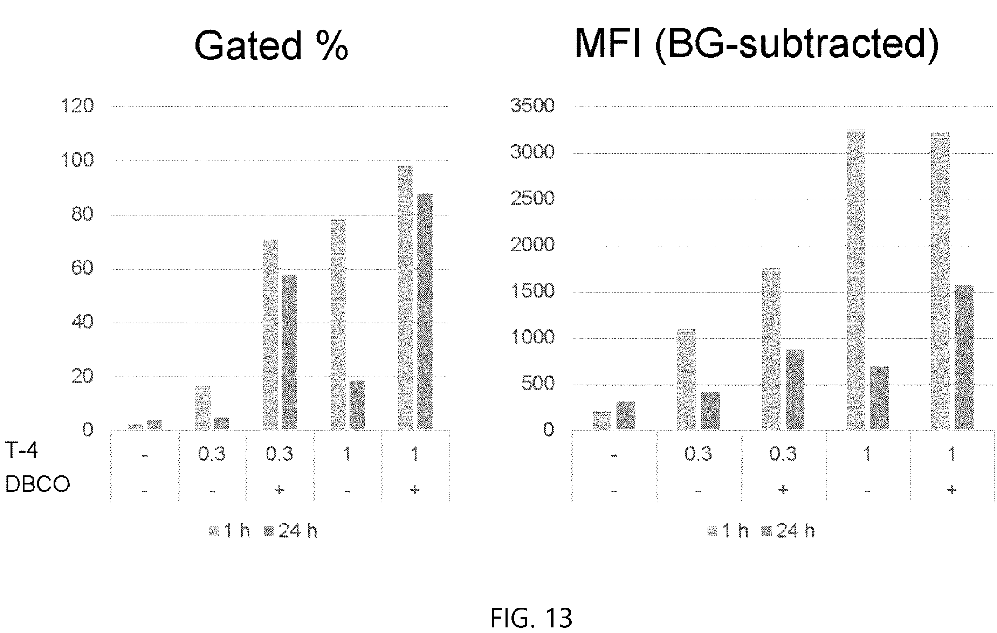

As shown in Fig. 13, the group in which T-4 antibody was reacted with the DBCO-modified cells had stronger fluorescence than the group in which T-4 antibody was reacted with the cells that had not been modified with DBCO, indicating that the antibody could be bound with higher efficiency in the former group.

### <Reference Examples>

As Reference Examples, a method in which an affinity substance containing two affinity peptides is used to synthesize a compound containing the affinity substance and a bioorthogonal functional group, and a method in which the compound is used to introduce the bioorthogonal functional group to only one of the heavy chain constant region-derived polypeptides constituting the Fc region of an antibody, are described below. By using the thus obtained antibody, in which the bioorthogonal functional group is introduced to only one of the heavy chain constant region-derived polypeptides constituting the Fc region, it is also possible to obtain an Fc-cell complex in which an antibody or an Fc fusion protein is chemically bound to a cell surface via a linker introduced to a functional group in the side chain of a particular amino acid residue present at one or more positions of one of the Fc-constituting heavy chain constant region-derived polypeptides.

### (Reference Example 1) Design of Affinity Substance and Secretory Expression of Affinity Substance in C. glutamicum

### (1-1) Outline of Design of Affinity Substance

In the present invention, an affinity substance containing, in the molecule, two or more sites capable of binding to an antibody needs to be designed. In other words, the affinity substance needs to be designed so as to be:
A) a molecule containing two or more sites capable of binding to an antibody (and optionally, a linker for linking these sites).

Further, in the present invention, in order to enable modification of an immunoglobulin unit, a compound having a reactive group for the immunoglobulin unit is preferably bound to the affinity substance.

Further, in order to remove the affinity substance after the modification of the immunoglobulin unit, a cleavable portion is preferably contained between the reactive group and the affinity substance.

Further, in order to achieve specific binding of the compound to a particular site in the affinity substance, the affinity substance is preferably designed such that only one site capable of reacting with the compound is present therein. Therefore, in cases where an affinity polypeptide is used as the affinity substance, and the affinity polypeptide is bound to the compound via the amino group in the side chain of a lysine residue (K) in the affinity polypeptide, the affinity polypeptide is preferably designed such that only one lysine residue is present in the affinity polypeptide. Accordingly, affinity polypeptides were designed as follows:
B) each polypeptide contains only one K residue; and
C) the N-terminal amino acid is set to Q (glutamine) to cause pyroglutamylation, thereby converting the N-terminal amino group to an amide.

In accordance with the above rules A), B), and C), polypeptide-based affinity substances were designed as (a) to (h) below.

### (1-2) Preparation of Affinity Substances

The following affinity substances were prepared.
(a) QET-Z34CM-PA32-Fc3K 
QET-FNMQCQRRFYEALHDPNLNEEQRNARIRSIREEC-
GGAAPAAPAPAAPAAPAPAAPAAPAPAAPAAPAPGG-RGNCAYHKGQIIWCTYH-NH₂
(SEQ ID NO: 17)
(b) QET-Z34CM-PA48-Fc3K 
QET-FNMQCQRRFYEALHDPNLNEEQRNARIRSIREEC-
GGAAPAAPAPAAPAAPAPAAPAAPAPAAPAAPAPAAPAAPAPAAPAAPAPGG-
RGNCAYHKGQIIWCTYH-NH2 (SEQ ID NO: 18)
(c) QET-Fc3K-PA32-Z34CM 
QETRGNCAYHKGQIIWCTYH-
GGAAPAAPAPAAPAAPAPAAPAAPAPAAPAAPAPGG-
FNMQCQRRFYEALHDPNLNEEQRNARIRSIREEC-NH2 (SEQ ID NO: 19)
(d) QET-Fc3K-PA48-Z34CM QETRGNCAYHKGQIIWCTYH- 
GGAAPAAPAPAAPAAPAPAAPAAPAPAAPAAPAPAAPAAPAPAAPAAPAPAAPAAPAPGG-
FNMQCQRRFYEALHDPNLNEEQRNARIRSIREEC-NH2 (SEQ ID NO: 20)
(e) QET-Fc3K-PA32-ProAR 
QETRGNCAYHKGQIIWCTYH-
GGAAPAAPAPAAPAAPAPAAPAAPAPAAPAAPAPGG-
FNREQQNAFYEILHLPNLNEEQRNGFIQSLRDDPSQSANLLAEA-NH2 (SEQ ID NO: 21)
(f) QET-Fc3K-PA48-ProAR 
QETRGNCAYHKGQIIWCTYH-
GGAAPAAPAPAAPAAPAPAAPAAPAPAAPAAPAPAAPAAPAPAAPAAPAPGG-
FNREQQNAFYEILHLPNLNEEQRNGFIQSLRDDPSQSANLLAEA-NH2 (SEQ ID NO: 22)
(g) QET-ProAR-PA32-Z34CK 
QET-FNREQQNAFYEILHLPNLNEEQRNGFIQSLRDDPSQSANLLAEA-
GGAAPAAPAPAAPAAPAPAAPAAPAPAAPAAPAPGG-
FNKQCQRRFYEALHDPNLNEEQRNARIRSIREEC-NH2 (SEQ ID NO: 23)
(h) QET-ProAR-PA48-Z34CK

QET-FNREQQNAFYEILHLPNLNEEQRNGFIQSLRDDPSQSANLLAEA-
GGAAPAAPAPAAPAAPAPAAPAAPAPAAPAAPAPAAPAAPAPAAPAAPAPGG-
FNKQCQRRFYEALHDPNLNEEQRNARIRSIREEC-NH2 (SEQ ID NO: 24)

### (1-3) Expression of Affinity Substances

Expression studies of these polypeptide-based affinity substances were carried out using Corynex^{®}. In expression using Corynex^{®}, the CspB fusion method (WO2013/062029) was utilized. Specifically, in this technique, a nucleotide sequence encoding an amino acid sequence containing the three N-terminal residues Gln-Glu-Thr (QET) of CspB mature protein is inserted between a nucleotide sequence encoding a signal peptide and a nucleotide sequence encoding a polypeptide of interest, to thereby improve the secretory production of the polypeptide of interest. Further, since the N-terminus of the CspB tag is Q, the Q at the first residue is pyroglutamylated after cleavage of the signal sequence, so that the N-terminal amino group can be protected, which is advantageous. Thus, expression of an affinity polypeptide using Corynex^{®} can be effectively improved by taking into account not only the above rules A), B), and C), but also the following rule D):
D) the three residues QET are added to the N-terminus to improve the secretion efficiency in Corynex^{®}.

Reference Examples of expression studies using Corynex^{®} are described below.

### (1-4) Construction of Secretory Expression Plasmids QET-Z34CM-PA32-Fc3K, QET-Z34CM-PA48-Fc3K, QET-Fc3K-PA32-Z34CM, QET-Fc3K-PA48-Z34CM, QET-Fc3K-PA32-ProAR, QET-Fc3K-PA48-ProAR, QET-ProAR-PA32-Z34CK, and QET-ProAR-PA48-Z34CK

As affinity polypeptides, eight amino acid sequences were designed for the above QET-Z34CM-PA32-Fc3K, QET-Z34CM-PA48-Fc3K, QET-Fc3K-PA32-Z34CM, QET-Fc3K-PA48-Z34CM, QET-Fc3K-PA32-ProAR, QET-Fc3K-PA48-ProAR, QET-ProAR-PA32-Z34CK, and QET-ProAR-PA48-Z34CK, respectively, and nucleotide sequences encoding these polypeptides were designed in consideration of the codon usage of C. *glutamicum.* Further, the following expression cassettes were each designed so as to enable secretory expression by C. *glutamicum.*

QET-Z34CM-PA32-Fc3K was produced by secretory expression as the fusion protein comprising the 30-amino-acid CspB signal peptide derived from the C. glutamicum ATCC13869 strain, the three N-terminal amino acid residues, QET, of the mature CspB protein derived from the same strain, and Z34CM-PA32-Fc3K (hereinafter referred to as "CspBss-QET-Z34CM-PA32-Fc3K"). The designed nucleotide sequence encoding CspBss-QET-Z34CM-PA32-Fc3K and the amino acid sequence encoded thereby are shown in SEQ ID NOs: 25 and 26, respectively.
Nucleotide sequence encoding CspBss-QET-Z34CM-PA32-Fc3K 
atgtttaacaaccgtatccgcactgcagctctcgctggtgcaatcgcaatctccaccgcagcttccggcgtagctatcccagc
attcgctcaggagaccttcaacatgcagtgccaacggcgcttttacgaagcgctccacgatcccaacctgaacgaggaacagcgtaacgc
gcgcattcgctccatccgtgaggaatgtggcggtgctgcccctgctgccccagcaccggcagcacctgcggcaccagctccggcagctc
cagctgcaccggcccctgccgctccagcagcgccagctcccggaggtcgaggcaattgcgcctatcacaaggggcagatcatctggtgc
acttaccattaa (SEQ ID NO: 25)
Amino acid sequence of CspBss-QET-Z34CM-PA32-Fc3K

MFNNRIRTAALAGAIAISTAASGVAIPAFAQETFNMQCQRRFYEALHDPNLNEE
QRNARIRSIREECGGAAPAAPAPAAPAAPAPAAPAAPAPAAPAAPAPGGRGNCAYHKGQII
WCTYH (SEQ ID NO: 26)

QET-Z34CM-PA48-Fc3K was produced by secretory expression as the fusion protein comprising the 30-amino-acid CspB signal peptide derived from the C. glutamicum ATCC13869 strain, the three N-terminal amino acid residues, QET, of the mature CspB protein derived from the same strain, and Z34CM-PA48-Fc3K (hereinafter referred to as "CspBss-QET-Z34CM-PA48-Fc3K"). The designed nucleotide sequence encoding CspBss-QET-Z34CM-PA48-Fc3K and the amino acid sequence encoded thereby are shown in SEQ ID NOs: 27 and 28, respectively.
Nucleotide sequence encoding CspBss-QET-Z34CM-PA48-Fc3K 
atgtttaacaaccgtatccgcactgcagctctcgctggtgcaatcgcaatctccaccgcagcttccggcgtagctatcccagc
attcgctcaggagacctttaacatgcagtgccaacgtcgtttttacgaagcgctgcacgatcccaacctgaacgaggaacagcgcaatgcg
cgaatacgctccattcgcgaggaatgtggcggagcagctcccgcggctcctgctccagcagcgcctgctgctcctgcccctgcggcacct
gcagcacctgcaccagcagctccggcagcaccagctccagctgcacccgccgccccagctcctgcagcgccagccgctccggccccc
ggaggtaggggcaactgcgcatatcacaaggggcagataatctggtgcacttatcactaa (SEQ ID NO: 27)
Amino acid sequence of CspBss-QET-Z34CM-PA48-Fc3K

MFNNRIRTAALAGAIAISTAASGVAIPAFAQETFNMQCQRRFYEALHDPNLNEE
QRNARIRSIREECGGAAPAAPAPAAPAAPAPAAPAAPAPAAPAAPAPAAPAAPAPAAPAAPA
PGGRGNCAYHKGQIIWCTYH (SEQ ID NO: 28)

QET-Fc3K-PA32-Z34CM was produced by secretory expression as the fusion protein comprising the 30-amino-acid CspB signal peptide derived from the C. glutamicum ATCC13869 strain, the three N-terminal amino acid residues, QET, of the mature CspB protein derived from the same strain, and Fc3K-PA32-Z34CM (hereinafter referred to as "CspBss-QET-Fc3K-PA32-Z34CM"). The designed nucleotide sequence encoding CspBss-QET-Fc3K-PA32-Z34CM and the amino acid sequence encoded thereby are shown in SEQ ID NOs: 29 and 30, respectively.
Nucleotide sequence encoding CspBss-QET-Fc3K-PA32-Z34CM 
atgtttaacaaccgtatccgcactgcagctctcgctggtgcaatcgcaatctccaccgcagcttccggcgtagctatcccagc
attcgctcaggagacccgtggcaactgtgcctaccacaagggccagatcatctggtgcacgtaccacggaggtgccgcgcctgccgcac
ctgctccagctgctcccgctgcccctgcaccagctgcgccagcagcaccagctccagcggcacctgcagcaccggcacctggtggcttc
aacatgcagtgccaacgacgcttctacgaggctctgcacgatccgaacctcaacgaggaacagcgcaatgcccgtattcggtccatcagg
gaggaatgctaa (SEQ ID NO: 29)
Amino acid sequence of CspBss-QET-Fc3K-PA32-Z34CM

MFNNRIRTAALAGAIAISTAASGVAIPAFAQETRGNCAYHKGQIIWCTYHGGAA
PAAPAPAAPAAPAPAAPAAPAPAAPAAPAPGGFNMQCQRRFYEALHDPNLNEEQRNARIR
SIREEC (SEQ ID NO: 30)

QET-Fc3K-PA48-Z34CM was produced by secretory expression as the fusion protein comprising the 30-amino-acid CspB signal peptide derived from the C. glutamicum ATCC13869 strain, the three N-terminal amino acid residues, QET, of the mature CspB protein derived from the same strain, and Fc3K-PA48-Z34CM (hereinafter referred to as "CspBss-QET-Fc3K-PA48-Z34CM"). The designed nucleotide sequence encoding CspBss-QET-Fc3K-PA48-Z34CM and the amino acid sequence encoded thereby are shown in SEQ ID NOs: 31 and 32, respectively.
Nucleotide sequence encoding CspBss-QET-Fc3K-PA48-Z34CM 
atgtttaacaaccgtatccgcactgcagctctcgctggtgcaatcgcaatctccaccgcagcttccggcgtagctatcccagc
attcgctcaggagacccgtggcaattgcgcctatcacaagggtcagatcatttggtgcacctaccatggtggtgcagcaccagctgccccg
 
gctccggctgcgcctgctgcacctgcgccagctgcacctgcagcaccggcacctgctgctccagcagcacccgctcctgcagctcccgct
gccccagctccagcggcgcctgcggcgccagcacctggagggttcaacatgcagtgccaacgtcgcttttacgaggctttgcatgatccta
acttgaacgaggaacagcggaatgcgcggatacgctccatacgcgaagaatgttaa (SEQ ID NO: 31)
Amino acid sequence of CspBss-QET-Fc3K-PA48-Z34CM

MFNNRIRTAALAGAIAISTAASGVAIPAFAQETRGNCAYHKGQIIWCTYHGGAA
PAAPAPAAPAAPAPAAPAAPAPAAPAAPAPAAPAAPAPAAPAAPAPGGFNMQCQRRFYEAL
HDPNLNEEQRNARIRSIREEC (SEQ ID NO: 32)

QET-Fc3K-PA32-ProAR was produced by secretory expression as the fusion protein comprising the 30-amino-acid CspB signal peptide derived from the C. glutamicum ATCC13869 strain, the three N-terminal amino acid residues, QET, of the mature CspB protein derived from the same strain, and Fc3K-PA32-ProAR (hereinafter referred to as "CspBss-QET-Fc3K-PA32-ProAR"). The designed nucleotide sequence encoding CspBss-QET-Fc3K-PA32-ProAR and the amino acid sequence encoded thereby are shown in SEQ ID NOs: 33 and 34, respectively.
Nucleotide sequence encoding CspBss-QET-Fc3K-PA32-ProAR 
atgtttaacaaccgtatccgcactgcagctctcgctggtgcaatcgcaatctccaccgcagcttccggcgtagctatcccagc
attcgctcaggagacccgcggcaactgtgcctaccacaaggggcagatcatctggtgcacctatcacggtggtgcggctccggctgctcc
agcgccagcagctcccgctgcaccagccccagcggcacctgccgcacctgcacctgcagctccagcggctcccgcaccgggaggcttt
aaccgcgaacagcagaacgccttctacgagattctccatctgcccaacctcaacgaggagcaacggaatggcttcatccagagcttgcgtg
acgatccgtctcaatccgccaatctgcttgcggaagcctaa (SEQ ID NO: 33)
Amino acid sequence of CspBss-QET-Fc3K-PA32-ProAR

MFNNRIRTAALAGAIAISTAASGVAIPAFAQETRGNCAYHKGQIIWCTYHGGAA
PAAPAPAAPAAPAPAAPAAPAPAAPAAPAPGGFNREQQNAFYEILHLPNLNEEQRNGFIQS
LRDDPSQSANLLAEA (SEQ ID NO: 34)

QET-Fc3K-PA48-ProAR was produced by secretory expression as the fusion protein comprising the 30-amino-acid CspB signal peptide derived from the C. glutamicum ATCC13869 strain, the three N-terminal amino acid residues, QET, of the mature CspB protein derived from the same strain, and Fc3K-PA48-ProAR (hereinafter referred to as "CspBss-QET-Fc3K-PA48-ProAR"). The designed nucleotide sequence encoding CspBss-QET-Fc3K-PA48-ProAR and the amino acid sequence encoded thereby are shown in SEQ ID NOs: 35 and 36, respectively.
Nucleotide sequence encoding CspBss-QET-Fc3K-PA48-ProAR 
atgtttaacaaccgtatccgcactgcagctctcgctggtgcaatcgcaatctccaccgcagcttccggcgtagctatcccagc
attcgctcaggagacccgtgggaactgcgcgtaccataagggccagatcatctggtgcacctatcatggcggtgctgcgcctgcagctccg
gctcctgccgctccagccgcgccagccccagcagctccagcagcacctgcacctgctgcaccagcggctccagctcctgcggcacccg
ctgcaccggcaccagccgctccggctgccccagctccgggtggctttaaccgcgaacagcagaacgccttctacgagatactccaccttc
 
ccaacctgaacgaggagcagcgaaacggcttcatccagtctcttcgcgatgatccatcccagagcgctaacttgcttgcagaagcgtaa
(SEQ ID NO: 35)
Amino acid sequence of CspBss-QET-Fc3K-PA32-ProAR

MFNNRIRTAALAGAIAISTAASGVAIPAFAQETRGNCAYHKGQIIWCTYHGGAA
PAAPAPAAPAAPAPAAPAAPAPAAPAAPAPAAPAAPAPAAPAAPAPGGFNREQQNAFYEIL
HLPNLNEEQRNGFIQSLRDDPSQSANLLAEA (SEQ ID NO: 36)

QET-ProAR-PA32-Z34CK was produced by secretory expression as the fusion protein comprising the 30-amino-acid CspB signal peptide derived from the C. glutamicum ATCC13869 strain, the three N-terminal amino acid residues, QET, of the mature CspB protein derived from the same strain, and ProAR-PA32-Z34CK (hereinafter referred to as "CspBss-QET-ProAR-PA32-Z34CK"). The designed nucleotide sequence encoding CspBss-QET-ProAR-PA32-Z34CK and the amino acid sequence encoded thereby are shown in SEQ ID NOs: 37 and 38, respectively.
Nucleotide sequence encoding CspBss-QET-ProAR-PA32-Z34CK 
atgtttaacaaccgtatccgcactgcagctctcgctggtgcaatcgcaatctccaccgcagcttccggcgtagctatcccagc
attcgctcaggagacctttaaccgcgaacagcagaacgccttctacgagatcctgcatctccccaatctgaacgaagagcagaggaatggc
tttatccagagccttcgtgacgatccgtcgcaatctgccaacctgttggcggaagctggaggtgccgcgccagcggcaccagcaccagct
gcaccggctgctcccgcacctgccgcaccagccgctcctgccccagcagctccggcagctcctgcacctggtggcttcaacaagcagtg
ccaacgtcgcttctatgaagcgctacacgatcccaacctcaacgaggaacagcgcaatgcgcgaatccggtccattcgcgaagagtgttaa
(SEQ ID NO: 37)
Amino acid sequence of CspBss-QET-ProAR-PA32-Z34CK

MFNNRIRTAALAGAIAISTAASGVAIPAFAQETFNREQQNAFYEILHLPNLNEEQ
RNGFIQSLRDDPSQSANLLAEAGGAAPAAPAPAAPAAPAPAAPAAPAPAAPAAPAPGGFN
KQCQRRFYEALHDPNLNEEQRNARIRSIREEC (SEQ ID NO: 38)

QET-ProAR-PA48-Z34CK was produced by secretory expression as the fusion protein comprising the 30-amino-acid CspB signal peptide derived from the C. glutamicum ATCC13869 strain, the three N-terminal amino acid residues, QET, of the mature CspB protein derived from the same strain, and ProAR-PA48-Z34CK (hereinafter referred to as "CspBss-QET-ProAR-PA48-Z34CK"). The designed nucleotide sequence encoding CspBss-QET-ProAR-PA48-Z34CK and the amino acid sequence encoded thereby are shown in SEQ ID NOs: 39 and 40, respectively.
Nucleotide sequence encoding CspBss-QET-ProAR-PA48-Z34CK 
atgtttaacaaccgtatccgcactgcagctctcgctggtgcaatcgcaatctccaccgcagcttccggcgtagctatcccagc
attcgctcaggagacctttaaccgcgaacagcagaacgccttctacgagatcctccatctgccaaacctgaacgaagaacagcgcaacgg
 
ctttatccagtccctcagggacgatccgtctcagtcggccaatttgctagccgaagcgggaggtgctgctccggcagcacccgcaccagcc
gcgcccgctgctccagcgccggcagctcctgcggcaccagctccagcagcaccagcggctccagcacctgccgcacctgcagctccgg
ctcctgcagcccccgccgctcctgcgccgggtggcttcaacaagcagtgccaacgacgcttctatgaggcgcttcacgatcccaacctgaa
tgaggagcaacggaatgcccgtatccgtagcattcgcgaagaatgttaa (SEQ ID NO: 39)
Amino acid sequence of CspBss-QET-ProAR-PA48-Z34CK

MFNNRIRTAALAGAIAISTAASGVAIPAFAQETFNREQQNAFYEILHLPNLNEEQ
RNGFIQSLRDDPSQSANLLAEAGGAAPAAPAPAAPAAPAPAAPAAPAPAAPAAPAPAAPAA
PAPAAPAAPAPGGFNKQCQRRFYEALHDPNLNEEQRNARIRSIREEC (SEQ ID NO: 40)

Expression cassettes for the eight affinity polypeptides were designed such that the promoter of the cspB gene derived from the C. *glutamicum* ATCC13869 strain was linked upstream of the nucleotide sequence described for CspBss-QET-Z34CM-PA32-Fc3K, CspBss-QET-Z34CM-PA48-Fc3K, CspBss-QET-Fc3K-PA32-Z34CM, CspBss-QET-Fc3K-PA48-Z34CM, CspBss-QET-Fc3K-PA32-ProAR, CspBss-QET-Fc3K-PA48-ProAR, CspBss-QET-ProAR-PA32-Z34CK, or CspBss-QET-ProAR-PA48-Z34CK, and such that a KpnI site and a BamHI site were added to the 5' side and the 3' side, respectively. These cassettes were prepared by total synthesis. Each DNA fragment (the expression cassette for each affinity polypeptide) prepared by total synthesis was inserted into the KpnI-BamHI site of pPK4 described in JP 9-322774 A, to construct each of the following secretory expression plasmids for the affinity polypeptides: pPK4_CspBss-QET-Z34CM-PA32-Fc3K, pPK4_CspBss-QET-Z34CM-PA48-Fc3K, pPK4_CspBss-QET-Fc3K-PA32-Z34CM, pPK4_CspBss-QET-Fc3K-PA48-Z34CM, pPK4_CspBss-QET-Fc3K-PA32-ProAR, pPK4_CspBss-QET-Fc3K-PA48-ProAR, pPK4_CspBss-QET-ProAR-PA32-Z34CK, and pPK4_CspBss-QET-ProAR-PA48-Z34CK. As a result of determination of the nucleotide sequence of each inserted fragment, it was confirmed that the designed expression cassette for each affinity polypeptide was constructed. The nucleotide sequences were determined using a BigDye^{®} Terminator v3.1 Cycle Sequencing Kit (Applied Biosystems) and a 3500xL Genetic Analyzer (Applied Biosystems).

### (1-5) Secretory Expression of Affinity Polypeptides in C. glutamicum

The C. *glutamicum* YDK0107 strain, described in WO2016/171224, was transformed with pPK4_CspBss-QET-Z34CM-PA32-Fc3K, pPK4_CspBss-QET-Z34CM-PA48-Fc3K, pPK4_CspBss-QET-Fc3K-PA32-Z34CM, pPK4_CspBss-QET-Fc3K-PA48-Z34CM, pPK4_CspBss-QET-Fc3K-PA32-ProAR, pPK4_CspBss-QET-Fc3K-PA48-ProAR, pPK4_CspBss-QET-ProAR-PA32-Z34CK, and pPK4_CspBss-QET-ProAR-PA48-Z34CK constructed above, to obtain the YDK0107/pPK4_CspBss-QET-Z34CM-PA32-Fc3K strain, the YDK0107/pPK4_CspBss-QET-Z34CM-PA48-Fc3K strain, the YDK0107/pPK4_CspBss-QET-Fc3K-PA32-Z34CM strain, the YDK0107/pPK4_CspBss-QET-Fc3K-PA48-Z34CM strain, the YDK0107/pPK4_CspBss-QET-Fc3K-PA32-ProAR strain, the YDK0107/pPK4_CspBss-QET-Fc3K-PA48-ProAR strain, the YDK0107/pPK4_CspBss-QET-ProAR-PA32-Z34CK strain, and the YDK0107/pPK4_CspBss-QET-ProAR-PA48-Z34CK strain, respectively.

Each of the obtained transformants was cultured at 30°C for 72 hours in MMTG liquid medium (120 g of glucose, 3 g of magnesium sulfate heptahydrate, 30 g of ammonium sulfate, 1.5 g of potassium dihydrogen phosphate, 0.03 g of iron sulfate heptahydrate, 0.03 g of manganese sulfate pentahydrate, 0.45 mg of thiamine hydrochloride, 0.45 mg of biotin, 0.15 g of DL-methionine, soybean hydrochloric-acid hydrolysate (total nitrogen amount: 0.2 g), and 50 g of calcium carbonate; water was added to a final volume of 1 L; pH was adjusted to 7.0) supplemented with 25 mg/L kanamycin.

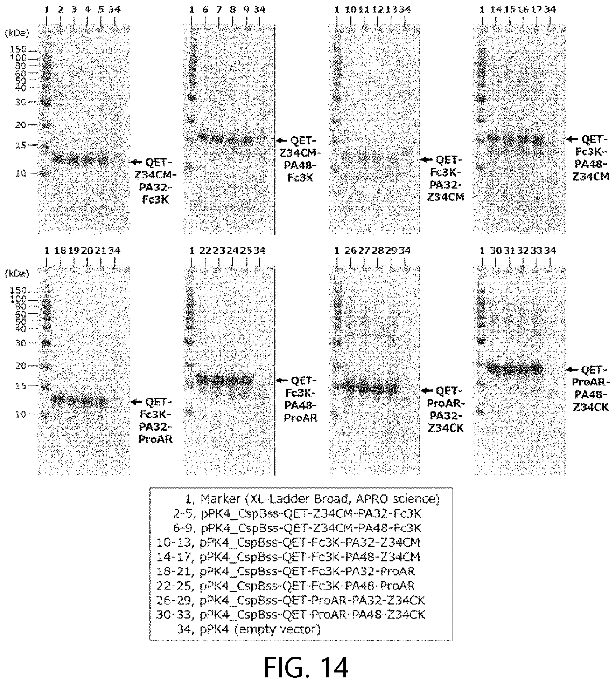

After completion of the culture, each culture liquid was centrifuged to obtain a culture supernatant, and 6.5 µL of the culture supernatant was subjected to reducing SDS-PAGE using NuPAGE^{®} 12% Bis-Tris Gel (Thermo Fisher Scientific), followed by staining with Quick-CBB (Wako). As a result, a polypeptide band assumed to correspond to QET-Z34CM-PA32-Fc3K was detected in the culture supernatant of the YDK0107/pPK4_CspBss-QET-Z34CM-PA32-Fc3K strain (Fig. 14, lanes 2 to 5); a polypeptide band assumed to correspond to QET-Z34CM-PA32-Fc3K was detected in the culture supernatant of the YDK0107/pPK4_CspBss-QET-Z34CM-PA32-Fc3K strain (Fig. 14, lanes 6 to 9); a polypeptide band assumed to correspond to QET-Fc3K-PA32-Z34CM was detected in the culture supernatant of the YDK0107/pPK4_CspBss-QET-Fc3K-PA32-Z34CM strain (Fig. 14, lanes 10 to 13); a polypeptide band assumed to correspond to QET-Fc3K-PA48-Z34CM was detected in the culture supernatant of the YDK0107/pPK4_CspBss-QET-Fc3K-PA48-Z34CM strain (Fig. 14, lanes 14 to 17); a polypeptide band assumed to correspond to QET-Fc3K-PA32-ProAR was detected in the culture supernatant of the YDK0107/pPK4_CspBss-QET-Fc3K-PA32-ProAR strain (Fig. 14, lanes 18 to 21); a polypeptide band assumed to correspond to QET-Fc3K-PA48-ProAR was detected in the culture supernatant of the YDK0107/pPK4_CspBss-QET-Fc3K-PA48-ProAR strain (Fig. 14, lanes 22 to 25); a polypeptide band assumed to correspond to QET-ProAR-PA32-Z34CK was detected in the culture supernatant of the YDK0107/pPK4_CspBss-QET-ProAR-PA32-Z34CK strain (Fig. 14, lanes 26 to 29); and a polypeptide band estimated to be QET-ProAR-PA48-Z34CK was detected in the culture supernatant of the YDK0107/pPK4_CspBss-QET-ProAR-PA48-Z34CK strain (Fig. 14, lanes 30 to 33).

### (Reference Example 2) Preparation of Compounds Containing Affinity Substance for Antibody, Cleavable Portion, and Reactive Group

A compound having a cleavable portion and a reactive group was bound to a purified polypeptide.

According to a previous publication (WO2019/0240287), affinity reagent (1) was prepared by amidating the affinity substance QET-Z34CM-PA32-Fc3K prepared in Reference Example 1-2. 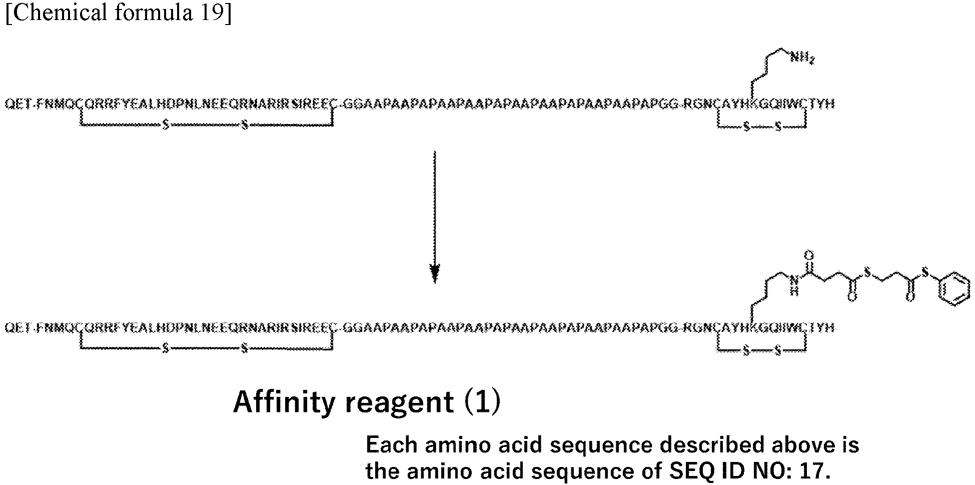
MS(ESI) m/z:z=11 885.80 [M+3H]³⁺
   Similarly, affinity reagent (2) was prepared from the affinity substance QET-Z34CM-PA48-Fc3K prepared in Reference Example 1-2.
MS(ESI) m/z:z=13 849.35 [M+3H]³⁺
   Similarly, affinity reagent (3) was prepared from the affinity substance QET-Fc3K-PA32-Z34CM prepared in Reference Example 1-2.
MS(ESI) m/z:z=11 886.15 [M+3H]³⁺
   Similarly, affinity reagent (4) was prepared from the affinity substance QET-Fc3K-PA48-Z34CM prepared in Reference Example 1-2.
MS (ESI) m/z:z=13 849.20 [M+3H]³⁺
   Similarly, affinity reagent (5) was prepared from the affinity substance QET-Fc3K-PA32-ProAR prepared in Reference Example 1-2.
MS (ESI) m/z:z=12 885.1 [M+3H]³⁺
   Similarly, affinity reagent (6) was prepared from the affinity substance QET-Fc3K-PA48-ProAR prepared in Reference Example 1-2.
MS (ESI) m/z:z=11 1082.00 [M+3H]³⁺
   Similarly, affinity reagent (7) was prepared from the affinity substance QET-ProAR-PA32-Z34CK prepared in Reference Example 1-2.
MS (ESI) m/z:z=15 855.20 [M+3H]³⁺
   Similarly, affinity reagent (8) was prepared from the affinity substance QET-ProAR-PA48-Z34CK prepared in Reference Example 1-2.
MS (ESI) m/z:z=17 830.75 [M+3H]³⁺

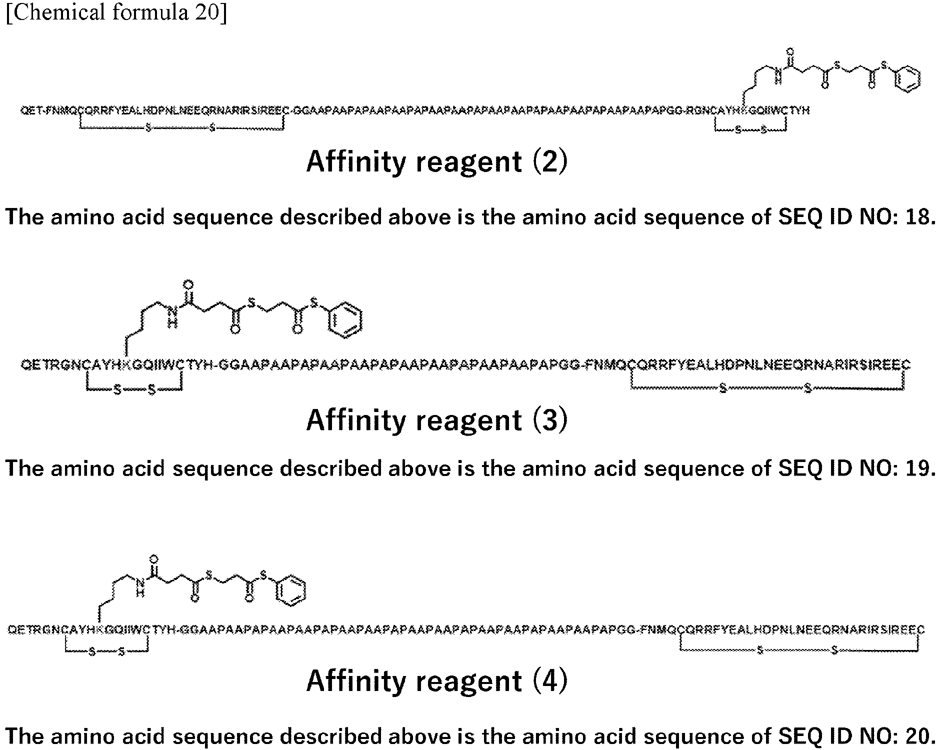

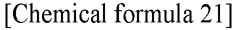

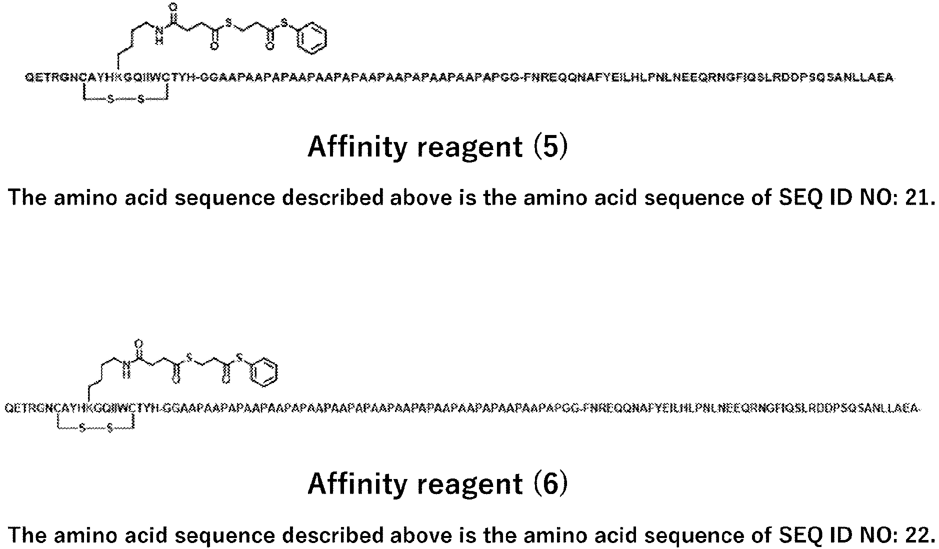

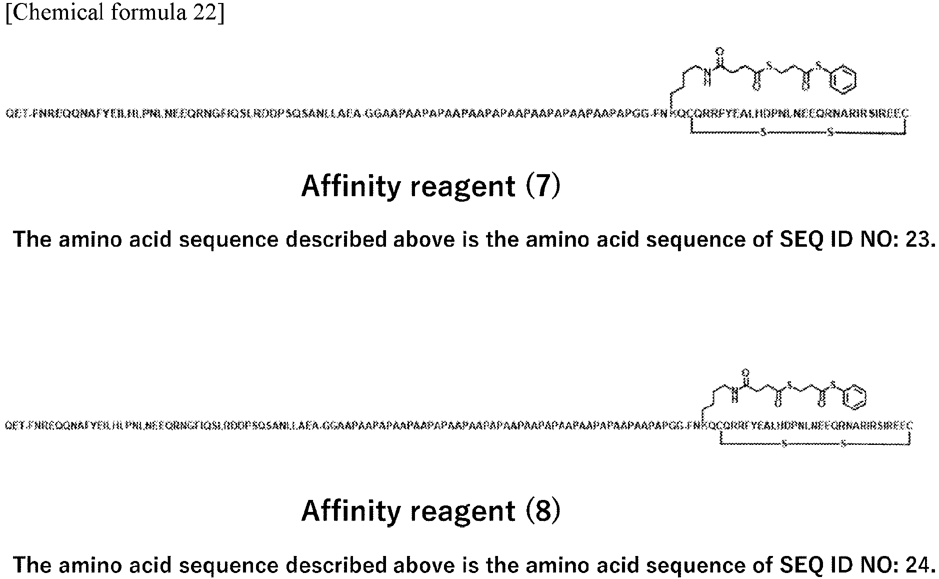

### (Reference Example 3) Specific Modification of Anti-HER2 IgG Antibody Trastuzumab and Synthesis of ADC Mimic

### (3-1) Specific Modification of Anti-HER2 Antibody Trastuzumab Using Affinity Reagent (4)

500 µg of an anti-HER2 IgG antibody trastuzumab (Chugai Pharmaceutical Co., Ltd.) was dissolved in 171 µL of 50 mM sodium acetate buffer (pH 5.5). A solution, in N,N'-dimethylformamide, of affinity reagent (4) synthesized in Reference Example 2 (10 equivalents) was added to the trastuzumab solution, and the resulting mixture was stirred at room temperature for 1 hour. After excess affinity reagent (4) was removed by ultrafiltration, mass measurement was performed by ESI-TOFMS. As a result, a peak was observed at 148,400 for the starting material, trastuzumab; a peak was observed at 159,300 for a product to which one binding peptide was introduced per Trastuzumab molecule; and a peak was confirmed at 170,390 for a product to which two binding peptides were introduced per Trastuzumab molecule.

### (3-2) Synthesis of Trastuzumab to Which One Thiol Group Was Introduced per Trastuzumab Molecule (T-6)

The antibody obtained above was subjected to a cleavage reaction for the thioester group (treatment with hydroxylamine) according to a previous publication (WO2019/240287), to obtain a thiol group-introduced antibody derivative (T-1-SH) having the following structural formula, to which one thiol group was introduced per antibody derivative molecule. Mass measurement by ESI-TOFMS confirmed a peak at 148,489, which corresponded to the product after the cleavage reaction had proceeded.

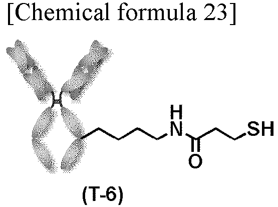

### (3-3) Specific Modification of Anti-HER2 Antibody Trastuzumab Using Affinity Reagent (6) and Synthesis of Thiol Group-Introduced Antibody Derivative by Cleavage of Thioester Group

Similarly, using affinity reagent (6) synthesized in Reference Example 2, a modification reaction of trastuzumab was carried out. After excess affinity reagent (6) was removed, mass measurement was performed by ESI-TOFMS. As a result, a peak was observed at 148,227 for the starting material, trastuzumab; a peak was observed at 160,165 for a product to which one binding peptide was introduced per Trastuzumab molecule; and a peak was confirmed at 171,953 for a product to which two binding peptides were introduced per Trastuzumab molecule. Table 1 shows the results of confirming the peptide/antibody binding ratio by DAR calculator (software manufactured by Agilent).

### [Table 1]

**Table 1**

| DAR Peak | Observed mass (Da) | Peak Area | %Area |
|---|---|---|---|
| 0 | 148227 | 9.64E+004 | 14.38 |
| 1 | 160165 | 5.24E+005 | 78.12 |
| 2 | 171953 | 4.99E+004 | 7.44 |

The antibody obtained above was subjected to a cleavage reaction for the thioester group according to a previous publication (WO2019/0240287), to obtain a thiol group-introduced antibody derivative to which one thiol group was introduced per antibody derivative molecule (T-1-SH). Mass measurement by ESI-TOFMS confirmed a peak at 148,489, corresponding to the product after the cleavage reaction had proceeded.

### (3-4) Peptide Mapping by Trypsin Treatment

For the thiol-introduced trastuzumab (T-1-SH) obtained in (3-2), peptide mapping was performed by the following steps.

### (3-4-1) Trypsin Treatment of Thiol-Introduced Trastuzumab

To a 1.5-mL low-adsorption microtest tube, 10 µL of the sample solution and 10 µL of an aqueous solution of 20 mM dithiothreitol in 150 mM Tris-hydrochloric acid buffer (pH 8.0) and 40% trifluoroethanol were added, and the resulting mixture was heated at 65°C for 1 hour. Thereafter, 10 µL of 50 mM aqueous iodoacetamide solution was added thereto, and then the resulting mixture was allowed to react under light-shielded conditions at room temperature for 30 minutes. Thereafter, 40 µL of 150 mM Tris-hydrochloric acid buffer (pH 8.0) was added, and the resulting mixture was stirred, followed by adding 10 µL of 20 ng/µL aqueous trypsin solution and allowing enzymatic digestion to proceed at 37°C for 16 hours. Thereafter, 2 µL of 20% aqueous trifluoroacetic acid solution was added to stop the reaction, and the resulting sample solution was diluted 10-fold with 0.1% formic acid and 2% aqueous acetonitrile solution. The diluted sample solution was subjected to LC-MS/MS measurement.

### (3-4-2) LC-MS/MS Measurement of Trastuzumab

### (Analytical Instruments)

Nano-HPLC: EASY-nLC 1000 (Thermo Fisher Scientific)
Mass spectrometer: Tribrid mass spectrometer Orbitrap Fusion (Thermo Fisher Scientific)

### (HPLC Analysis Conditions)

Trap column: Acclaim PepMap^{®} 100, 75 µm × 2 cm (Thermo Fisher Scientific)
Analytical column: ESI-column (NTCC-360/75-3-125, 75 µm × 12.5 cm, 3 µm; Nikkyo Technos, Co., Ltd.)
Mobile phase A: 0.1% aqueous formic acid solution
Mobile phase B: 0.1% formic acid/acetonitrile solution
Loading solution: 0.1% aqueous trifluoroacetic acid solution
Flow rate: 300 nL/min
Sample injection volume: 0.6 µL
Gradient conditions (B%): 2% (0.0 to 0.5 min), 2% to 50% (0.5 to 50 min), 50% (50 to 55.5 min), 50% to 95% (55.5 to 56.5 min), 95% (56.5 to 60 min)

### (Mass Spectrometer Analysis Conditions)

Ionization method: ESI, Positive mode
Scan type: Data Dependent Acquisition
Activation Type: Collision Induced Dissociation (CID)
Data acquisition was carried out using the bundled software Xcalibur 4.3 (Thermo Fisher Scientific) and Thermo Orbitrap Fusion Tune Application 3.3 (Thermo Fisher Scientific).

### Example 7 Preparation of Natural Killer Cell (Hereinafter, NK Cell)-Antibody Complexes

### Example 7-1 Culture of NK cells

An NK cell medium and an NK cell culture plate were prepared using a CellXVivo^{™} Human NK Cell Expansion Kit (R&D Systems, Inc.). NK cells (Lonza) were cultured on the NK cell culture plate using an NK cell medium supplemented with IL-2, Human, Recombinant (PeproTech, Inc.) at a final concentration of 10002 U/mL and recombinant human IL-15 (PeproTech, Inc.) at a final concentration of 2.50 ng/mL (hereinafter referred to as NK activation medium).

### Example 7-2 Preparation of Linker-Modified Antibodies

### Example 7-2-1 Synthesis of Trastuzumab to Which One Thiol Group Was Introduced per Trastuzumab Molecule (T-6)

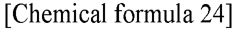

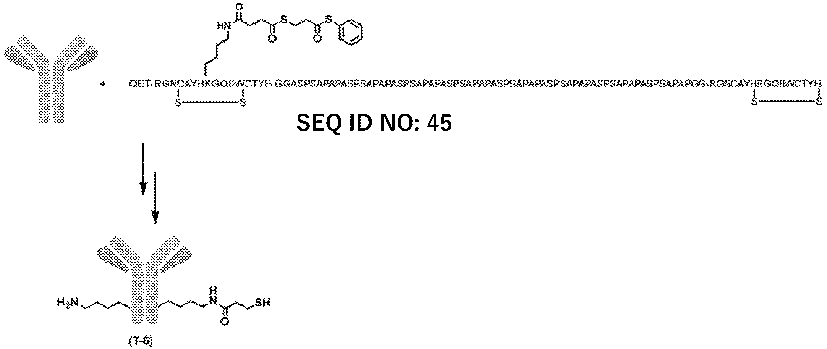

According to Reference Example (3-1) and Reference Example (3-2), Trastuzumab to which one thiol group was introduced per Trastuzumab molecule (T-6) was synthesized.

### Example 7-2-2 Synthesis of Trastuzumab to Which One Cy5 Molecule Was Introduced per Trastuzumab Molecule (T-7)

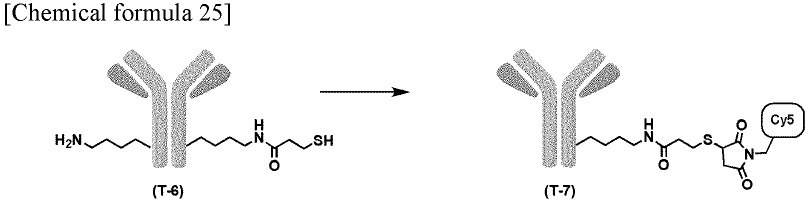

To a 3 mg/mL solution of Trastuzumab obtained in Example 7-2-1 (T-6), to which one thiol group had been introduced per antibody molecule, in PBSE buffer (10 mM phosphate buffered saline (PBS), 10 mM EDTA, pH 7.4), a solution of N-(m-PEG4)-N'-(PEG3-Mal)-Cy5 (BroadPharm) in dimethylformamide (2.5 equivalents relative to the antibody, dimethylformamide (DMF) (8% v/v) solution) was added, and the resulting mixture was shaken at room temperature for 1 hour. The reaction solution was purified using an NAP-25 desalting column (manufactured by Cytiva) to obtain an antibody to which one Cy5 molecule was introduced (T-5) per antibody molecule. HIC-HPLC analysis was performed according to a previous publication (Anal. Chem., 2019, 91, 20, 12724-12732) to confirm that Cy5 was introduced.

### Example 7-2-3 Synthesis of Trastuzumab to Which One Cy5 Molecule and One Peptide Reagent Molecule Were Introduced per Trastuzumab Molecule (T-8)

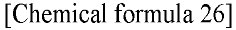

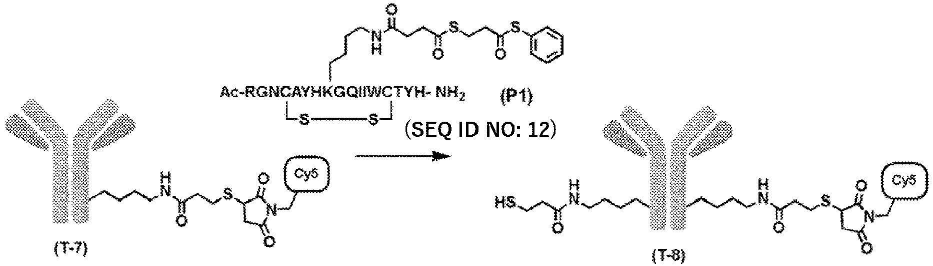

Using Trastuzumab obtained in Example 7-2-2 (T-7), to which one Cy5 molecule had been introduced per Trastuzumab molecule, and reagent P1 described in a previous publication (WO2019/240287A1), (T-8) was obtained by introducing one thiol group to T-7 according to the method of WO2019/240287A1.

### Example 7-2-4 Synthesis of Trastuzumab to Which One Cy5 Molecule and One PEG4-Benzocyclooctyne (DBCO) Molecule Were Introduced per Trastuzumab Molecule (T-9)

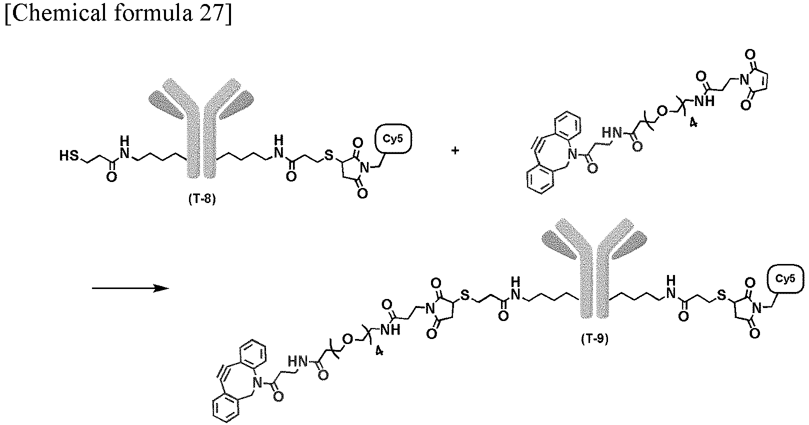

To a 3 mg/mL solution of Trastuzumab obtained in Example 7-2-3 (T-8), in PBSE buffer (10 mM phosphate buffered saline (PBS), 10 mM EDTA, pH 7.4), to which one Cy5 molecule and one thiol group had been introduced per Trastuzumab molecule, a solution of DBCO-PEG4-maleimide (BroadPharm) in dimethylformamide (2.5 equivalents relative to the antibody, dimethylformamide (DMF) (8% v/v) solution) was added, and the resulting mixture was shaken at room temperature for 1 hour. The reaction solution was purified using an NAP-25 desalting column (manufactured by Cytiva) to obtain Trastuzumab to which one Cy5 molecule and one PEG4-DBCO molecule were introduced per Trastuzumab molecule (T-9). HIC-HPLC analysis was performed according to a previous publication (Anal. Chem., 2019, 91, 20, 12724-12732) to confirm that PEG4-DBCO was introduced.

### Example 7-2-5 Synthesis of Trastuzumab to Which One Cy5 Molecule and One PEG12-Benzocyclooctyne (DBCO) Molecule Were Introduced per Trastuzumab Molecule (T-10)

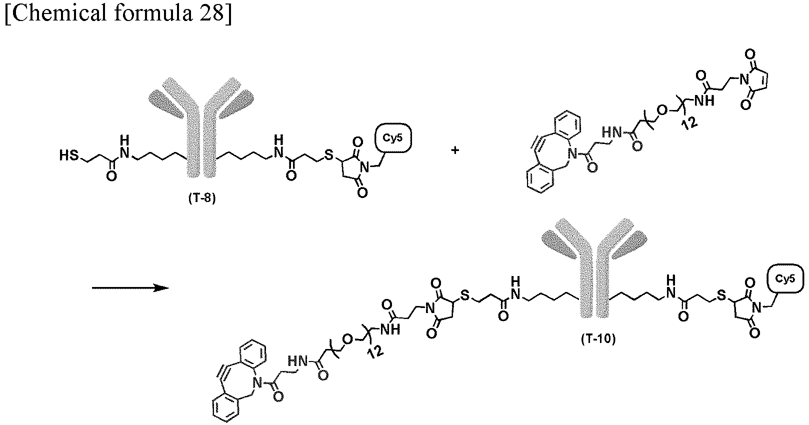

To a 3 mg/mL solution of Trastuzumab obtained in Example 7-2-3 (T-8), to which one Cy5 molecule and one thiol group had been introduced per Trastuzumab molecule, in PBSE buffer (10 mM phosphate buffered saline (PBS), 10 mM EDTA, pH 7.4), a solution of DBCO-PEG12-maleimide (BroadPharm) in dimethylformamide (2.5 equivalents relative to the antibody, dimethylformamide (DMF) (8% v/v) solution) was added, and the resulting mixture was shaken at room temperature for 1 hour. The reaction solution was purified using an NAP-25 desalting column (manufactured by Cytiva) to obtain Trastuzumab to which one Cy5 molecule and one PEG12-DBCO molecule were introduced per Trastuzumab molecule (T-10). HIC-HPLC analysis was performed according to a previous publication (Anal. Chem., 2019, 91, 20, 12724-12732) to confirm that PEG12-DBCO was introduced.

### Example 7-2-6 Synthesis of Trastuzumab to Which One Cy5 Molecule and One PEG24-Benzocyclooctyne (DBCO) Molecule Were Introduced per Trastuzumab Molecule (T-11)

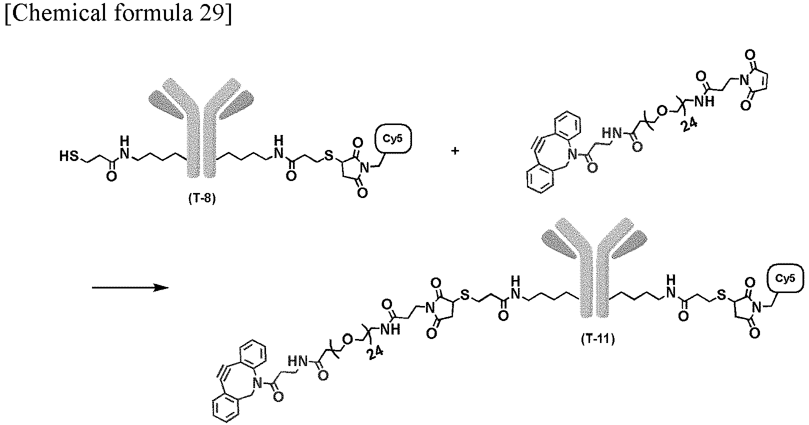

To a 3 mg/mL solution of Trastuzumab obtained in Example 7-2-3 (T-8), to which one Cy5 molecule and one thiol group had been introduced per Trastuzumab molecule, in PBSE buffer (10 mM phosphate buffered saline (PBS), 10 mM EDTA, pH 7.4), a solution of DBCO-PEG24-maleimide (BroadPharm) in dimethylformamide (2.5 equivalents relative to the antibody, dimethylformamide (DMF) (8% v/v) solution) was added, and the resulting mixture was shaken at room temperature for 1 hour. The reaction solution was purified using an NAP-25 desalting column (manufactured by Cytiva) to obtain Trastuzumab to which one Cy5 molecule and one PEG24-DBCO molecule were introduced per Trastuzumab molecule (T-11). HIC-HPLC analysis was performed according to a previous publication (Anal. Chem., 2019, 91, 20, 12724-12732) to confirm that PEG24-DBCO was introduced.

### Example 7-3 Preparation of Antibody-NK Cell Conjugate Using Amine-Reactive Reagents

### 7-3-1 Peptide Synthesis

Peptides having the amino acid sequences of SEQ ID NOs: 41 and 42 were synthesized by solid-phase synthesis according to the method described in WO2018/199337 (the same applies to the peptide synthesis hereinafter). The N-terminus was modified with 4-azidobenzoic acid (Az, Tokyo Chemical Industry Co., Ltd.) to introduce an azide group thereto.
SEQ ID NO: 41: Az-EEEEC-NH₂
SEQ ID NO: 42: Az-EEEEEEEEEEEEEEEEEEEEEEEEEEEEEEC-NH₂

### 7-3-2 Preparation of Amine-Reactive Reagent

As amine-reactive reagents containing a bioorthogonal reactive group, azido-PEG4-NHS ester (BroadPharm), azido-PEG12-NHS ester (BroadPharm), azido-PEG24-NHS ester (BroadPharm), DBCO-PEG12-NHS ester (BroadPharm), DBCO-sulfo-NHS ester (Click Chemistry Tools), and sulfo DBCO-PEG4-TFP ester (BroadPharm) were used. In addition, Az-EEEEC-NH₂ (SEQ ID NO: 41) and Az-EEEEEEEEEEEEEEEEEEEEEEEEEEEEEEC-NH₂ (SEQ ID NO: 42) synthesized in Example (7-3-1) were dissolved in DMSO. To each resulting solution, NHS-PEG6-maleimide (Funakoshi Co., Ltd.) was added at 0.77 molar equivalents, and the resulting mixture was allowed to react at 37°C for 8 hours, to synthesize peptide-based reagents each containing an amine-reactive group and a bioorthogonal reactive group at the termini.

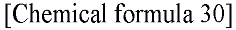

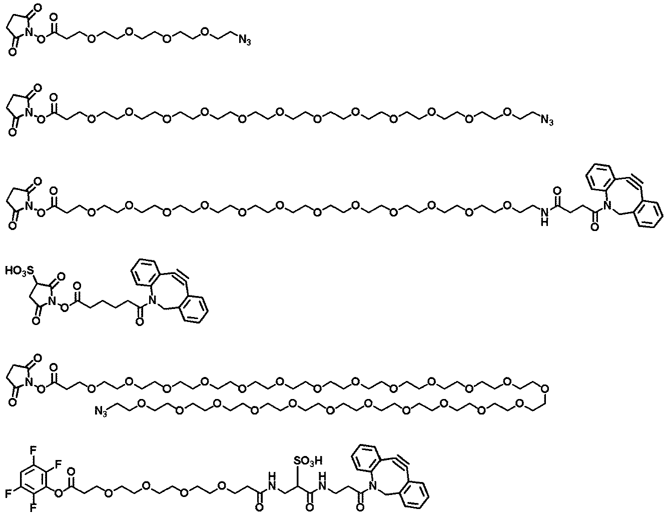

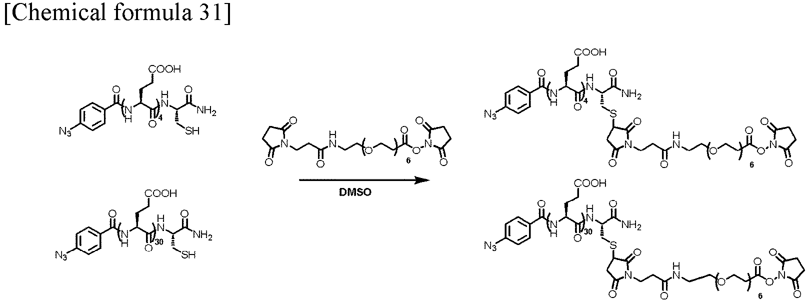

### 7-3-3 Preparation of NK Cells Having Azide Group on Cell Surface

The number of NK cells cultured according to Example 7-1 was quantified using a NucleoCounter^{®} NC-200^{™} (ChemoMetec). After collection of 1.0 × 10⁶ NK cells, centrifugation was performed (at 4000 rpm for 5 minutes), followed by removing the supernatant. A step of adding not less than 1 mL of PBS to the cells, performing centrifugation (at 4000 rpm for 5 minutes), and then removing the supernatant (hereinafter referred to as washing step) was repeated twice. Thereafter, 1 mL of 250 µM azido-PEG24-NHS ester (BroadPharm) (solution in PBS) was added to the cells. The resulting mixture was mixed by repeating inversion for 30 minutes using a rotary mixer, and then centrifugation was performed (at 4000 rpm for 5 minutes), followed by removing the supernatant. Subsequently, the above washing step was repeated twice again, and 200 µL of NK activation medium was added to the cells, to prepare NK cells having an azide group on the cell surface.

### 7-3-4 Preparation of NK Cells Having Various Bioorthogonal Reactive Group on the Cell Surface

In the same manner as in Example 7-3-3, NK cells having a bioorthogonal reactive group on the cell surface were prepared using the amine-reactive reagents prepared in Example 7-3-2.

### 7-3-5 Preparation of Antibody-NK Cell Conjugate

### 7-3-5-1 Preparation of Antibody-NK Cell Conjugate Having PEG28 Linker (ACC-1)

To the NK cells having azide on the cell surface, obtained in Example 7-3-3, trastuzumab synthesized in Example (7-2-4), to which one Cy5 molecule and one PEG4-DBCO molecule had been introduced per Trastuzumab molecule, was added to a final concentration of 2 µL, and the resulting mixture was left to stand at 37°C in an incubator for 1 hour. After centrifugation (at 4000 rpm for 5 minutes) and removal of the supernatant, the washing step was repeated twice, and then NK activation medium was added to the cells, to synthesize an antibody-NK cell conjugate having a PEG28 linker (ACC-1).

### 7-3-5-2 Preparation of Antibody-NK Cell Conjugate Having PEG48 Linker (ACC-2)

To the NK cells having azide on the cell surface, obtained in Example 7-3-3, trastuzumab synthesized in Example (7-2-6), to which one Cy5 molecule and one PEG24-DBCO molecule had been introduced per Trastuzumab molecule, was added to a final concentration of 2 µL, and the resulting mixture was left to stand at 37°C in an incubator for 1 hour. After centrifugation (at 4000 rpm for 5 minutes) and removal of the supernatant, the washing step was repeated twice, and then NK activation medium was added to the cells, to synthesize an antibody-NK cell conjugate having a PEG48 linker (ACC-2).

### 7-3-6 Evaluation of Antibody-NK Cell Conjugate

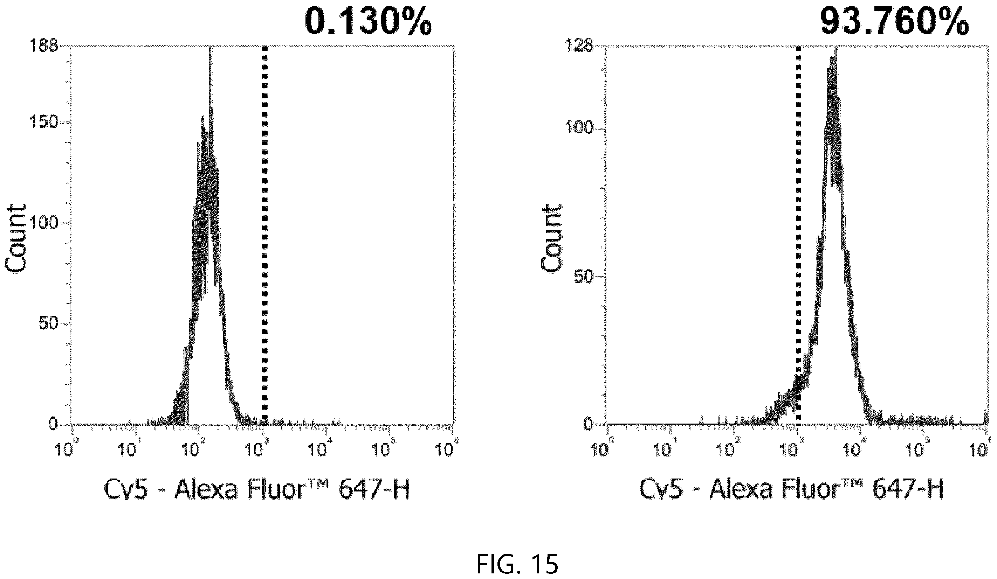

For ACC-1, flow cytometry analysis was carried out using an Attune NxT Flow Cytometer (Thermo Fisher Scientific) to observe binding of the antibody to the cells. By using, as a threshold, the maximum autofluorescence value of NK cells whose surface had not been modified, strong fluorescence derived from Cy5 was observed in the antibody-NK cell conjugate as shown in Fig. 15. Thus, highly efficient binding of the antibody to the NK cells could be confirmed.

### Example 7-4 Preparation of Antibody-NK Cell Conjugate Using Metabolic Labeling Method

### 7-4-1 Preparation of NK Cells Having Azide Group on Cell Surface

To NK cells cultured according to Example 7-1, N-azidoacetylmannosamine tetraacylated (Tokyo Chemical Industry Co., Ltd.) was added to a final concentration of 25 µM, 50 µM, or 100 µM. After performing incubation overnight at 37°C, the cells were centrifuged (at 4000 rpm for 5 minutes), and the supernatant was removed. Washing (addition of not less than 1 mL of PBS followed by centrifugation to remove the supernatant) was performed twice using PBS, and then NK activation medium was added to the cells to provide NK cells having an azide group on the cell surface.

### 7-4-2 Preparation of Antibody-NK Cell Conjugates

### 7-4-2-1 Preparation of Antibody-NK Cell Conjugate Having PEG4 Linker (ACC-3)

To the NK cells having azide on the cell surface, obtained in Example 7-4-1, trastuzumab synthesized in Example (7-2-4), to which one Cy5 molecule and one PEG4-DBCO molecule had been introduced per Trastuzumab molecule, was added to a final concentration of 2 µL, and the resulting mixture was left to stand at 37°C in an incubator for 1 hour. After centrifugation (at 4000 rpm for 5 minutes) and removal of the supernatant, the washing step was repeated twice, and then NK activation medium was added to the cells to synthesize an antibody-NK cell conjugate having a PEG4 linker (ACC-3).

### 7-4-2-2 Preparation of Antibody-NK Cell Conjugate (ACC-4) Having PEG12 Linker

To the NK cells having azide on the cell surface, obtained in Example 7-4-1, trastuzumab synthesized in Example 7-2-5, to which one Cy5 molecule and one PEG12-DBCO molecule had been introduced per Trastuzumab molecule, was added to a final concentration of 2 µL, and the resulting mixture was left to stand at 37°C in an incubator for 1 hour. After centrifugation (at 4000 rpm for 5 minutes) and removal of the supernatant, the washing step was repeated twice, and then NK activation medium was added to the cells, to synthesize an antibody-NK cell conjugate having a PEG12 linker (ACC-4).

### 7-4-2-3 Preparation of Antibody-NK Cell Conjugate Having PEG24 Linker (ACC-5)

To the NK cells having azide on the cell surface, obtained in Example 7-4-1, trastuzumab synthesized in Example 7-2-6, to which one Cy5 molecule and one PEG24-DBCO molecule had been introduced per Trastuzumab molecule, was added to a final concentration of 2 µL, and the resulting mixture was left to stand at 37°C in an incubator for 1 hour. After centrifugation (at 4000 rpm for 5 minutes) and removal of the supernatant, the washing step was repeated twice, and then NK activation medium was added to the cells to synthesize an antibody-NK cell conjugate having a PEG24 linker (ACC-5).

### 7-4-3 Evaluation of Antibody-NK Cell Conjugates

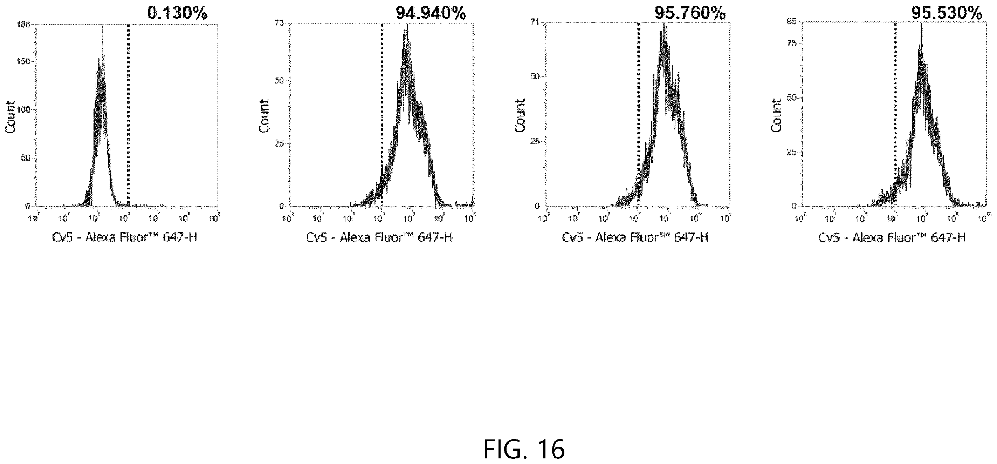

By performing the same procedure as in Example 7-3-6 for the antibody-NK cell conjugates obtained in Example 7-4-2, it could be confirmed that the antibody was bound to the cell surface with higher efficiency (Fig. 16).

### Example 8 Evaluation of Cytotoxicity of Antibody-NK Cell Conjugates

### 8-1 Culture of SK-BR-3 Cells

SK-BR-3 (ATCC) was selected as HER2-positive target cells. SK-BR-3 was cultured in RPMI 1640 Medium, GlutaMAX^{™} Supplement medium (Thermo Fisher Scientific), supplemented with 10% FBS (Thermo Fisher Scientific). The above SK-BR-3 culture medium will be referred to hereinafter as RPMI medium.

### 8-2 Cytotoxicity Evaluation Assay

As a cytotoxicity evaluation system, an assay system using a DELFIA EuTDA Cytotoxicity Detection kit (Revvity) (hereinafter referred to as the DELFIA assay) was used. SK-BR-3 cells cultured according to Example 8-1 were washed twice with PBS, and then TrypLE^{™} Select Enzyme (1X), no phenol red (Thermo Fisher Scientific) was added to the cells. The cells were suspended by incubation at 37°C for 3 minutes, and then centrifuged (at 120 g for 5 minutes), followed by removing the supernatant and adding 1 mL of RPMI medium (see Example 8-1) thereto. The number and viability of the cells in the obtained cell suspension were measured with a Countess Automated Cell Counter (Thermo Fisher Scientific), and then RPMI medium was further added to the cells to achieve a concentration of 2.5 × 10⁶ cells/mL. One milliliter of the obtained cell suspension was collected, and 5 µL of DELFIA BATDA labeling reagent (included in the DELFIA EuTDA Cytotoxicity Detection kit) was added thereto, followed by incubating the resulting mixture at 37°C for 1 hour. Centrifugation was carried out, and then the supernatant was removed. Thereafter, a step of adding 1 mL of PBS to the cells, washing the cells, performing centrifugation, and then removing the supernatant was repeated twice, and then 500 µL of NK activation medium was added to the cells. The number and viability of the cells in the obtained cell suspension were measured with a Countess Automated Cell Counter (Thermo Fisher Scientific), and NK activation medium was further added to the cells to achieve a concentration of 50,000 cells/mL. The obtained cell suspension was aliquoted in 100-µL volumes into wells of a 96-well plate, to provide target cells for an assay. To the target cells, NK cells prepared according to Example 7-1 or antibody-NK cell conjugates prepared according to Examples 7-3-5 and 7-4-2 were added such that the ratio of the NK cells or each antibody-NK cell conjugate to the SK-BR-3 cells was 6:1, 3:1, 1:1, or 0:1. The final volume was adjusted to 200 µL/well. At this time, the numbers of the NK cells and the antibody-NK conjugates were measured using a NucleoCounter^{®} NC-200^{™} (ChemoMetec). In addition, a well to which 90 µL of NK activation medium and 10 µL of lysis buffer (included in the DELFIA EuTDA Cytotoxicity Detection kit) were added instead of the effector cells (hereinafter referred to as a 100% kill well), and a well to which 100 µL of NK activation medium was added (hereinafter referred to as a control well), were also provided.

The above 96-well plate was incubated at 37°C for 4 hours and then centrifuged (at 500 g for 5 minutes), followed by adding 20 µL of the resulting supernatant to a 96-well plate for the DELFIA assay that had been separately provided (included in the DELFIA EuTDA Cytotoxicity Detection kit). After adding 200 µL of DELFIA Eu-Solution thereto, the plate was shaken at room temperature for 15 minutes, and then subjected to time-resolved fluorescence measurement of each well using a Revvity Nivo multimode plate reader (Revvity). Based on the obtained intensity, cytotoxicity was calculated according to the following equation. [Mathematical Formula 1] $Cytotoxicity \left(%\right) = 100 \times \frac{\left(Fluorescence intensity of sampling well\right) - \left(Fluorescence intensity of control well\right)}{\left(Fluorescence intensity of control well\right) - \left(Fluorescence intensity of sampling well\right)}$

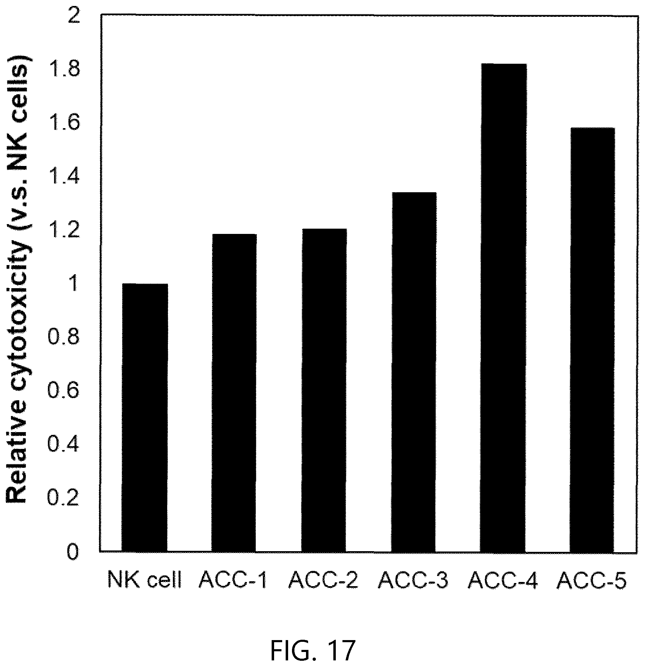

As a result, as shown in Fig. 17, the antibody-NK cell conjugates synthesized by the modification method using the NHS reagent or the metabolic labeling method exhibited higher cytotoxicities than the NK cells.

### Comparative Example 1 Preparation of Antibody-Cell Conjugate Prepared Based on Prior Literature

### 1-1 Synthesis of Trastuzumab to Which Single-Stranded DNA Was Introduced per Trastuzumab Molecule (T-13)

Using Trastuzumab (Chugai Pharmaceutical Co., Ltd.) and a single-stranded DNA (hereinafter referred to as DNA1, SEQ ID NO: 43), Trastuzumab to which the single-stranded DNA was introduced (T-13) was synthesized according to prior literature (WO2023/114719A1, Cytotherapy 22 (2020) 135-143). HIC-HPLC analysis was performed according to a previous publication (Anal. Chem., 2019, 91, 20, 12724-12732), and introduction of the DNA was confirmed.
SEQ ID NO: 43: s_ccctagagtgagtcgtatga (s=Thiol C6)

### 1-2 Synthesis of NK Cells to Which Single-Stranded DNA Was Introduced (C-1)

Using NK cells and a single-stranded DNA (hereinafter referred to as DNA2, SEQ ID NO: 44), NK cells having a surface modified with single-stranded DNA2 (C-1) were prepared according to prior literature (WO2023/114719A1, Langmuir. 2009 June 16; 25(12): 6985-6991). SEQ ID NO: 44: s_tcatacgactcactctaggg (s=Thiol C6)

### 1-3 Preparation of Antibody-NK Cell Conjugate Having DNA Linker (ACC-6)

Using Comparative Examples (1-1) and (1-2), an antibody-NK cell conjugate (ACC-6) was synthesized according to prior literature (WO2023/114719A1, Cytotherapy 22 (2020) 135-143, Langmuir. 2009 June 16; 25(12): 6985-6991).

### Comparative Example 2 Comparison of Cytotoxicity with That of Antibody-NK Cell Conjugate Prepared Based on Prior Literature

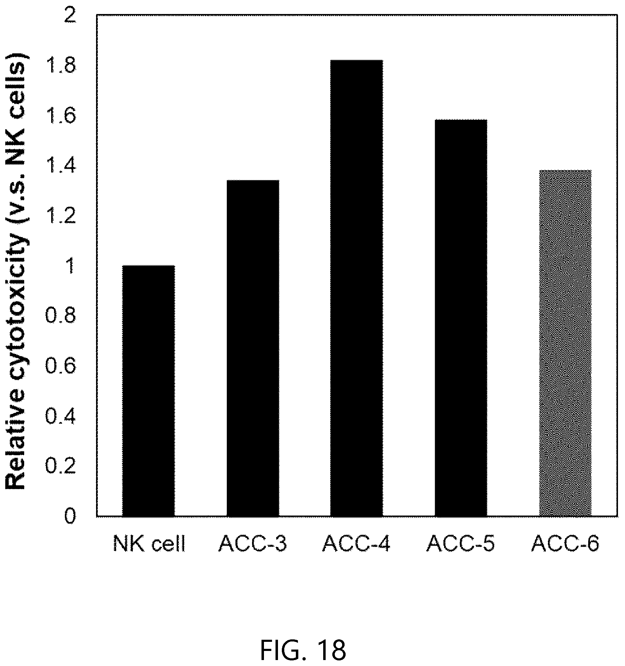

According to the method of Example 8-2, the cytotoxicity was evaluated for the antibody-NK cell conjugate synthesized based on Comparative Example 1-3 (ACC-6) and the antibody-NK cell conjugates synthesized in Example 7-4-2 (ACC-3, 4, and 5). As a result, as shown in Fig. 18, ACC-3, 4, and 5 exhibited higher activities than ACC-6.

It was thus shown that the antibody-NK cell conjugates synthesized in Example 7-4-2 (ACC-3, 4, and 5) exhibit higher cytotoxicities than the antibody-NK cell conjugate synthesized in Comparative Example 1-3 (ACC-6).

### Comparative Example 3 Comparison of Antigen-Binding Ability with That of Antibody-NK Cell Conjugate Prepared Based on Prior Literature

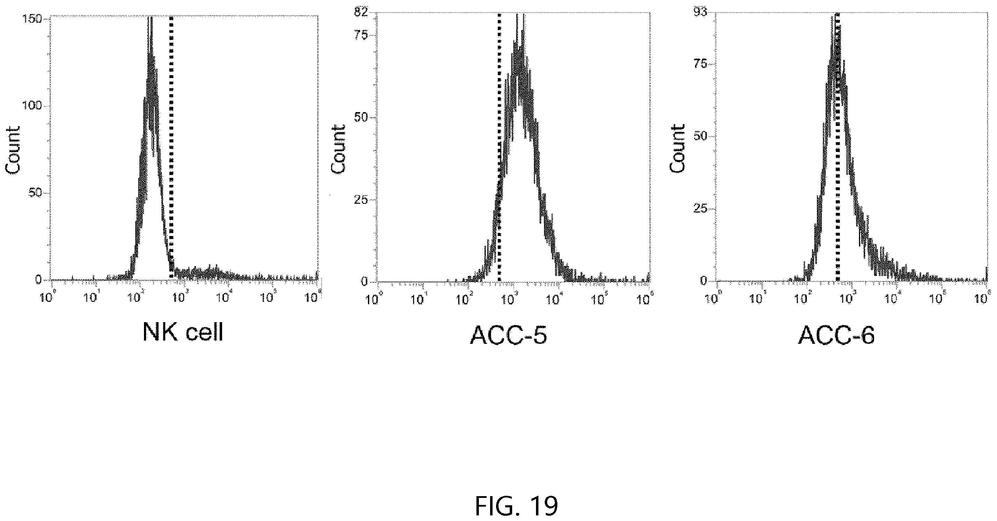

The antibody-NK cell conjugate synthesized based on Comparative Example 1-3 (ACC-6) and the NK cell conjugate synthesized based on Example 7-4-2 (ACC-5) were cultured for one day after their preparation, and their HER2-binding abilities were evaluated based on Example 5-2. As a result, as shown in Fig. 19, ACC-5 exhibited higher antigen-binding ability than ACC-6.

## Claims

1. An Fc-cell complex comprising a cell and an antibody Fc region bound to a surface of the cell,
wherein the antibody Fc region is formed by disulfide bonding of two Fc-constituting polypeptides, and
wherein the antibody Fc region is chemically bound to the cell surface via a linker introduced into a functional group in a side chain of a particular amino acid residue present at one or more positions of one of the two antibody Fc-constituting polypeptides.

2. The Fc-cell complex according to claim 1, wherein the particular amino acid residue is one or more of the following 14 amino acid residues: asparagine, glutamine, methionine, proline, serine, threonine, tryptophan, tyrosine, aspartic acid, glutamic acid, arginine, histidine, and lysine.

3. The Fc-cell complex according to claim 1, wherein the particular amino acid residue is a lysine residue.

4. The Fc-cell complex according to claim 3, wherein the lysine residue is one or more of the lysine residues at positions 246, 248, 274, 288, 290, 317, 320, 322, 360, 414, and 439.

5. The Fc-cell complex according to claim 1, wherein the antibody Fc region has a functional substance introduced therein via a linker introduced into a functional group in a side chain of a particular amino acid residue present at one or more positions of the other of the two Fc-constituting polypeptides.

6. The Fc-cell complex according to claim 5, wherein the functional substance is a drug, a labeling substance, an affinity substance, a transporting substance, or a stabilizer.

7. The Fc-cell complex according to claim 1, wherein the antibody Fc region comprises an antibody variable region.

8. The Fc-cell complex according to claim 1, wherein the antibody Fc region comprises a fusion protein of an antibody Fc-constituting polypeptide and a functional polypeptide.

9. The Fc-cell complex according to claim 1, wherein the antibody Fc region is introduced into the cell surface by reaction between a bioorthogonal functional group introduced via a linker that is introduced into a functional group in a side chain of a particular amino acid residue present at one or more positions of one of the two Fc-constituting polypeptides, and a group that is present on or introduced into the cell surface and reacts with the bioorthogonal functional group.

10. A method of producing a cell-Fc region complex, the method comprising
(I) preparing an antibody Fc region formed by disulfide bonding of two antibody Fc-constituting polypeptides;
(II) reacting the antibody Fc region with a compound represented by the following Formula (Ia) or Formula (Ib):
[Chemical formula 1]
R-L₁-CLE(B)-L₂-A (Ia)
wherein
R represents a reactive group for the Fc region,
L₁ represents a first linker,
L₂ represents a second linker,
CLE(B) represents a cleavable portion capable of generating, by cleavage, a bioorthogonal functional group on the reactive group side, and
A represents an affinity substance containing an affinity peptide, or 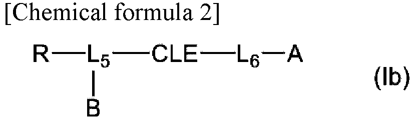
wherein R represents the reactive group,
L₅ represents a fifth linker,
L₆ represents a sixth linker,
B represents a group containing a bioorthogonal functional group,
CLE represents a cleavable portion, and
A represents the affinity substance,
to form an Fc-Compound (Ia) complex or an Fc-Compound (Ib) complex;
(III) subjecting the Fc-Compound (Ia) complex or the Fc-Compound (Ib) complex to cleavage treatment at the cleavable portion, to form an Fc region that does not comprise the affinity substance and to which the bioorthogonal functional group has been introduced;
(IV) preparing a cell having, on a surface thereof, a functional group that reacts with the bioorthogonal functional group; and
(V) introducing the Fc region to the cell surface by reacting the Fc region, to which the bioorthogonal functional group is introduced, with the cell, through reaction of the bioorthogonal functional group of the Fc region with the functional group of the cell that reacts with the bioorthogonal functional group.

11. The method according to claim 10, wherein A is represented by AP1-L_{A}-AP2,
wherein AP1 represents a first affinity peptide having affinity for a constant region of a heavy chain of an antibody, AP2 represents a second affinity peptide having affinity for a constant region of a heavy chain of an antibody, and L_{A} represents a linker.

12. The method of producing a cell-Fc region complex according to claim 10, wherein Compound (Ia) is represented by the following Formula (Ia-1): 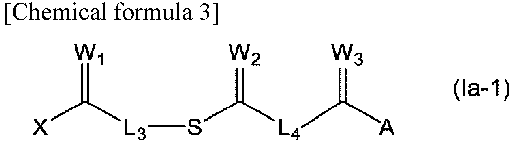 wherein
X represents a leaving group,
W₁, W₂, and W₃ each independently represent an oxygen atom or a sulfur atom,
L₃ represents a third linker,
L₄ represents a fourth linker,
S represents a sulfur atom, and
A represents the affinity substance.

13. The method of producing a cell-Fc region complex according to claim 10, wherein Compound (Ib) is represented by the following Formula (Ib-1): 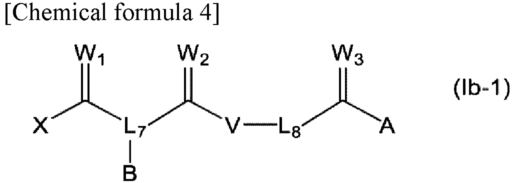 wherein
X represents a leaving group,
W₁, W₂, and W₃ each independently represent an oxygen atom or a sulfur atom,
L₇ represents a seventh linker,
L₈ represents an eighth linker,
B represents a group containing a bioorthogonal functional group,
V represents an oxygen atom or a sulfur atom, and
A represents the affinity substance.

14. The method of producing a cell-Fc region complex according to claim 10, wherein the bioorthogonal functional group is an azide group, an alkyne residue, a tetrazine residue, an alkene residue, a thiol group, a maleimide residue, a furan residue, or a halocarbonyl residue.

15. The method of producing a cell-Fc region complex according to claim 10, wherein the cell-Fc region complex is the Fc-cell complex according to any one of claims 1 to 9.

16. The method of producing a cell-Fc region complex according to claim 10, wherein the cell-Fc region complex is the Fc-cell complex according to claim 5, and
wherein the method comprises introducing the functional substance to the functional group in the side chain of the particular amino acid residue present at one or more positions of the other of the two Fc-constituting polypeptides in the antibody Fc region.
